(19) Europäisches Patentamt
European Patent Office
Office européen des brevets

(11) Numéro de publication : 0 449 699 A2

(12) # DEMANDE DE BREVET EUROPEEN

(21) Numéro de dépôt : 91400717.4

(22) Date de dépôt : 18.03.91

(51) Int. Cl.[5] : **C07D 231/22**, C07D 403/10, C07D 231/24, C07D 231/20, C07C 69/738, C07C 205/56, C07C 255/57, C07D 231/12, C07D 231/14, C07D 409/10, C07D 409/14, // C07D333/38, A61K31/415, A61K31/41

(30) Priorité : 19.03.90 FR 9003485

(43) Date de publication de la demande : 02.10.91 Bulletin 91/40

(84) Etats contractants désignés : AT BE CH DE DK ES FR GB GR IT LI LU NL SE

(71) Demandeur : LABORATOIRES UPSA
1 bis, rue du Docteur Camille Bru
F-47000 Agen (FR)

(72) Inventeur : Bru-Magniez, Nicole
24-26, avenue Raphael
F-75016 Paris (FR)
Inventeur : Nicolai, Eric
52, rue Saint-Ouen, Résidence le Hameau-Bat. C
F-14000 Caen (FR)
Inventeur : Teulon, Jean-Marie
13, avenue Gulbert
F-78170 La Celle Saint Cloud (FR)

(74) Mandataire : Portal, Gérard et al
Cabinet Beau de Loménie 55, rue d'Amsterdam
F-75008 Paris (FR)

(54) **Dérivés de pyrazole antagonistes des récepteurs à l'angiotensine II, leurs procédés de préparation, compositions pharmaceutiques les contenant.**

(57) La présente invention concerne les dérivés de formule (I) :

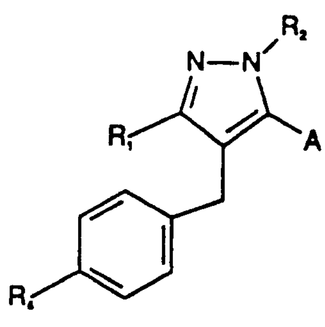

Formule (I)

Ainsi que leurs sels d'addition et leur utilisation en thérapeutique, notamment pour le traitement des maladies cardiovasculaires en particulier pour le traitement de l'hypertension et de l'insuffisance cardiaque.

EP 0 449 699 A2

# NOUVEAUX DERIVES DE PYRAZOLE ANTAGONISTES DES RECEPTEURS A L'ANGIOTENSINE II; LEURS PROCEDES DE PREPARATION, COMPOSITIONS PHARMACEUTIQUES LES CONTENANT

La présente invention concerne en tant que produits nouveaux, les dérivés de pyrazole de formule générale (I) ci-dessous ainsi que leurs sels.

Les composés en question présentent un profil pharmacologique très intéressant dans la mesure où ils sont doués de propriétés antagonistes des récepteurs à l'angiotensine II. Ils sont donc particulièrement indiqués pour le traitement des maladies cardiovasculaires, en particulier pour le traitement de l'hypertension et pour le traitement de l'insuffisance cardiaque.

La présente invention concerne également le procédé de préparation des dits produits et leurs applications en thérapeutique. Elle concerne en outre les composés intermédiaires nouveaux permettant la synthèse des dits produits.

Ces dérivés de pyrazole sont caractérisés en ce qu'ils répondent à la formule générale (I) :

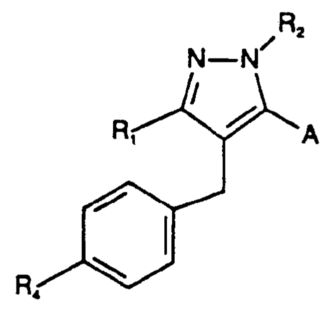

Formule (I)

dans laquelle :

$R_1$ est un radical alkyle inférieur de 1 à 6 atomes de carbone ou un radical alkényle inférieur de 2 à 6 atomes de carbone, ou un radical cycloalkyle en $C_3$-$C_7$ ou un radical cycloalkényle en $C_4$-$C_7$,

$R_2$ est l'atome d'hydrogène, un radical alkyle inférieur de 1 à 6 atomes de carbone, un radical halogéno alkyle inférieur de 1 à 6 atomes de carbone, ou un radical cycloalkyle en $C_3$-$C_7$, un groupement $-(CH_2)_m-COOR_5$ ou un groupement $-CH_2-(CH_2)_m-OR_5$ ou un groupement $-CH_2-(CH_2)_m-SR_5$, m étant un nombre entier de 0 à 5, $R_5$ étant un atome d'hydrogène ou un radical alkyle inférieur de 1 à 6 atomes de carbone,

A peut représenter un groupement:

$-(CH_2)_qOR'$, R' étant un atome d'hydrogène ou un radical alkyle inférieur de 1 à 6 atomes de carbone, ou un radical cycloalkyle en $C_3$-$C_7$, q étant un nombre entier de 1 à 5

$-(CH_2)_qL$, L étant un atome d'halogène, préférentiellement chlore ou brome, q ayant la même signification que ci-dessus

-CHO, un acétal, un dioxolane,

-COOR', R' étant défini comme ci-dessus

-CONR"R‴, R" et R‴ représentant indépendamment un atome d'hydrogène, un radical alkyle inférieur de 1 à 6 atomes de carbone, un radical cycloalkyle en $C_3$-$C_7$ ou pouvant former avec l'atome d'azote auquel ils sont rattachés un hétérocycle comme la pyrrolidine, la pipéridine, la morpholine, la thiomorpholine ou une pipérazine

-CN

$-(CH_2)_qCN$, q étant défini comme ci-dessus

$-(CH_2)_qCOOR'$, R' et q étant défini comme ci-dessus

$-(CH_2)_q$ CONR"R‴, R", R‴ et q étant définis comme ci-dessus

$-(CH_2)_qNR"R‴$, R", R‴ et q étant définis comme ci-dessus

$-(CH_2)_q-S-R'$, R' et q étant définis comme ci-dessus

$-OR_3$, $R_3$ étant un atome d'hydrogène, un radical alkyle inférieur de 1 à 6 atomes de carbone ou un radical cycloalkyle en $C_3$-$C_7$, un groupement $-(CH_2)_n-COOR_6$, un groupement $-CH_2-(CH_2)_n-CN$, un groupement $-CH_2-(CH_2)_n-O-R_6$, un groupement $-CH_2-(CH_2)_n-S-R_6$, un groupement $-COR_6$, n étant un nombre entier de 0 à 5, $R_6$ étant un atome d'hydrogène ou un radical alkyle inférieur de 1 à 6 atomes de carbone, $R_3$ peut encore représenter un groupement $-(CH_2)_p-CO-NR_7R_8$ ou $-(CH_2)_p-CH_2-CH_2NR_7R_8$, p étant un nombre entier de 0 à 5, $R_7$ et

$R_8$ représentant indépendamment un atome d'hydrogène ou un radical alkyle inférieur de 1 à 6 atomes de carbone, un radical cycloalkyle en C3-$C_7$ ou pouvant former avec l'atome d'azote auquel ils sont rattachés un hétérocycle comme la pyrrolidine, la pipéridine, la morpholine, la thiomorpholine ou une pipérazine,

$R_4$ peut représenter un groupement nitro, amino, -COOR$_9$, $R_9$ étant l'atome d'hydrogène ou un radical alkyle inférieur de 1 à 6 atomes de carbone, $R_4$ peut également représenter les radicaux suivants :

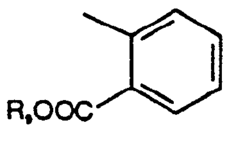 , 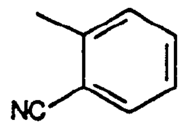 , 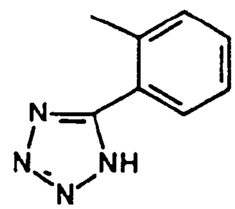 , 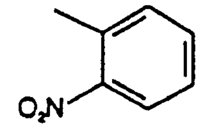

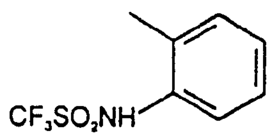 , 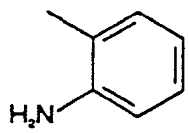 , 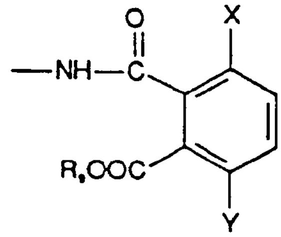

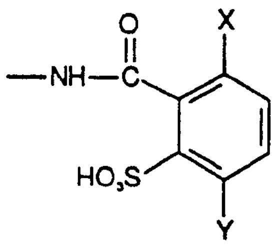 , 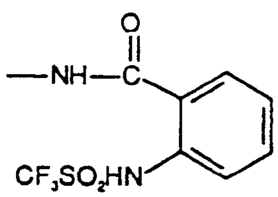 , 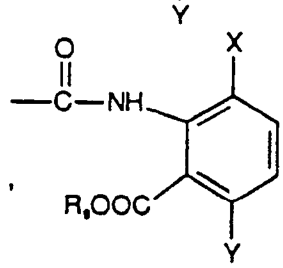

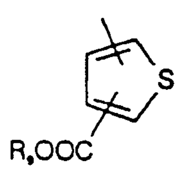 , 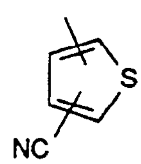 , 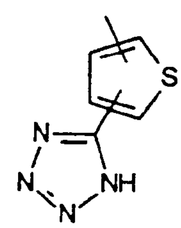

dans lesquels $R_9$ a la même signification que ci-dessus,

X et Y représentant indépendamment un atome d'hydrogène, un radical alkyle inférieur, un atome d'halogène, un radical alkoxy, ou un radical trifluorométhyl.

Dans la description et les revendications, on entend par radical alkyle inférieur une chaîne hydrocarbonée ayant de 1 à 6 atomes de carbone, linéaire ou ramifiée. Un radical alkyle inférieur est par exemple un radical méthyle, éthyle, propyle, isopropyle, butyle, isobutyle, tertiobutyle, pentyle, isopentyle, hexyle ou isohexyle.

Par radical cycloalkyle en $C_3$-$C_7$, on entend un radical cyclique saturé, il s'agit de préférence d'un radical cyclopropane, cyclobutane, cyclopentane, cyclohexane ou cycloheptane.

On entend par radical alkényle inférieur, une chaîne hydrocarbonée ayant de 2 à 6 atomes de carbone linéaire ou ramifiée et présentant une insaturation. Un radical alkényle inférieur est par exemple un radical éthène, propène, isopropène, butène, isobutène, pentène, isopentène, hexène, isohexène.

On entend par radical cycloalkényle en $C_4$-$C_7$ un radical cyclique possédant une insaturation, il s'agit de préférence d'un radical cyclobutène, cyclopentène, cyclohexène ou cycloheptène.

Par radical halogéno alkyle inférieur de 1 à 6 atomes de carbone, on entend un radical alkyle dont 1 à 6 atomes d'hydrogène ont été substitués par 1 à 6 atomes d'halogène. Un radical halogéno alkyle inférieur est par exemple un radical trifluorométhyl ou un radical trifluoro-2,2,2 éthyl.

Par radical alkoxy on entend un groupement O-alkyle inférieur, alkyle inférieur étant défini comme ci-dessus.

Par hétérocycle on entend désigner un cycle de 5 à 7 atomes comportant de 1 à 3 hétéroatomes choisis parmi l'oxygène, le soufre ou l'azote, éventuellement substitué par un groupement alkyle inférieur, halogénoalkyle inférieur, alkoxy ou un noyau phényl éventuellement substitué par un de ces groupements.

Dans la description et les revendications on entend par halogène un atome de chlore, de brome, d'iode ou de fluor.

Le document EP-A-0323841 de Du Pont de Nemours décrit des pyrroles, pyrazoles et triazoles. Ces composés possèdent toujours la substitution benzyle sur un atome d'azote :

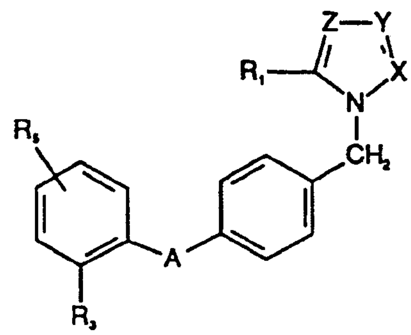

Or, la demanderesse a découvert, de façon surprenante, que contrairement à ce qui ressort du document Du Pont de Nemours, il n'était pas essentiel que la substitution benzyle se trouve sur un atome d'azote, en particulier en position -1 (ou -2) du cycle pyrazole mais qu'on pouvait obtenir des produits très performants lorsque cette substitution benzyle était sur un atome de carbone en position -4 du cycle pyrazole. De plus, la demanderesse a découvert que la présence d'un groupe A et notamment d'un atome d'oxygène en position -3 (ou -5) du pyrazole combinée avec la substitution benzyle en position -4 conduisait à des composés particulièrement actifs comme antagonistes des récepteurs à l'angiotensine II.

Selon une variante de réalisation, $R_1$ est un groupement n-propyl ;

selon une autre variante de réalisation, $R_1$ est un groupement n-butyl ;

selon une variante de réalisation, $R_2$ est un groupement méthyl ;

selon une autre variante de réalisation, $R_2$ est un groupement trifluoro-2,2,2,-éthyl ;

selon une variante de réalisation, A est un groupement éthoxy carbonyl méthylène oxy ;

selon une autre variante de réalisation, A est un groupement diméthyl amino carbonyl oxy;

selon une autre variante de réalisation, A est un groupement méthoxy méthylène ;

selon une autre variante de réalisation, A est un groupement hydroxy-2 éthyl oxy ;

selon une variante de réalisation, $R_4$ est un groupement carboxy-2 dichloro-3,6 benzoyl amino;

selon une autre variante de réalisation, $R_4$ est un groupement acide sulfonique-2 benzoyl amino;

selon une autre variante de réalisation, $R_4$ est un groupement (tétrazol-yl-5)-2 phényl ;

les composés de l'invention particulièrement préférés sont ceux qui sont choisis parmi les produits de formule :

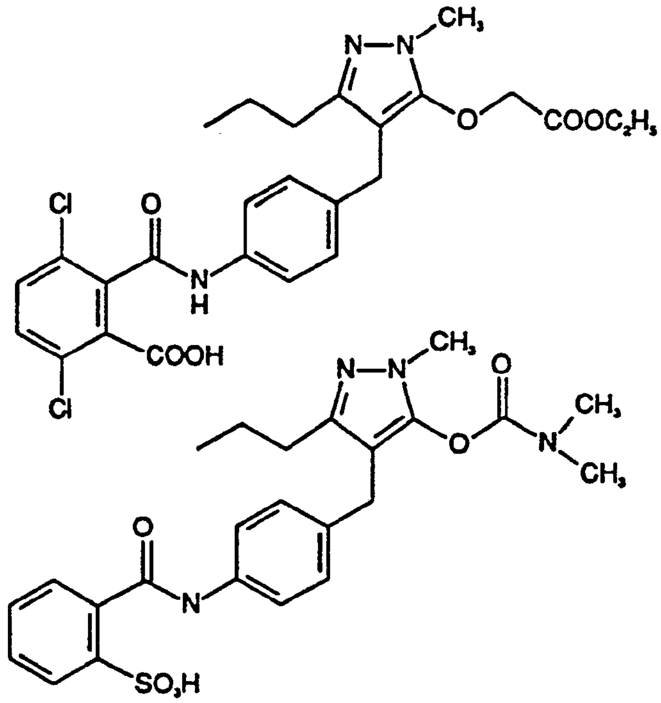

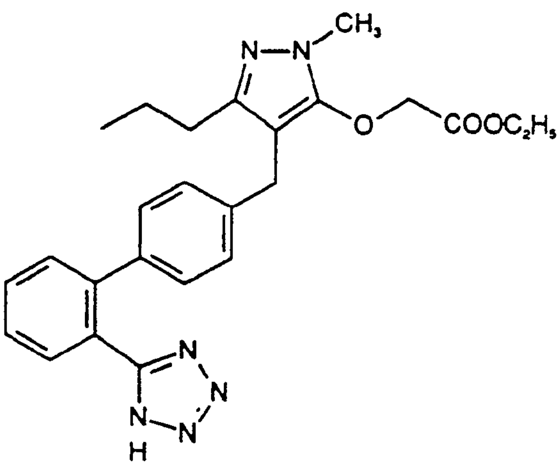

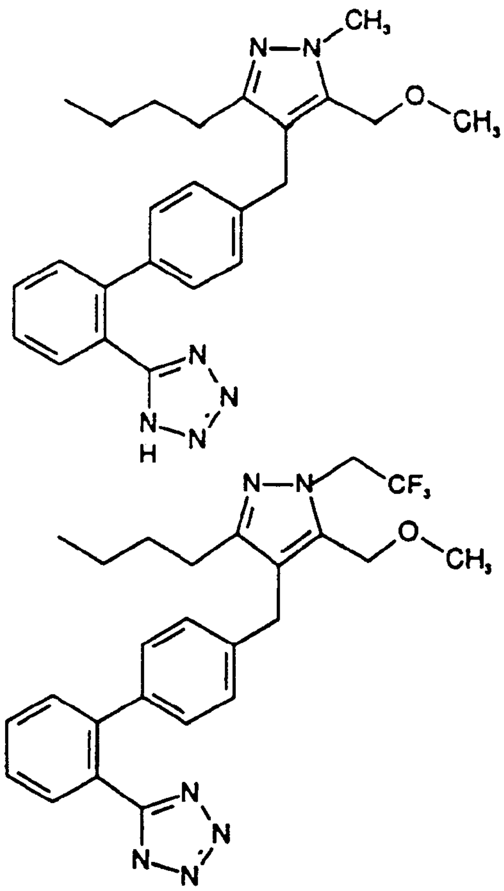

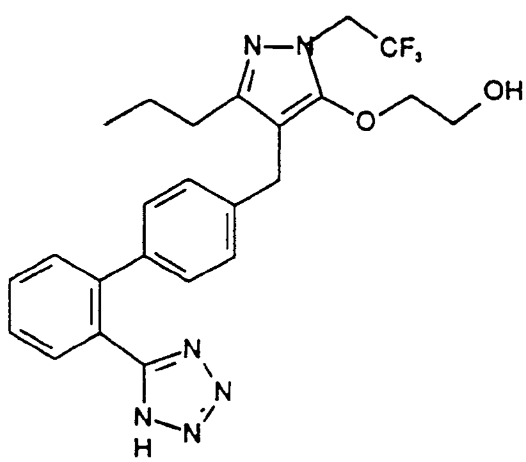

Selon l'invention, les composés de formule (I) pourront être préparés selon le schéma suivant :

on préparera :

– les oxo-3 alkanoates d'alkyle de formule (II) :

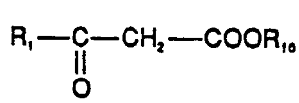

Formule (II)

dans laquelle $R_1$ est défini comme ci-dessus et $R_{10}$ représente un radical alkyle inférieur, de préférence méthyle ou éthyle,

– les dicétones-1,3 de formule (III) :

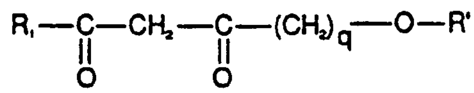

Formule (III)

dans laquelle $R_1$, R'et q sont définis comme ci-dessus,

ces composés de formule (II) et (III) peuvent être préparés par des méthodes classiques, telles que la réaction de Claisen ou la méthode de l'acide de Meldrum. On pourra trouver des méthodes de préparation de ce type de composés dans les références suivantes :

- OIKAWA. Y; SUGANO. K; YONEMITSU. O; J. Org. Chem., 1978, 43 (10), 2087-88.
- WIERENGA. W; SKULNICK. H.I.; J. Org. Chem., 1979, 44, 310.
- HOUGHTON. R; LAPHAM. D; SYNTHESIS, 1982, 6, 451-2.
- BRAM. G ; VILKAS. M; Bull. Soc. Chim. France, 1964 (5), 945-51.
- BALYAKINA. M.V; ZHDANOVICH. E.S ; PREOBRAZHENSKII. N.A ; Tr. Vses. Nauchn. Issled. Vitam in. Inst., 1961, 7, 8-16.
- RENARD. M; MAQUINAY. A ; Bull. Soc. Chim. Belg., 1946, 55, 98-105.
- BRUCE. F.W; COOVER H.W ; J. Am. Chem. Soc., 1944, 66, 2092-94.
- EBY. C.J; et HAUSER. C.R ; J. Am. Chem. Soc., 1957, 79, 723-5.

Par benzylation des composés de formule (II) ou (III) en présence d'une base telle qu'un carbonate de sodium ou de potassium dans l'acétone, un alcoolate de sodium ou de potassium dans un alcool, un hydrure de sodium ou de lithium dans des solvants comme le tétrahydrofurane, le dioxane ou le diméthyl formamide par exemple, à une température comprise entre 50 et 100°C ou encore, en présence d'un équivalent de chlorure ou de bromure de lithium et de deux équivalents de diisopropyl éthyl amine au reflux du tétrahydrofurane selon la référence SUNG-EUN YOO ; KYU YANG YI ; Bull. Korean. Chem. Soc. 1989, 10 (1), 112,
à l'aide de composés de formule (IV)

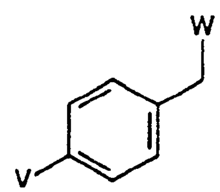

Formule (IV)

on obtiendra les composés de formules (V) et (VI) :

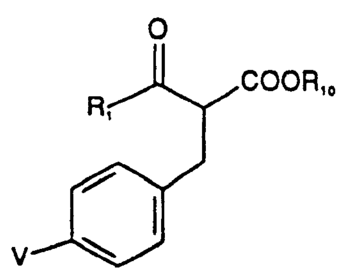

Formule (V)

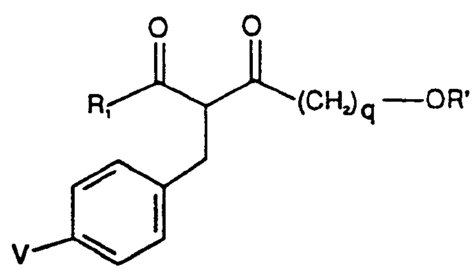

Formule (VI)

les composés de formules (V) et (VI) peuvent être également obtenus par condensation d'un aldéhyde de formule (VII)

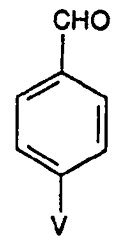

Formule (VII)

sur un composé de formule (II) ou de formule (III) suivie d'une hydrogénation catalytique, par exemple en présence de Nickel de Raney dans un solvant comme un alcool, sous pression ou à pression ordinaire, lorsque les substitutions présentes le permettent.

D'une manière plus générale, on trouvera des méthodes de préparation des composés de formule (V) ou de formule (VI) dans les références suivantes :

- DURGESHWARI. P; CHAUDHURY. N.D; J. Ind. Chem. Soc., 1962, 39, 735-6.
- HEINZ. P; KREGLEWSKI. A; J. Prakt. Chem., 1963, 21 (3-4), 186-197.
- ZAUGG. H.E; DUNNIGAN. D.A; MICHAELS. R.J; SWETT. L.R; J. Org. Chem., 1961, 26, 644-51.
- KAGAN. H.B; HENG SUEN. Y; Bull. Soc. Chim. France, 1966 (6), 1819-22.
- RATHKE. M.W; DEITCH. J; Tetrahedron Lett, 1971 (31), 2953-6.
- BORRIES KUBEL. ; Liebigs. Ann. Chem., 1980, 1392-1401.
- MARQUET. J; MORENO-MANAS. M; Chem. Lett, 1981, 2, 173-6.
- IOFFE. T; POPOV. E.M; VATSURO. K.V; TULIKOVA. E.K ; KABACHNIK. M.I; Tetrahedron, 1962, 18, 923-940.
- SHEPHERD. T.M; Chem. Ind. (London), 1970, 17, 567.

Dans la formule (IV), W représente un atome d'halogène, préférentiellement le chlore ou le brome.
Dans la même formule :

- V peut être un groupement nitro, le dérivé de formule (IV) est alors commercial.
- V peut être un groupement $COOR_{11}$, $R_{11}$ étant un radical alkyle inférieur ou benzyle, le dérivé de formule (IV) sera alors préparé par chloration ou bromation à l'aide du N-chloro succinimide ou du N-bromosuccinimide dans un solvant comme le tétrachlorure de carbone ou le dibromoéthane d'un ester de l'acide p-méthyl benzoïque qui est commercial, selon la référence :
  - JULIA. M ; CHASTRETTE. F; Bull. Soc. Chim. France, 1962 (2), 2247.

-V peut être un groupement 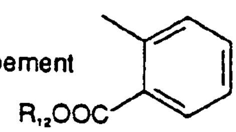 ,

$R_{12}$ étant un radical alkyle inférieur ou benzyle, les composés de formule (IV) sont alors préparés par réaction d'un magnésien du p-bromo toluène sur un composé de formule :

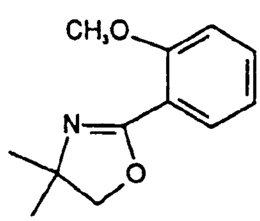

pour obtenir un composé de formule :

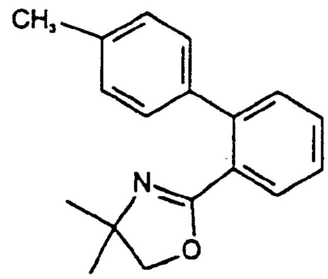

qui est hydrolysé pour conduire au composé de formule:

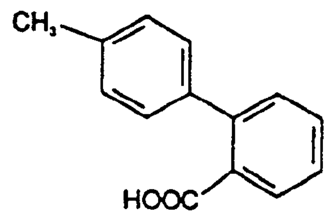

On trouvera des modes opératoires pour les trois étapes décrites ci-dessus dans la référence :

– MEYERS. A.I ; MIHELICH. E.D ; J. Am. Chem. Soc., 1975, 97, 7383.

L'acide est ensuite estérifié par un alcool de formule $R_{12}OH$, $R_{12}$ étant défini comme ci-dessus.

Ces dérivés sont alors bromés ou chlorés par exemple par le N-bromo succinimide ou le N-chloro succinimide dans un solvant comme le tétrachlorure de carbone ou le dibromoéthane pour conduire aux composés de formule (IV) où V est le groupement

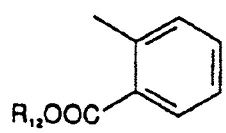

- V peut être le groupement 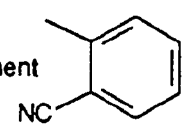 ,

dans ce cas le composé:

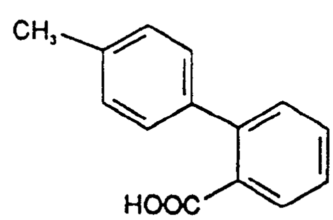

précédemment préparé sera transformé en amide primaire par action du chlorure d'acide, obtenu avec le chlorure de thionyle ou l'oxychlorure de phosphore, sur l'ammoniac et cet amide sera transformé en nitrile par action de l'oxychlorure de phosphore dans le diméthylformamide ou du chlorure de thionyle. Le nitrile obtenu :

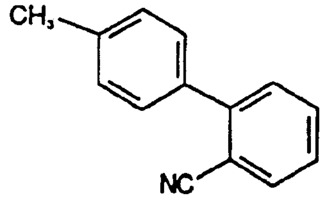

sera ensuite bromé ou chloré dans les mêmes conditions que l'ester précédent pour conduire aux composés de formule (IV) où V est le groupement

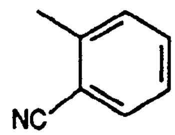

- V peut être le groupement 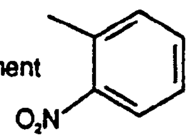

dans ce cas, le composé

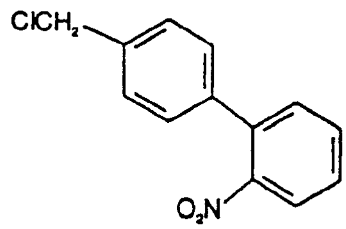

sera préparé par chlorométhylation du 2-nitrobiphényl commercial, selon les références :

- CA : 70 (25) : 114837 d
- CA: 69 (2) : 3704 t

pour conduire aux composés de formule (IV) où V est le groupement

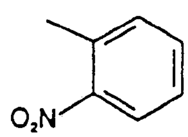

- V peut être un groupement 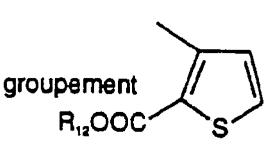 ,

$R_{12}$ étant un radical alkyl inférieur ou benzyle, les composés de formule (IV) correspondants sont obtenus de la façon suivante :

A partir du composé:

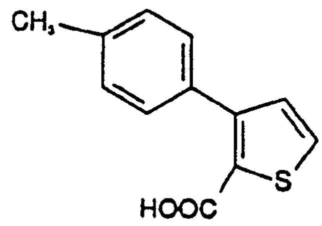

dont on peut trouver la préparation dans la référence :

– FISSELMANN. H; HABITCH. H; Ger. Offen.: 1,092,929 (1960); CA: 57: 5894 g

on obtiendra les composés de formule:

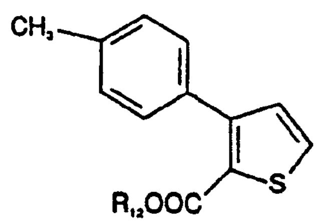

par estérification à l'aide d'un alcool de formule $R_{12}OH$, $R_{12}$ étant défini comme ci-dessus par des méthodes classiques connues de l'homme de l'art.

Ces composés sont ensuite traités par le N-chloro succinimide ou le N-bromo succinimide dans un solvant comme le tétrachlorure de carbone ou le dibromoéthane par exemple, pour donner les composés de formule (IV) dans lesquels V représente le

groupement 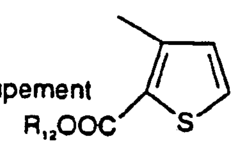 ,

$R_{12}$ étant défini comme ci-dessus.

- V peut être le groupement 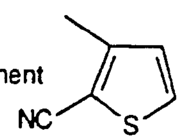

dans ce cas les composés de formule (IV) correspondants seront préparés de la façon suivante :

à partir du composé acide (p-méthyl phényl)-3 thiophène-2 carboxylique dont la préparation est donnée ci-dessus, par traitement avec le chlorure de thionyle puis l'ammoniac on obtient le composé amide qui est ensuite déshydraté par le chlorure de thionyle ou l'oxy chlorure de phosphore sans solvant ou dans le diméthyl formamide pour conduire au composé nitrile:

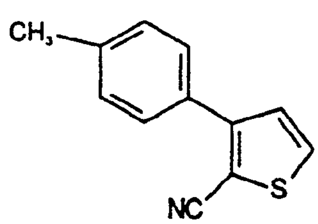

Ce composé nitrile est ensuite halogéné par le N-chloro succinimide ou le N-bromo succinimide dans un

solvant comme le tétrachlorure de carbone ou le dibromoéthane pour donner les composés de formule (IV) dans lesquels V représente le groupement

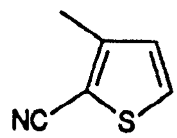

- V peut être le groupement 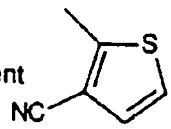

dans ce cas les composés de formule (IV) correspondants sont synthétisés de la façon suivante :

à partir de la chloro-4 butyrophénone de formule:

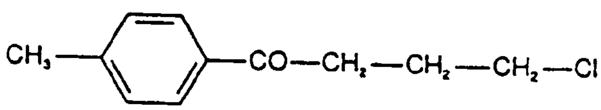

dont la préparation peut être trouvée dans le brevet BE : 577,977 du 15 mai 1959, CA: 54, 4629 c,

par traitement par de l'oxychlorure de phosphore et du diméthyl formamide selon les conditions décrites dans la référence:

– VOLODINA. M.A; TERENT'EV. A.P; KUDRYASHOVA. V.A; KABOSHINA. L.N; Khim. Geterosikl. Soedim ; 1967, 5-8 ;

on obtiendra le composé de formule:

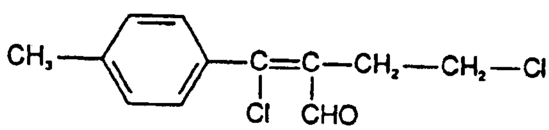

Ce composé est ensuite traité par le sulfure de sodium dans un solvant comme le tétrahydrofurane au reflux pour donner le dérivé

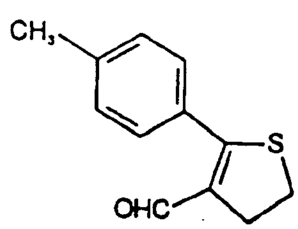

qui est ensuite transformé en deux étapes en dérivé nitrile par déshydratation de l'oxime formée à partir de l'aldéhyde et de l'hydroxylamine. Cette déshydratation pourra être effectuée par exemple à l'aide de l'anhydride acétique pour donner le composé nitrile :

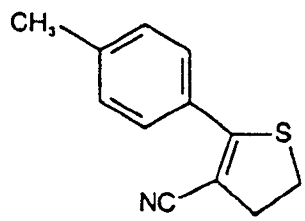

qui pourra être ensuite aromatisé par traitement avec du brome dans le tétrachlorure de carbone puis avec le tertiobutylate de potassium dans le tétrahydrofurane pour donner le composé :

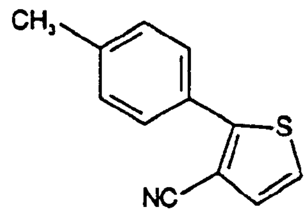

Ce composé peut être ensuite chloré ou bromé par des agents d'halogénation tels que le N-chloro succinimide ou le N-bromo succinimide dans un solvant comme le tétrachlorure de carbone ou le dibromoéthane pour donner les composés de formule (IV) dans lesquels V représente le groupement

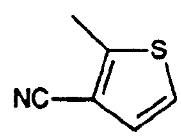

- V peut être le groupement 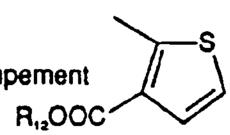 ,

$R_{12}$ étant défini comme ci-dessus, ils pourront être préparés à partir du composé de formule

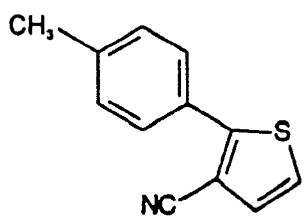

par hydrolyse classique de la fonction nitrile puis estérification de l'acide obtenu ou passage directement de la fonction nitrile à la fonction ester selon les méthodes connues de l'homme de l'art, suivie d'une chloration ou d'une bromation de l'ester par le N-chloro succinimide ou le N-bromo succinimide dans le tétrachlorure de carbone ou le dibromo éthane par exemple.

Dans les formules (V) et (VI), $R_1$, $R_{10}$, R' et q sont définis comme précédemment et V a la même signification que dans la formule (IV).

Certains dérivés de la formule (V) et de la formule (VI) lorsque V représente un alkoxy carbonyl-2 phényl, cyano-2 phényl, nitro-2 phényl, alkoxy carbonyl-thiophène ou cyano-thiophène sont des intermédiaires de synthèse nouveaux qui sont revendiqués en eux-mêmes.

Dans la formule (VII) V à la même définition que dans la formule (IV) mais cette méthode de condensation ne sera utilisée que lorsque V possède une fonction que respecte l'hydrogénation. Dans certains cas ces aldéhydes pourront être préparés à partir des dérivés de formule (IV) selon des réactions connues de l'homme de

l'art, on peut citer la réaction de Sommelet ( Bull. Soc. Chim. France 1918, [4] 23, 95) ou celle au nitropropane (Organic Syntheses Collec. Vol. IV, 932).

Par action d'une hydrazine de formule (VIII)

$$H_2N—NH—R_2$$

Formule (VIII)

dans laquelle $R_2$ est défini comme ci-dessus, sur les composés de formule (V), par simple chauffage au reflux d'un alcool par exemple, on obtiendra les composés de formule (IX) :

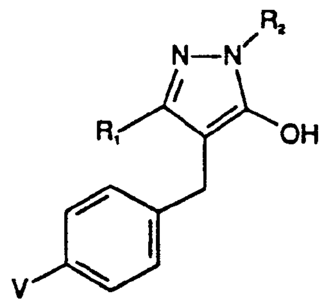

Formule (IX)

dans laquelle $R_1$, $R_2$ et V ont les définitions données ci-dessus.

Ces composés de formule (IX) sont des intermédiaires de synthèse nouveaux qui sont revendiqués en eux mêmes.

L'alkylation des composés de formule (IX) réalisée en présence d'une base telle que le carbonate de potassium ou de sodium dans des solvants tels que l'acétone, la butanone-2 ou le diméthyl formamide ou en présence d'alcoolate de sodium ou de potassium dans un alcool ou en présence d'hydrure de sodium ou de lithium dans le tétrahydrofurane par des dérivés de formule (X)

$$Z—R_3$$

Formule (X)

Z étant un atome de brome, de chlore ou d'iode, $R_3$ étant défini comme ci-dessus conduit aux composés de formule (XI) :

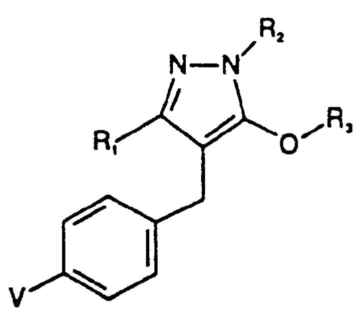

Formule (XI)

dans laquelle $R_1$, $R_2$, $R_3$ et V sont définis comme ci-dessus.

De même, par action d'une hydrazine de formule (VIII), définie comme ci-dessus, sur un composé de formule (VI), par simple chauffage au reflux d'un alcool par exemple, on obtiendra un mélange de composés de formule (XII) et de formule (XII')

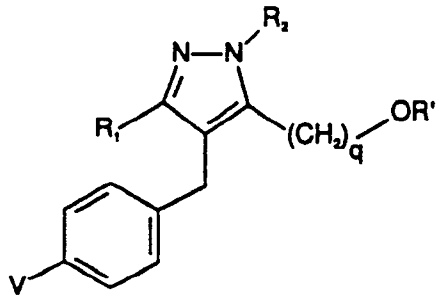

Formule (XII)

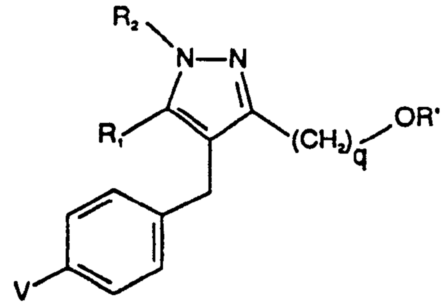

Formule (XII')

dans lesquelles $R_1$, $R_2$, R', q et V sont définis comme ci-dessus.

A partir de ce mélange, on obtiendra les composés de formule (XII) purs par une purification soit par chromatographie sur silice soit par recristallisation ou tout autre procédé de purification connu de l'homme de l'art.

Une autre méthode de préparation des composés de formule (XII) consiste à traiter dans un premier temps, les composés de formule (VI) par l'hydrate d'hydrazine selon les mêmes conditions afin d'obtenir les composés de formule (XIII) :

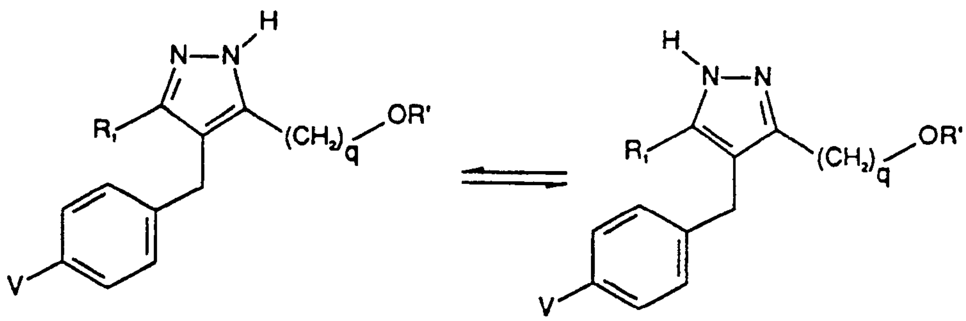

Formule (XIII)

dans laquelle $R_1$, R', q et V sont définis comme ci-dessus,
ces composés de formule (XIII) sont ensuite alkylés en présence de DBU (1,8-diazabicyclo-[5.4.0] undec-7-ene), dans un solvant tel que l'acétone ou l'acétonitrile par exemple, par des dérivés halogénés de formule (XIV) :

$$Z-R_2$$

Formule (XIV)

dans laquelle $R_2$ a la même signification que ci-dessus et Z étant un atome de brome, de chlore ou d'iode pour conduire au mélange des composés de formule (XII) et de formule (XII') tels que définies ci-dessus, les composés de formule (XII) étant alors obtenus purs après purification comme mentionné. Cette seconde méthode de préparation peut être particulièrement avantageuse dans le cas où $R_2$ est un groupement alkyle inférieur car elle permet d'obtenir de façon prépondérante les composés de formule (XII) par rapport aux composés de formule (XII').

Les composés de formule (XII) dans lesquels R' est un hydrogène seront préparés en deux étapes : par traitement avec du tribromure de bore des composés de formule (XII) dans lesquels R' est un groupement alkyle inférieur on obtiendra les dérivés bromés de formule (XV)

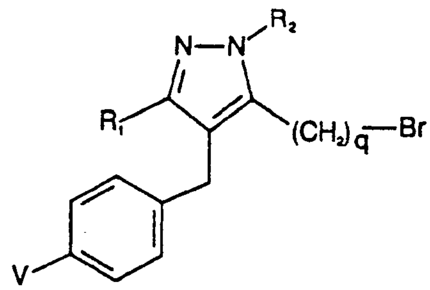

Formule (XV)

dans laquelle $R_1$, $R_2$, q et V sont définis comme ci-dessus, le traitement de ces dérivés bromés de formule (XV) par le carbonate de potassium ou de sodium au reflux du mélange dioxane-eau permet d'obtenir les composés de formule (XII) dans lesquels R' est l'atome d'hydrogène.

Les dérivés de formule (XI) ou de formule (XII) dans lesquels :

– V est un groupement nitro pourront subir une hydrogénation catalytique, par exemple en présence de Nickel de Raney dans un alcool à pression atmosphérique ou sous pression pour conduire aux composés de formule (I) où $R_4$ est un groupement amino et A un groupement $-(CH_2)_q-OR'$ ou $OR_3$, R', $R_3$ et q étant définis comme ci-dessus.

Par action sur ces dérivés d'un anhydride phtalique convenablement substitué on obtiendra les composés de formule générale (I) où $R_4$ représente le groupement

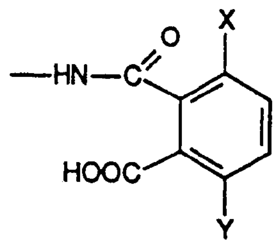

dans lequel X et Y sont définis comme ci-dessus et où A est un groupement $-(CH_2)_q-OR'$ ou $OR_3$, q, R'et $R_3$ étant définis comme ci-dessus,
l'acide obtenu pouvant être ensuite estérifié pour obtenir le groupement

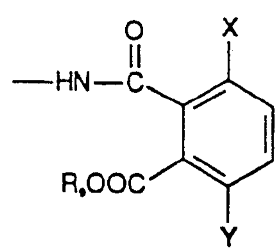

De même par action de l'anhydride cyclique d'un acide orthosulfobenzoïque convenablement substitué sur ces composés aminés on obtiendra les composés de formule générale (I) où $R_4$ représente le groupement

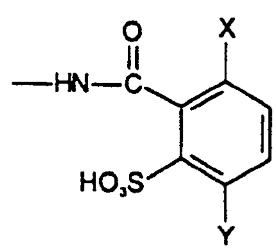

et A est un groupement $-(CH_2)_q-OR'$ ou $OR_3$, q, R', $R_3$, X et Y étant définis comme ci-dessus.

De même par action du chlorure de l'acide N-(trifluorométhyl sulfonyl) anthranilique dont on peut trouver la préparation dans les références :

CA 96 (13) : 103651 z

CA 97 (7): 55500 w

sur ces composés aminés on obtiendra les composés de formule générale (I) où $R_4$ représente le groupement

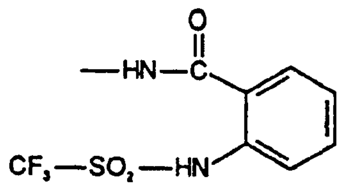

et A est un groupement $-(CH_2)_q-OR'$ ou $OR_3$, q, R' et $R_3$ étant définis comme ci-dessus.

Les composés de formules (XI) ou (XII) dans lesquels :

– V est un groupement $-COOR_{11}$ pourront être hydrolysés en milieu acide ou basique ou hydrogénés dans le cas où $R_{11}$ est un benzyle afin de respecter les autres fonctions esters présentes, pour conduire aux composés de formule (I) où $R_4$ est un groupement -COOH et A est un groupement $-(CH_2)_q-OR'$ ou $OR_3$, q, R' et $R_3$ étant définis comme ci-dessus.

Ces dérivés acides pourront conduire, après avoir été transformés en chlorure d'acide avec le chlorure de thionyle ou en anhydride mixte avec le chloroformiate d'éthyle, par réaction sur des dérivés anthraniliques de formule :

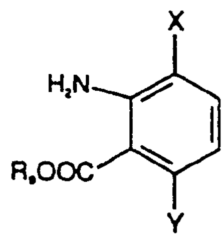

dans lesquels X, Y et $R_9$ sont définis comme ci-dessus, aux composés de formule générale (I) où $R_4$ représente le groupement :

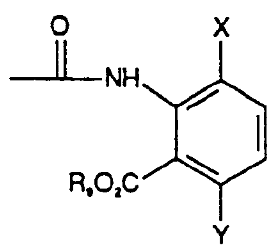

et A est un groupement $-(CH_2)_q-OR'$ ou $OR_3$, q, R' et $R_3$ étant définis comme ci-dessus.

Les composés de formules (XI) ou (XII) dans lesquels:

- V est un groupement 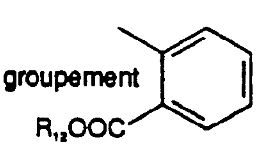

selon la même manière seront hydrolysés ou hydrogénés en présence d'un catalyseur tel que le palladium sur charbon dans le cas où $R_{12}$ est un benzyle, pour conduire aux composés de formule (I) où $R_4$ est un groupement:

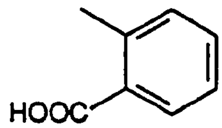

et A un groupement $-(CH_2)_q-OR'$ ou $OR_3$, q, R' et $R_3$ étant définis comme ci-dessus.

Les composés de formules (XI) et (XII) dans lesquels:

- V est un groupement 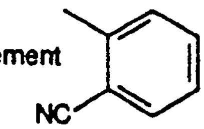

pourront réagir avec un équivalent d'azoture de sodium dans un solvant tel que le diméthyl formamide en présence d'un sel d'ammonium comme le chlorure d'ammonium ou avec un azoture de trialkyl étain au reflux du toluène puis avec l'acide chlorhydrique gazeux dans le tétrahydrofurane pour conduire aux composés de formule générale (I) où $R_4$ représente un groupement :

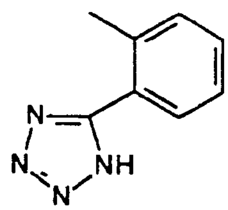

et A est un groupement $-(CH_2)_q-OR'$ ou $OR_3$, q, R' et $R_3$ étant définis comme ci-dessus.

Les composés de formules (XI) et (XII) dans lesquels:

- V est un groupement 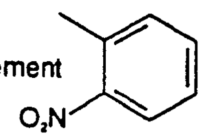

pourront subir une hydrogénation catalytique par exemple en présence de Nickel de Raney, dans un alcool à pression atmosphérique ou sous pression pour conduire à des composés de formule générale (I) où $R_4$ représente un groupement :

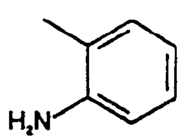

et A est un groupement $-(CH_2)_q-OR'$ ou $OR_3$, q, R' et $R_3$ étant définis comme ci-dessus.

Par action du chlorure de l'acide trifluorométhane sulfonique sur ces derniers dans un solvant tel que le chloroforme ou dans un solvant aromatique comme le toluène en présence d'une base comme la triéthylamine ou la pyridine ou dans la pyridine, on obtiendra les composés de formule générale (I) où $R_4$ représente un groupement :

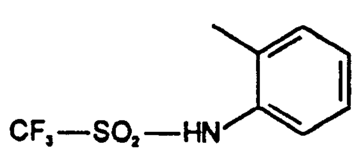

et A est un groupement $-(CH_2)_q-OR'$ ou $OR_3$, q, R' et $R_3$ étant définis comme ci-dessus.

Les composés de formules (XI) et (XII) dans lesquels:

– V représente le groupement

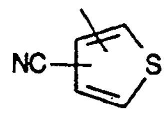

pourront être traités par un azoture de trialkyl étain dans le toluène au reflux puis par l'acide chlorhydrique gazeux dans le tétrahydrofurane pour conduire aux dérivés de formule (I) dans lesquels $R_4$ représente le groupement

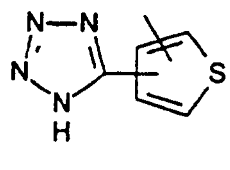

et A est un groupement $-(CH_2)_q-OR'$ ou $OR_3$, q, R' et $R_3$ étant définis comme ci-dessus.

Les composés de formules (XI) et (XII) dans lesquels:

– V représente le groupement

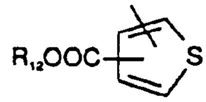

pourront être hydrolysés ou hydrogénés en présence d'un catalyseur tel que le Palladium sur charbon dans le cas où $R_{12}$ est un benzyle, pour conduire aux composés de formule (I) dans lesquels $R_4$ représente le groupement

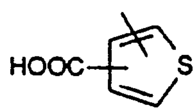

et A est un groupement $-(CH_2)_q-OR'$ ou $OR_3$, q, R' et $R_3$ étant définis comme ci-dessus.

Les composés de formule (I) dans lesquels A représente un groupement $CH_2OH$ pourront être oxydés par un agent oxydant doux comme le dioxyde de manganèse dans un solvant comme le chloroforme pour donner les composés de formule (I) dans lesquels A représente le groupement CHO qui pourra être transformé selon les méthodes classiques connues de l'homme de l'art en acétal ou en dioxolane, par chauffage avec un alcool ou un diol en présence d'acide paratoluène sulfonique par exemple (Synthesis 1981, 501 ).

Une oxydation plus poussée de ces mêmes composés aldéhydes ou directement des composés alcool, par exemple avec des agents d'oxydation tels que le permanganate de potassium conduira aux composés de formule (I) dans lesquels A représente le groupement $CO_2H$.

Les composés de formule (I) dans lesquels A représente le groupement $CO_2H$ pourront être estérifiés par des méthodes classiques d'estérification pour donner les composés de formule (I) dans lesquels A représente un groupement $CO_2R'$, R' étant un radical alkyle inférieur.

Les composés de formule (I) dans lesquels A représente le groupement $CO_2H$ pourront également être transformés en amide en plusieurs étapes, après éventuellement des étapes de protection d'autres groupements fonctionnels si nécessaire, par transformation en chlorure d'acide puis traitement par l'ammoniac ou par une amine de formule HN R''R''', R'' et R''' étant définis comme ci-dessus pour conduire aux composés de formule (I) dans lesquels A représente un groupement CON R''R'''.

Les composés de formule (I) dans lesquels A représente un groupement $CONH_2$ pourront être traités par un agent tel que le chlorure de thionyle ou l'oxychlorure de phosphore pour donner les composés de formule (I) dans lesquels A représente le groupement CN.

Les composés de formule (I) dans lesquels A représente un groupement $(CH_2)_qOR'$, R' étant un radical

alkyle inférieur et q un nombre entier de 1 à 5 pourront être traités par le tribromure de bore dans le chloroforme pour donner, après éventuellement des étapes de protection et déprotection d'autres groupements fonctionnels si nécessaire, les composés de formule (I) dans lesquels A représente le groupement $(CH_2)_qBr$.

Les composés de formule (I) dans lesquels A représente le groupement $(CH_2)_qBr$ pourront être traités par du cyanure de sodium ou de potassium dans des solvants tels que l'alcool, ou un mélange alcool-eau, le diméthyl sulfoxyde ou l'acétonitrile, pour donner les composés de formule (I) dans lesquels A représente le groupement $(CH_2)_qCN$ ou encore par des amines de formule HNR″R‴ dans lesquelles R″ et R‴ sont définis comme ci-dessus pour conduire aux composés de formule (I) où A représente $(CH_2)_q$ NR″R‴ ou encore par des thiols de formule HS-R′, R′ étant défini comme ci-dessus pour conduire aux dérivés de formule (I) où A représente $(CH_2)_q$-S-R′.

Les composés de formule (I) dans lesquels A représente ce groupement $(CH_2)_qCN$ pourront être hydrolysés selon les méthodes classiques d'hydrolyse des nitriles pour donner les composés de formule (I) dans lesquels A représente le groupement $(CH_2)_qCO_2H$.

Ces composés acides pourront eux-mêmes être estérifiés par les méthodes classiques d'estérification pour conduire aux composés de formule (I) dans lesquels A représente un groupement $(CH_2)_qCO_2R'$, R′ étant un radical alkyle inférieur et q un nombre entier de 1 à 5 ou transformés en amide -$(CH_2)_q$-CONR″R‴ comme indiqué précédemment.

Les composés de formule (I) dans lesquels A représente le groupement $(CH_2)_qL$, L étant un halogène et q un nombre entier de 1 à 5, pourront être synthétisés par traitement des dérivés de formule (I) dans lesquels A représente le groupement $(CH_2)_qOH$ par des agents d'halogénations tels que le chlorure de thionyle, l'oxychlorure de phosphore ou le tribromure de phosphore par exemple.

Dans le cas où $R_4$ possède une fonction non compatible avec ces suites de réaction, on utilisera comme produit de transformation le dérivé de formule (XII), V étant ensuite transformé en fonction $R_4$ comme il a été mentionné ci-dessus.

Des sels d'addition de certains composés de formule (I) peuvent s'obtenir, en particulier des sels d'addition pharmaceutiquement acceptables. On peut citer en particulier lorsque $R_2$, $R_4$ ou A présentent une fonction acide les sels de sodium, potassium, calcium, d'amine comme la dicyclohexylamine ou d'amino acide comme la lysine. Lorsque A ou $R_4$ présentent une fonction amine, un sel d'acide minéral ou organique comme chlorhydrate, méthane sulfonate, acétate, maléate, succinate, fumarate, sulfate, lactate ou citrate, par exemple.

Les nouveaux composés selon l'invention possèdent des propriétés pharmacologiques remarquables comme antagonistes des récepteurs à l'angiotensine II et peuvent être utilisés en thérapeutique pour le traitement de maladies cardiovasculaires en particulier pour traiter l'hypertension et l'insuffisance cardiaque.

Ainsi, l'invention s'étend aux compositions pharmaceutiques renfermant comme principe actif, les médicaments constitués par une quantité pharmaceutiquement efficace d'au moins un composé de formule (I), tel que précédemment défini, ainsi qu'éventuellement un de ses sels d'addition pharmaceutiquement acceptable.

Ces compositions peuvent être administrées par voie buccale, rectale, par voie parentérale, par voie transdermique ou par voie oculaire.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les systèmes transdermiques et les collyres : elles sont préparées selon les méthodes usuelles. Le principe actif, constitué par une quantité pharmaceutiquement efficace d'au moins un composé de formule (I) défini comme ci-dessus ou un de ses sels d'addition pharmaceutiquement acceptable peut y être incorporé à des excipients habituellement employés dans ces compositions pharmaceutiques, tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végétale, les glycols, les divers agents mouillants, dispersants ou émulsifiants, les gels de silicone, certains polymères ou copolymères, les conservateurs, arômes et colorants.

L'invention couvre encore une composition pharmaceutique à activité antagoniste des récepteurs à l'angiotensine II permettant notamment de traiter favorablement les maladies cardiovasculaires, en particulier l'hypertension et l'insuffisance cardiaque caractérisée en ce qu'elle comprend une quantité pharmaceutiquement efficace d'au moins un composé de formule (I) précité ou un de ses sels d'addition pharmaceutiquement acceptable pouvant être incorporé dans un excipient, véhicule ou support pharmaceutiquement acceptable.

La posologie varie notamment en fonction de la voie d'administration, de l'affection traitée et du sujet en cause.

Par exemple, chez l'adulte de poids moyen 60 à 70 kg, elle peut varier entre 1 et 400 mg de principe actif en une ou plusieurs doses journalières par voie orale, ou de 0,01 à 50 mg en une ou plusieurs doses journalières par voie parentérale.

L'invention couvre encore un procédé de préparation d'une composition pharmaceutique, caractérisé en ce qu'on incorpore une quantité pharmaceutiquement efficace d'au moins un composé de formule (I) tel que défini précédemment, ou un de ses sels d'addition pharmaceutiquement acceptable, dans un excipient, véhicule ou support pharmaceutiquement acceptable. Selon une caractéristique particulière, cette composition pharmaceutique est formulée sous forme de gélules, de comprimés dosés de 1 à 400 mg ou sous forme de préparations injectables dosées de 0,01 à 50 mg.

L'invention couvre encore un procédé de traitement thérapeutique des mammifères, caractérisé en ce qu'on administre à ce mammifère une quantité thérapeutiquement efficace d'au moins un composé de formule (I) tel que précédemment défini ou un de ses sels d'addition pharmaceutiquement acceptable.

En thérapeutique animale, la dose journalière utilisable devrait habituellement se situer de 1 à 100 mg par kg.

D'autres caractéristiques et avantages de l'invention seront mieux compris à la lecture qui va suivre de quelques exemples de préparation nullement limitatifs, mais donnés à titre d'illustration.

Exemple 1 : **oxo-3 heptanoate d'éthyle**

**Formule (II)** : $R_1$= n-Butyl $R_{10}$ = Ethyl

70g d'acide de Meldrum sont dissous dans 200ml de dichlorométhane en présence de 78,5 ml de pyridine, le mélange est refroidi à 0°C et 64,5g de chlorure d'acide de l'acide valérique sont ajoutés goutte à goutte à cette température. A la fin de l'addition le mélange est abandonné à température ambiante et agité pendant deux heures. La solution est lavée avec une solution d'acide chlorhydrique dilué, séchée sur sulfate de magnésium et évaporée sous vide pour donner 110g d'une huile utilisée telle quelle pour la suite. Cette huile est dissoute dans 400 ml d'éthanol absolu et le mélange est chauffé au reflux durant deux heures et abandonné une nuit à température ambiante. L'éthanol est évaporé sous vide et le résidu huileux est distillé à pression réduite pour donner 63,3g d'oxo-3 heptanoate d'éthyle sous forme d'un liquide de point d'ébullition $E_{b20}$ = 115-120°C.

Exemple 2 : **oxo-3 hexanoate d'éthyle**

**Formule (II)** : $R_1$ = n-Propyl, $R_{10}$ = Ethyl

Préparé selon le même mode opératoire que l'exemple 1.

Liquide de point d'ébullition $E_{b20}$ = 95-100°C

Exemple 3 : **(nitro-4 benzyl)-2 oxo-3 heptanoate d'éthyle**

**Formule (V)** : $R_1$ = n-Butyl, V = $NO_2$, $R_{10}$ = Ethyl

57,3g d'oxo-3 heptanoate d'éthyle sont dissous dans 300 ml d'éthanol. Une solution d'éthylate de sodium préparée par addition de 7,7g de sodium dans 50 ml d'éthanol est ajoutée et le mélange est agité durant 20 minutes à température ambiante. 72g de bromure de nitro-4 benzyle sont alors ajoutés par portions et le mélange est ensuite agité deux heures à température ambiante puis deux heures au reflux. L'éthanol est évaporé sous vide et le résidu obtenu est repris à l'eau puis extrait au chloroforme. La phase organique est séchée sur sulfate de magnésium et concentrée sous vide. L'huile obtenue est reprise dans un mélange ether pentane et les cristaux formés sont essorés afin d'éliminer le dérivé dibenzylé (F = 135°C), les eaux mères concentrées sous vide à 120°C pour éliminer le céto-ester non substitué de départ, donnent 69,2g de (nitro-4 benzyl)-2 oxo-3 heptanoate d'éthyle sous forme d'une huile utilisée telle quelle pour la suite.

Exemple 4 : **(nitro-4 benzyl)-2 oxo-3 hexanoate d'éthyle**

**Formule (V)** : $R_1$ = n-Propyl, V = $NO_2$, $R_{10}$ = Ethyl

Préparé selon le mode opératoire de l'exemple 3 à partir de l'oxo-3 hexanoate d'éthyle préparé à l'exemple 2.

Huile utilisée telle quelle pour la suite.

Exemple 5 : **(Méthoxy carbonyl-2' biphényl yl-4)méthyl-2 oxo-3 heptanoate d'éthyle**

**Formule (V)** : $R_1$ = Butyl , V = 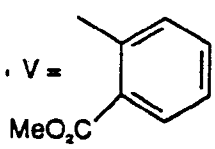 , $R_{10}$ = Ethyl

Préparé selon le mode opératoire de l'exemple 3 à partir de l'oxo-3 heptanoate d'éthyle préparé à l'exemple 1 et du (bromométhyl-4' biphényl-yl-2) carboxylate de méthyle.

Huile utilisée telle quelle pour la suite.

Préparation du (bromométhyl-4' biphényl-yl-2) carboxylate de méthyle

A) (Méthyl-4' biphényl-yl-2) carboxylate de méthyle.

A 300 ml de méthanol refroidi à 0°C, sont ajoutés 15 ml de chlorure d'acétyle. Le mélange est agité 10 minutes à cette température puis 15g d'acide (Méthyl-4' biphényl-yl-2) carboxylique (préparé selon MEYERS.A.I ; MIHELICH E.D., J.Am.Chem.Soc, 1975, 97 (25), 7383 par action du bromure de (méthyl-4 phényl) magnésium sur la (méthoxy-2 phényl)-2 diméthyl-4,4-oxazolidine-1,3) sont ajoutés. Le mélange est alors chauffé au reflux pendant 4 heures, les solvants sont évaporés sous vide pour donner 16g de (méthyl-4' biphényl-yl-2) carboxylate de méthyle sous forme d'une huile utilisée telle quelle pour la suite.

B) (bromométhyl-4' biphényl-yl-2) carboxylate de méthyle.

16g de (méthyl-4' biphényl-yl-2) carboxylate de méthyle préparés en A) sont dissous dans 120 ml de tetrachlorure de carbone en présence de 12,6g de N-bromo succinimide et de 0,5g de peroxyde de benzoyle. Le mélange est porté au reflux durant 6 heures, les cristaux sont filtrés et la solution restante est lavée avec une solution de bicarbonate de sodium puis évaporée sous vide. Le résidu est repris à l'éther puis la solution est filtrée sur charbon et évaporée sous vide pour donner 14,5g de (bromométhyl-4' biphényl-yl-2) carboxylate de méthyle sous forme d'une huile utilisée telle quelle pour la suite.

Exemple 6 : **(Cyano-2' biphényl-yl-4) méthyl-2 oxo-3 heptanoate d'éthyle**

**Formule (V)** : $R_1$ = Butyl , V = 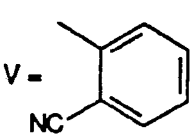 , $R_{10}$ = Ethyl

30 g d'oxo-3 heptanoate d'éthyle, préparé à l'exemple 1 sont dissous dans 300 ml de tétrahydrofurane. 31,6 g de bromométhyl-4' cyano-2 biphényle sont ajoutés ainsi que 40 ml de N,N-diisopropyl amine et 10 g de bromure de Lithium. Le mélange est chauffé 15 heures au reflux, puis concentré sous vide et additionné d'eau glacée et d'acide chlorhydrique dilué avant d'être extrait à l'acétate d'éthyle. La phase organique est lavée à l'eau puis séchée et évaporée sous vide. Le résidu obtenu est chauffé sous vide à 130°C afin d'en retirer l'oxo-3 heptanoate d'éthyle résiduel, pour donner 41 g de (cyano-2' biphényl-yl-4) méthyl-2 oxo-3 heptanoate d'éthyle brut sous forme d'une huile utilisée telle quelle pour la suite.

Préparation du bromométhyl-4' cyano-2 biphényl :

A) Méthyl-4' cyano-2 biphényl :

18,5g d'acide (méthyl-4' biphényl-yl-2) carboxylique préparé comme à l'exemple 5 A), sont chauffés au reflux dans 60 ml de chlorure de thionyle pendant deux heures. Le chlorure de thionyle est concentré sous vide et le résidu est versé sur une solution à 28% d'hydroxyde d'ammonium, le mélange est agité pendant 30 minutes et les cristaux obtenus sont essorés et lavés à l'éther puis séchés pour donner 14,5g de (méthyl-4' biphényl-yl-2) carboxamide sous forme de cristaux de point de fusion 128°C. Ces cristaux sont repris dans 50 ml de chlorure de thionyle et le mélange est chauffé 3 heures au reflux puis concentré sous vide pour donner 9g de méthyl-4' cyano-2 biphényl sous forme de cristaux de point de fusion 45-46°C.

B) Bromométhyl-4' cyano-2 biphényl :

7,9g de méthyl-4' cyano-2 biphényl préparé en A) sont dissous dans 100 ml de tétrachlorure de carbone en présence de 7,3g de N-bromo succinimide et de 0,3g de peroxyde de benzoyle. Le mélange est chauffé au reflux pendant 6 heures et les cristaux sont filtrés, la solution restante est concentrée sous vide et le résidu est cristallisé dans l'éther pour donner 6,6g de bromométhyl-4' cyano-2 biphényl sous forme de cristaux de point de fusion 115-118°C.

Exemple 7 : **Méthyl-1 n-Butyl-3 (nitro-4 benzyl)-4 hydroxy-5 pyrazole**

**Formule (IX)** : $R_1$ = n-Butyl, $R_2 = CH_3$, V = $NO_2$

20g de (nitro-4 benzyl)-2 oxo-3 heptanoate d'éthyle préparé à l'exemple 3 sont dissous dans 150 ml d'éthanol et 4 ml de méthyl hydrazine sont ajoutés. Le mélange est chauffé au reflux pendant 6 heures. L'éthanol est évaporé sous vide et le résidu est repris à l'eau puis extrait à l'acétate d'éthyle, la phase organique est alors lavée plusieurs fois avec une solution de soude diluée et les fractions aqueuses combinées sont acidifiées par le dioxyde de soufre, puis extraites au chloroforme. La phase chloroformique est séchée sur sulfate de magnésium et évaporée sous vide pour donner un résidu qui cristallise dans l'éther. Les cristaux sont essorés et recristallisés dans l'acétate d'éthyle pour donner 10,9g de méthyl-1 n-Butyl-3 (nitro-4 benzyl)-4 hydroxy-5 pyrazole sous forme de cristaux de point de fusion 136°C.

Exemple 8 : **Méthyl-1 n-Propyl-3 (nitro-4 benzyl)-4 hydroxy-5 pyrazole**

**Formule (IX)** : $R_1$ = n-Propyl, $R_2 = CH_3$, V = $NO_2$

Préparé selon le mode opératoire de l'exemple 7 à partir du (nitro-4 benzyl)-2 oxo-3 hexanoate d'éthyle préparé à l'exemple 4.

Cristaux de point de fusion 174°C.

Exemple 9 : **n-Propyl-3 (nitro-4 benzyl)-4 hydroxy-5 pyrazole**

**Formule (IX)** : $R_1$ = n-Propyl, $R_2$ = H, V = $NO_2$

Préparé selon le mode opératoire de l'exemple 7 à partir du (nitro-4 benzyl)-2 oxo-3 hexanoate d'éthyle préparé à l'exemple 4 et de l'hydrazine.

Cristaux de point de fusion 196°C.

Exemple 10 : **(Ethoxy carbonyl méthyl)-1 n-propyl-3 (nitro-4 benzyl)-4 hydroxy-5 pyrazole**

**Formule (IX)** : $R_1$ = n-Propyl, $R_2 = CH_2CO_2Et$, V = $NO_2$

Préparé selon le mode opératoire de l'exemple 7 à partir de l'hydrazinoacétate d'éthyle.

Cristaux de point de fusion 134°C.

Exempe 11 : **(Trifluoro-2,2,2 éthyl)-1 n-propyl-3 (nitro-4 benzyl)-4 hydroxy-5 pyrazole**

**Formule (IX)** : $R_1$ = n-Propyl, $R_2 = CH_2CF_3$, V = $NO_2$

Préparé selon le mode opératoire de l'exemple 7 à partir de la (trifluoro-2,2,2 éthyl) hydrazine.

Cristaux de point de fusion 160°C.

Exemple 12 : **Méthyl-1 n-Butyl-3 (méthoxy carbonyl-2' biphényl-yl-4) méthyl-4 hydroxy-5 pyrazole**

**Formule (IX)** : $R_1$ = n-Butyl,

$R_2 = CH_3$ , V =

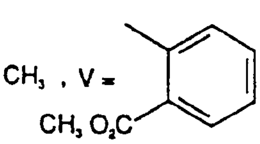

Préparé selon le mode opératoire de l'exemple 7 à partir du (méthoxy carbonyl-2' biphényl-yl-4) méthyl-2 oxo-3 heptanoate d'éthyle préparé à l'exemple 5.

Cristaux de point de fusion 108°C.

Exemple 13 : **Méthyl-1 n-Butyl-3 (cyano-2' biphényl-yl-4) méthyl-4 hydroxy-5 pyrazole**

**Formule (IX)** : $R_1$ = n-Butyl, $R_2 = CH_3$ ,

V =
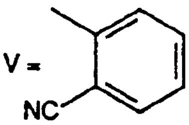

Préparé selon le mode opératoire de l'exemple 7 à partir du (cyano-2' biphényl-yl-4) méthyl-2 oxo-3 heptanoate d'éthyle préparé à l'exemple 6.

Cristaux de point de fusion 138°C.

Exemple 14 : **[Méthyl-1 n-propyl-3 (nitro-4 benzyl)-4 pyrazol-yl-5] oxy acétate d'éthyle**

**Formule (XI)** : $R_1$ = n-Propyl, $R_2$ = Méthyl, $R_3 = CH_2CO_2Et$, $V = NO_2$

22,4g de méthyl-1 n-propyl-3 (nitro-4 benzyl)-4 hydroxy-5 pyrazole préparé à l'exemple 8 sont dissous dans 200 ml d'acétone et 8,7g de carbonate de sodium sont ajoutés ainsi que 9,2 ml de bromo acétate d'éthyle. Le mélange est porté au reflux pendant 5 heures et les solvants sont concentrés à sec. Le résidu est repris à l'eau puis extrait à l'éther. La phase organique est séchée sur sulfate de magnésium et évaporée à sec. Le résidu obtenu est repris à l'éther isopropylique et les cristaux obtenus sont essorés pour donner 9g de [méthyl-1 n-propyl-3 (nitro-4 benzyl)-4 oxo-5 pyrazol-yl-2] acétate d'éthyle de point de fusion 62°C.

Les eaux mères sont concentrées et l'huile obtenue est chromatographiée sur gel de silice dans un éluant dichlorométhane/acétone (90/10) pour donner 10g de [méthyl-1 n-propyl-3 (nitro-4 benzyl)-4 pyrazol-yl-5] oxyacétate d'éthyle sous forme de cristaux de point de fusion 60-61°C.

Exemple 15: **[Méthyl-1 n-Butyl-3 (nitro-4 benzyl)-4 pyrazol-yl-5] oxyacétate d'éthyle**

**Formule (XI)** : $R_1$ = n-Butyl, $R_2$ = Méthyl, $R_3 = CH_2\ CO_2\ Et$, $V = NO_2$

Préparé selon le mode opératoire de l'exemple 14.

Cristaux de point de fusion 68°C.

Exemple 16 : **[n-propyl-3 (nitro-4 benzyl)-4 pyrazol-yl-5] oxyacétate d'éthyle**

**Formule (XI)** : $R_1$ = n-Propyl, $R_2$ = H, $R_3 = CH_2\ CO_2\ Et$, $V = NO_2$

Préparé selon le mode opératoire de l'exemple 14.

Cristaux de point de fusion 116°C.

Exemple 17 : **[Méthyl-1 n-propyl-3 (nitro-4 benzyl)-4 pyrazol-yl-5] oxy acétate de méthyle**

**Formule(XI)** : $R_1$ = n-Propyl, $R_2$ = méthyl, $R_3 = CH_2\ CO_2\ Me$, $V = NO_2$

Préparé selon le mode opératoire de l'exemple 14.

Cristaux de point de fusion 58°C.

Exemple 18 : **[(trifluoro-2,2,2 éthyl)-1 n-propyl-3 (nitro-4 benzyl)-4 pyrazol-yl-5] oxyacétate de méthyle**

**Formule (XI)**: $R_1$ = n-Propyl, $R_2 = CH_2\ CF_3$, $R_3 = CH_2\ CO_2\ Me$, $V = NO_2$

Préparé selon le mode opératoire de l'exemple 14.

Cristaux de point de fusion 73°C.

Exemple 19 : **[Méthyl-1 n-butyl-3 (méthoxy carbonyl-2' biphényl-yl-4) méthyl-4 pyrazol-yl-5] oxyacétate d'éthyle**

**Formule (XI)** : $R_1$ = n-Butyl, $R_2$ = Méthyl,

$R_3 = CH_2\ CO_2\ Et$, V =
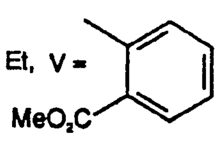

Préparé selon le mode opératoire de l'exemple 14.
Huile utilisée telle quelle pour la suite.

Exemple 20 : **[Méthyl-1 n-butyl-3 (cyano-2' biphényl-yl-4)méthyl-4 pyrazol-yl-5] oxyacétate d'éthyle**

**Formule (XI)** : $R_1$ = n-Butyl, $R_2$ = Méthyl, $R_3$ = $CH_2$ $CO_2$ Et,

V =
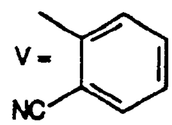

Préparé selon le mode opératoire de l'exemple 14.
Huile utilisée telle quelle pour la suite.

Exemple 21 : **[Méthyl-1 n-butyl-3 (amino-4 benzyl)-4 pyrazol-yl-5] oxy acétate d'éthyle**

**Formule (I)** : $R_1$ = n-Butyl, $R_2$ = Méthyl, A = $OR_3$, $R_3$ = $CH_2$ $CO_2$ Et, $R_4$ = $NH_2$

3,4g de [méthyl-1 n-butyl-3 (nitro-4 benzyl)-4 pyrazol-yl-5] oxyacétate d'éthyle préparé à l'exemple 15 sont dissous dans 50 ml d'éthanol absolu en présence de 500 mg de Nickel de Raney. Le mélange est hydrogéné à pression atmosphérique et à température ambiante et quand la prise d'hydrogène a cessé, le catalyseur est filtré, l'éthanol est évaporé sous vide et le résidu est repris au pentane pour donner 2,9g de [méthyl-1 n-butyl-3 (amino-4 benzyl)-4 pyrazol-yl-5] oxyacétate d'éthyle sous forme de cristaux de point de fusion 65°C.

Exemple 22 : **[Méthyl-1 n-propyl-3 (amino-4 benzyl)-4 pyrazol-yl-5] oxy acétate d'éthyle**

**Formule (I)** : $R_1$ = n-Propyl, $R_2$ = Méthyl, A = $OR_3$, $R_3$ = $CH_2$ $CO_2$ Et, $R_4$ = $NH_2$
Préparé selon le mode opératoire de l'exemple 21.
Cristaux de point de fusion 103°C.

Exemple 23 : **[Méthyl-1 n-propyl-3 (amino-4 benzyl)-4 pyrazol-yl-5] oxy acétate de méthyle**

**Formule (I)** : $R_1$ = n-Propyl, $R_2$ = Méthyl, A = $OR_3$,$R_3$= $CH_2$ $CO_2$ Me, $R_4$ = $NH_2$
Préparé selon le mode opératoire de l'exemple 21.
Huile utilisée telle quelle pour la suite.

Exemple 24 : **[(trifluoro-2,2,2 éthyl)-1 n-propyl-3 (amino-4 benzyl)-4 pyrazol-yl-5] oxyacétate de méthyle**

**Formule (I)** : $R_1$ = n-Propyl, $R_2$ = $CH_2$ $CF_3$, A = $OR_3$, $R_3$ = $CH_2$ $CO_2$ Me, $R_4$ = $NH_2$
Préparé selon le mode opératoire de l'exemple 21.
Cristaux de point de fusion 63°C.

Exemple 25 : **(Ethoxy carbonyl méthyl)-1 n-propyl-3 (amino-4 benzyl)-4 hydroxy-5 pyrazole**

**Formule (I)** : $R_1$ = n-Propyl, $R_2$ = $CH_2$ $CO_2$ Et, A = $OR_3$, $R_3$ = H, $R_4$ = $NH_2$
Préparé selon le mode opératoire de l'exemple 21.
Huile utilisée telle quelle pour la suite.

Exemple 26 : **[n-propyl-3 (amino-4 benzyl)-4 pyrazol-yl-5] oxyacétate d'éthyle**

**Formule (I)** : $R_1$ = n-Propyl, $R_2$ = H, A = $OR_3$, $R_3$ = $CH_2$ $CO_2$ Et, $R_4$ = $NH_2$
Préparé selon le mode opératoire de l'exemple 21.
Huile utilisée telle quelle pour la suite.

<u>Exemple 27</u> : **[Méthyl-1 n-butyl-3 ((carboxy-2 benzoyl amino)-4 benzyl)-4 pyrazol-yl-5] oxyacétate d'éthyle**

**Formule (I)** : $R_1$ = n-butyl, $R_2$ = Méthyl, A = $OR_3$ $R_3$ = $CH_2$ $CO_2$ Et,

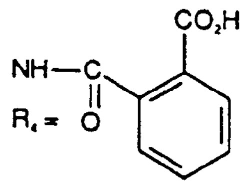

3g de [Méthyl-1 n-butyl-3 (amino-4 benzyl)-4 pyrazol-yl-5] oxyacétate d'éthyle, préparé à l'exemple 21, sont dissous dans 50ml d'acétonitrile. 1,3g d'anhydride phtalique sont ajoutés et le mélange est abandonné à température ambiante pendant une nuit. Les cristaux obtenus sont essorés, lavés à l'ether isopropylique et séchés pour donner 2,5g de [Méthyl-1 n-butyl-3 ((carboxy-2 benzoyl amino)-4 benzyl)-4 pyrazol-yl-5] oxyacétate d'éthyle sous forme de cristaux de point de fusion 140-141°C.

Selon le même mode opératoire nous avons préparé les exemples suivants:

<u>Exemple 28</u> : **[Méthyl-1 n-propyl-3 ((carboxy-2 benzoyl amino)-4 benzyl)-4 pyrazol-yl-5] oxyacétate de méthyle**

**Formule (I)** : $R_1$ = n-Propyl, $R_2$ = $CH_3$, A = $OR_3$, $R_3$ = $CH_2CO_2Me$,

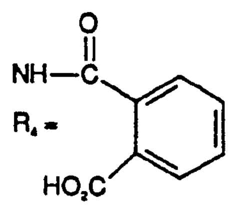

Cristaux, sous forme de sel de dicyclohexylamine, de point de fusion 173-174°C.

<u>Exemple 29</u> : **[Méthyl-1 n-propyl-3 ((carboxy-2 benzoyl amino)-4 benzyl)-4 pyrazol-yl-5] oxyacétate d'éthyle**

**Formule (I)** : $R_1$ = n-Propyl, $R_2$ = $CH_3$, A = $OR_3$, $R_3$ = $CH_2$ $CO_2$ Et,

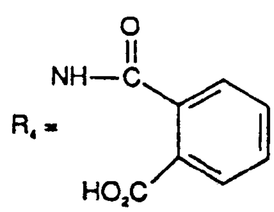

Cristaux de point de fusion 139-140°C.

<u>Exemple 30</u> : **[Méthyl-1 n-propyl-3 ((carboxy-2 dichloro-3,6- benzoyl amino)-4 benzyl)-4 pyrazol-yl-5] oxyacétate d'éthyle**

**Formule (I)** : $R_1$ = n-Propyl, $R_2$ = $CH_3$, A = $OR_3$ $R_3$ = $CH_2$ $CO_2$ Et,

$R_4$ = 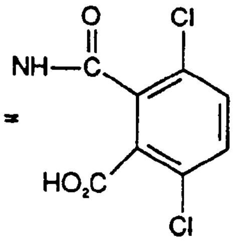

A partir de l'anhydride dichloro-3,6 phtalique.
Cristaux sous forme de sel de dicyclohéxylamine, de point de fusion 199-200°C.

Exemple 31 : **[Méthyl-1 n-propyl-3 ((carboxy-2 dichloro-3,6- benzoyl amino)-4 benzyl)-4 pyrazol-yl-5] oxyacétate de méthyle**

**Formule (I)** : $R_1$ = n-Propyl, $R_2$ = Méthyl, A = $OR_3$, $R_3$ = $CH_2CO_2Me$,

$R_4$ = 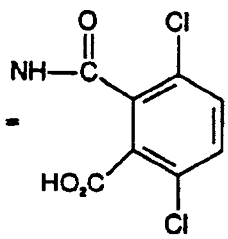

A partir de l'anhydride dichloro-3,6 phtalique.
Cristaux de point de fusion 150-151°C.

Exemple 32 : **[(trifluoro-2,2,2 éthyl)-1 n-propyl-3 ((carboxy-2 dichloro-3,6-benzoyl amino)-4 benzyl)-4 pyrazol-yl-5] oxyacétate de méthyle**

**Formule (I)** : $R_1$ = n-Propyl, $R_2$ = $CH_2$ $CF_3$, A = $OR_3$, $R_3$ = $CH_2CO_2Me$,

$R_4$ = 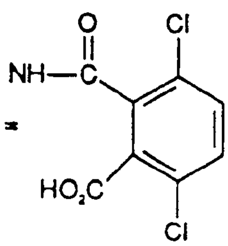

A partir de l'anhydride dichloro-3,6 phtalique.
Cristaux de point de fusion 169-170°C.

Exemple 33 : **[(trifluoro-2,2,2 éthyl)-1 n-propyl-3 ((carboxy-2 benzoyl amino)-4 benzyl)-4 pyrazol-yl-5] oxyacétate de méthyle**

**Formule (I)** : $R_1$ = n-Propyl, $R_2$ = $CH_2$ $CF_3$, A = $OR_3$, $R_3$ = $CH_2CO_2Me$,

$R_4$ = 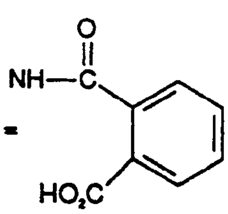

Cristaux de point de fusion 189-192°C.

Exemple 34 : **Acide [n-propyl-3 ((carboxy-2 benzoyl amino)-4 benzyl)-4 pyrazol-yl-5] oxyacétique**

**Formule (I)** : $R_1$ = n-Propyl, $R_2$ = H, A = $OR_3$, $R_3$ = $CH_2CO_2H$ ,

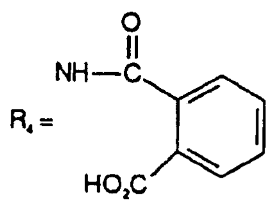

0,9 g de [n-propyl-3 (amino-4 benzyl)-4 pyrazolyl-5] oxyacétate d'éthyle préparé à l'exemple 26 sont dissous dans 20ml d'acétonitrile en présence de 0,45g d'anhydride phtalique. Le mélange est abandonné à température ambiante durant une nuit. L'acétonitrile est évaporé sous vide et le résidu est repris dans un mélange acétate d'éthyle/ether pour donner des cristaux que l'on essore. Ces cristaux sont dissous dans du dichlorométhane et la solution est lavée plusieurs fois avec une solution de soude 1N. Les phases aqueuses combinées sont acidifiées par barbotage de dioxide de soufre et les cristaux obtenus sont essorés et séchés pour donner 0,5g d'acide [n-propyl-3 ((carboxy-2 benzoyl amino)-4 benzyl)-4 pyrazol-yl-5] oxyacétique sous forme de cristaux de point de fusion 170-171°C.

Exemple 35 : **Acide [Méthyl-1 n-propyl-3 [(sulfo-2 benzoyl amino)-4 benzyl]-4 pyrazol-yl-5] oxy acétique**

**Formule (I)** : $R_1$ = n-Propyl, $R_2$ = $CH_3$, A = $OR_3$, $R_3$ = $CH_2CO_2H$,

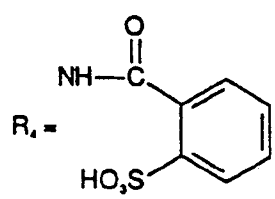

Préparé selon le mode opératoire de l'exemple 34 à partir de l'anhydride cyclique de l'acide orthosulfobenzoïque.

Cristaux de point de fusion 168-170°C.

Exemple 36 : **Acide [Méthyl-1 n-butyl-3 hydroxy-5 pyrazol-yl-4] méthyl-4' biphényl-2 carboxylique**

**Formule (I)** : $R_1$ = n-Butyl, $R_2$ = Méthyl, A = $OR_3$, $R_3$ = H,

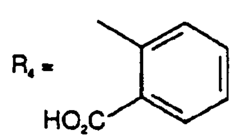

1,5g de Méthyl-1 n-butyl-3 hydroxy-5 (méthoxycarbonyl-2' biphényl-yl-4) méthyl-4 pyrazole préparé à l'exemple 12 sont mis en suspension dans 15ml d'une solution de soude 1N et agités une heure à 40°C. La solution est lavée au dichlorométhane puis acidifiée par barbotage de dioxyde de soufre et extraite au dichlorométhane. La phase organique est séchée sur sulfate de magnésium puis évaporée sous vide. Le résidu est dilué dans l'acétate d'éthyle et extrait avec une solution de bicarbonate de sodium puis la phase aqueuse est acidifiée par le dioxyde de soufre et les cristaux obtenus sont essorés puis séchés pour donner 1,1 g d'acide [Méthyl-1 n-butyl-3 hydroxy-5 pyrazol-yl-4] méthyl-4' biphényl-2 carboxylique sous forme de cristaux de point de fusion 228-30°C.

Exemple 37 : **Acide [Méthyl-1 n-butyl-3 (carboxy-2' biphényl-yl-4) méthyl-4 pyrazol-yl-5] oxyacétique**

**Formule (I)** : $R_1$ = n-Butyl, $R_2$ =$CH_3$, A = $OR_3$, $R_3$ = $CH_2CO_2H$,

$R_4$ =

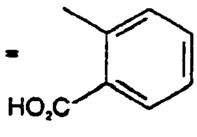

2,6g de [Méthyl-1 n-butyl-3 (méthoxycarbonyl-2' biphényl-yl-4) méthyl-4 pyrazol-yl-5] oxyacétate d'éthyle préparé à l'exemple 19 sont dissous dans 30ml d'éthanol. 1g de soude en pastille est rajouté ainsi que 10ml d'eau et le mélange est agité à température ambiante durant deux heures puis chauffé 3 heures à 50-55°C. Après refroidissement la solution est diluée à l'eau et lavée à l'ether, la phase aqueuse est acidifiée par barbotage de dioxyde de soufre et extraite au chloroforme. La phase organique est séchée sur sulfate de magnésium et évaporée sous vide pour donner un résidu qui cristallise dans un mélange acétate d'éthyle/ether en donnant 2g d'acide [Méthyl-1 n-butyl-3 (carboxy-2' biphényl-yl-4) méthyl-4 pyrazol-yl-5] oxyacétique sous forme de cristaux de point de fusion 153-154°C.

Exemple 38 : **Méthyl-1 n-butyl-3 (nitro-4 benzyl)-4 méthoxy carbonyl oxy-5 pyrazole**

**Formule (I)** : $R_1$ = n-Butyl, $R_2$ =Méthyl, A = $OR_3$,

$R_3$ = 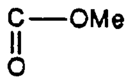 , $R_4$ = $NO_2$

12 g de Méthyl-1 n-butyl-3 (nitro-4 benzyl)-4 hydroxy-5 pyrazole préparé à l'exemple 7 sont dissous dans 120ml de dichloro-1,2-éthane en présence de 6ml de triéthylamine. 3,3g de chloroformiate de méthyle sont ajoutés goutte à goutte et le mélange est agité 2 heures à température ambiante puis 4 heures au reflux. La solution est lavée à l'eau après refroidissement, puis elle est séchée sur sulfate de magnésium et concentrée sous vide. Le résidu huileux est chromatographié sur gel de silice dans un éluant dichlorométhane/acétone (95/5) pour donner 6,6g de Méthyl-1 n-butyl-3 (nitro-4 benzyl)-4 méthoxy carbonyl oxy-5 pyrazole sous forme de cristaux de point de fusion 44-47°C.

Exemple 39 : **Méthyl-1 n-butyl-3 (amino-4 benzyl)-4 méthoxy carbonyl oxy-5 pyrazole**

**Formule (XI)** : $R_1$ = n-Butyl, $R_2$ =Méthyl

$R_3$ = 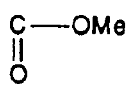 , V = $NH_2$

Préparé selon le mode opératoire de l'exemple 21.
Huile utilisée telle quelle pour la suite.

Exemple 40 : **Acide [[Méthyl-1 n-butyl-3 hydroxy-5 pyrazolyl-4]méthyl phényl-yl-4] amino carbonyl-2 dichloro-3,6 benzoïque**

**Formule (I)** : $R_1$ = n-Butyl, $R_2$ =Méthyl, A= $OR_3$, $R_3$ = H ,

$R_4$ = 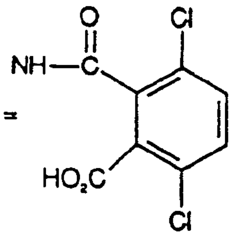

Préparé selon le mode opératoire de l'exemple 27 à partir de l'anhydride dichloro-3,6 phtalique. Cristaux de point de fusion 172-174°C.

<u>Exemple 41</u> : **Acide [[éthoxy carbonyl méthyl-1 n-propyl-3 hydroxy-5 pyrazol-yl-4] méthyl phényl-yl-4] amino carbonyl-2 dichloro-3,6 benzoïque**

**Formule (I)** : $R_1$ = n-propyl, $R_2 = CH_2\ CO_2$ Et, A = $OR_3$, $R_3$ = H ,

$R_4$ = 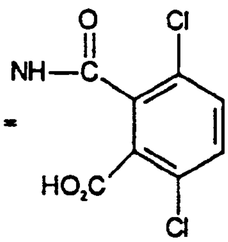

Préparé selon le mode opératoire de l'exemple 27 à partir de l'anhydride dichloro-3,6 phtalique. Cristaux de point de fusion 150-153°C.

<u>Exemple 42</u> : **[éthoxy carbonyl méthyl-1 n-propyl-3 (nitro-4 benzyl)- 4 pyrazol-yl-5] oxyacétate d'éthyle**

**Formule (XI)** : $R_1$ = n-propyl, $R_2 = CH_2\ CO_2$ Et, $R_3 = CH_2\ CO_2$ Et, V = $NO_2$
Préparé selon le mode opératoire de l'exemple 14.
Huile utilisée telle quelle pour la suite.

<u>Exemple 43</u> : **[Ethoxy carbonyl méthyl-1 n-propyl-3 (amino-4 benzyl)- 4 pyrazol-yl-5] oxyacétate d'éthyle**

**Formule (I)** : $R_1$ = n-propyl, $R_2 = CH_2\ CO_2$ Et, A = $OR_3$, $R_3 = CH_2\ CO_2$ Et, $R_4 = NH_2$
Préparé selon le mode opératoire de l'exemple 21.
Huile utilisée telle quelle pour la suite.

<u>Exemple 44</u> : **Acide [[(Ethoxy carbonyl méthyl)-1 n-propyl-3 (Ethoxy carbonyl méthyl oxy)- 5 pyrazol-yl-4] méthyl phényl-yl-4] amino carbonyl-2 dichloro-3,6- benzoïque**

**Formule (I)** : $R_1$ = n-propyl, $R_2 = CH_2\ CO_2$ Et, A = $OR_3$, $R_3 = CH_2CO_2Et$ ,

$R_4$ = 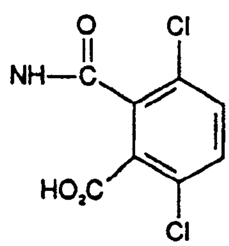

Préparé selon le mode opératoire de l'exemple 27 à partir de l'anhydride dichloro-3,6 phtalique. Cristaux de point de fusion 189-191°C. Sous forme de sel de dicyclohexylamine.

<u>Exemple 45</u> : **Acide [[Méthyl-1 n-propyl-3 (Ethoxy carbonyl méthyl oxy)- 5 pyrazol-yl-4] méthyl phényl-yl-4] amino carbonyl-2 benzène sulfonique.**

**Formule (I)** : $R_1$ = n-propyl, $R_2$ =Méthyl, A = $OR_3$, $R_3$ = $CH_2CO_2H$ ,

$R_4$ = 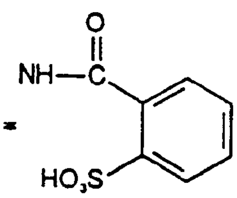

Préparé selon le mode opératoire de l'exemple 27 mais avec l'anhydride cyclique de l'acide orthosulfobenzoïque.

Cristaux de point de fusion 203-205°C.

<u>Exemple 46</u> : **N-[[Méthyl-1 n-butyl-3 (nitro-4 benzyl)- 4 pyrazol-yl-5] oxyacétyl] morpholine**

**Formule (XI)** : $R_1$ = n-butyl, $R_2$ = Méthyl,

$R_3$ = 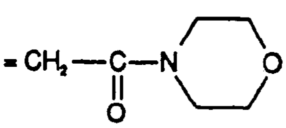 , V = $NO_2$

Préparé selon le mode opératoire de l'exemple 14 à partir de la N-(chloro acétyl) morpholine.

Cristaux de point de fusion 98°C.

Préparation de la N-(chloro acétyl) morpholine:

21,7g de morpholine sont dissous dans 250ml de dichlorométhane et la solution obtenue est refroidie par un mélange glace/eau.

14, 1 g de chlorure de chloroacétyle sont ajoutés goutte à goutte en maintenant la température à 0°C puis le mélange est agité 3 heures à température ambiante et lavé avec une solution diluée d'acide chlorhydrique. La phase organique est séchée sur sulfate de magnésium et évaporée sous vide pour donner 29,8g de N–(chloro acétyl) morpholine sous forme d'une huile de pureté suffisante pour la suite.

<u>Exemple 47</u> : **N-[[Méthyl-1 n-butyl-3 (amino-4 benzyl)- 4 pyrazol-yl-5] oxyacétyl] morpholine**

**Formule (I)** : $R_1$ = n-butyl, $R_2$ = Méthyl, A = $OR_3$,

$R_3$ = 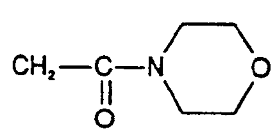 , $R_4$ = $NH_2$

Préparé selon le mode opératoire de l'exemple 21.

Cristaux de point de fusion 130°C.

<u>Exemple 48</u> : **N-[[Méthyl-1 n-butyl-3 [(sulfo-2 benzoyl amino)- 4 benzyl]-4 pyrazol-yl-5] oxyacétyl] morpholine**

**Formule (I)** : $R_1$ = n-butyl, $R_2$ = Méthyl, A = $OR_3$,

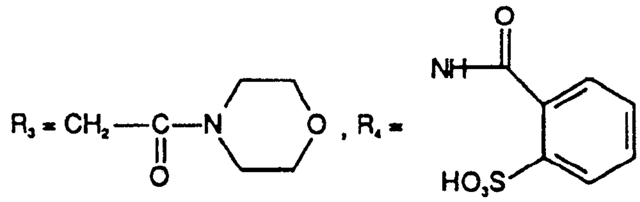

Préparé selon le mode opératoire de l'exemple 27 en utilisant l'anhydride cyclique de l'acide orthosulfobenzoïque.

Cristaux de point de fusion 236-237°C.

<u>Exemple 49</u> : **[Méthyl-1 n-butyl-3 (nitro-4 benzyl)-4 pyrazol-yl-5] oxy-2 éthanol**

**Formule (XI)**: $R_1$ = n-butyl, $R_2$ = Méthyl, $R_3 = CH_2CH_2OH$, $V = NO_2$

12g de méthyl-1 n-butyl-3 (nitro-4 benzyl)-4 hydroxy-5 pyrazole préparé à l'exemple 7 sont dissous dans 150ml de butanone-2. 4,6g de carbonate de sodium et 6 g de bromo-2 éthanol sont rajoutés et le mélange est chauffé 12 heures au reflux. Après refroidissement la solution est concentrée puis reprise à l'eau et le pH est rendu alcalin par addition de soude diluée. Après extraction au dichlorométhane la phase organique est séchée sur sulfate de magnésium puis évaporée à sec. Le résidu obtenu est chromatographié sur gel de silice dans un éluant acétate d'éthyle/acétone (6/4) pour donner 5g de [méthyl-1 n-butyl-3 (nitro-4 benzyl)-4 pyrazol-yl-5] oxy-2 éthanol sous forme de cristaux de point de fusion 81°C.

<u>Exemple 50</u> : **[Méthyl-1 n-butyl-3 (amino-4 benzyl)-4 pyrazol-yl-5] oxy-2 éthanol**

**Formule (I)** : $R_1$ = n-butyl, $R_2$ = Méthyl, $A = OR_3$, $R_3 = CH_2CH_2OH$, $R_4 = NH_2$

Préparé selon le mode opératoire de l'exemple 21.

Huile utilisée telle quelle pour la suite.

<u>Exemple 51</u> : **Acide [[Méthyl-1 n-butyl-3 (hydroxy-2 éthoxy)-5 pyrazol-yl-4] méthyl phényl-yl-4] amino carbonyl-2 benzène sulfonique**

**Formule (I)** : $R_1$ = n-butyl, $R_2$ = Méthyl, $A = OR_3$, $R_3 = CH_2 -CH_2 -OH$ ,

$R_4$ = 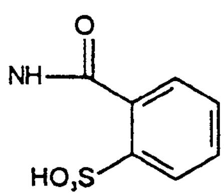

Préparé selon le mode opératoire de l'exemple 27 mais en utilisant l'anhydride cyclique de l'acide orthosulfobenzoïque.

Cristaux de point de fusion 170-171°C.

<u>Exemple 52</u> : **Méthyl-1 (nitro-4 benzyl)-4 n-Propyl-3 (N,N-diméthyl carbamoyl) oxy-5 pyrazole**

**Formule (XI)** : $R_1$ = n-Propyl, $R_2$ = Méthyl, $R_3$ = 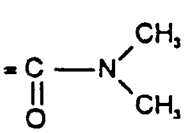 ,

$V = NO_2$

10 g de Méthyl-1 (nitro-4 benzyl)-4 n-Propyl-3 hydroxy-5 pyrazole, préparé à l'exemple 8, sont dissous dans 100 ml de dichlorométhane et 5 ml de triéthylamine. 3,2 ml de chlorure de N,N-diméthyl carbamoyle sont ajoutés goutte à goutte et le mélange est ensuite chauffé 10 heures au reflux. Après refroidissement le mélange est repris à l'eau et lavé avec une solution de bicarbonate de potassium. La phase chloroformique est séchée sur

sulfate de magnésium puis évaporée sous vide pour donner 9,1 g de Méthyl-1 (nitro-4 benzyl)-4 n-Propyl-3 (N,N-diméthyl carbamoyl) oxy-5 pyrazole sous forme de cristaux de point de fusion 90°C.

Exemple 53 : **Méthyl-1 (amino-4 benzyl)-4 n-Propyl-3 (N,N-diméthyl carbamoyl) oxy-5 pyrazole**

**Formule (I)** : $R_1$ = n-Propyl, $R_2$ = Méthyl, A = $OR_3$, $R_3$ = 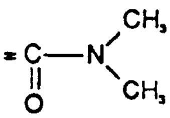

$R_4 = NH_2$

Préparé selon le mode opératoire de l'exemple 21.
Cristaux de point de fusion 138°C.

Exemple 54 : **Acide [[Méthyl-1 n-propyl-3 (N,N-diméthyl carbamoyl) oxy-5 pyrazol-yl-4] méthyl phényl-yl-4] aminocarbonyl-2 benzène sulfonique**

**Formule (I)** : $R_1$ = n-Propyl, $R_2$ = Méthyl, A = $OR_3$,

$R_3$ = 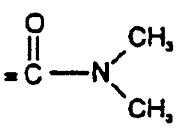 , $R_4$ = 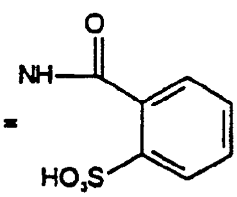

Préparé selon le mode opératoire de l'exemple 27 en utilisant l'anhydride cyclique de l'acide orthosulfobenzoïque.
Cristaux de point de fusion 210-2°C.

Exemple 55 : **Méthyl-1 n-Propyl-3 (nitro-4 benzyl)-4 (N,N-diéthyl carbamoyl) oxy-5 pyrazole**

**Formule (XI)** : $R_1$ = n-Propyl, $R_2$ = Méthyl, $R_3$ = 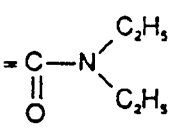

$V = NO_2$

Préparé selon le mode opératoire de l'exemple 52.
Huile utilisée telle quelle pour la suite.

Exemple 56 : **Méthyl-1 n-Propyl-3 (amino-4 benzyl)-4 (N,N-diéthyl carbamoyl) oxy-5 pyrazole**

**Formule (I)** : $R_1$ = n-Propyl, $R_2$ = Méthyl, A = $OR_3$, $R_3$ = 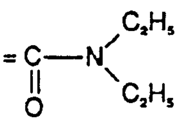

$R_4 = NH_2$

Préparé selon le mode opératoire de l'exemple 21.
Cristaux de point de fusion 100°C.

Exemple 57: **Acide [[Méthyl-1 n-Propyl-3 (N,N-diéthyl carbamoyl) oxy-5 pyrazol-yl-4] méthyl phényl-yl-4] amino carbonyl-2 benzène sulfonique**

**Formule (I)** : $R_1$ = n-Propyl, $R_2$ = Méthyl, A = $OR_3$,

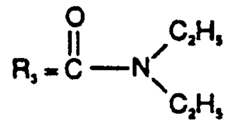 , 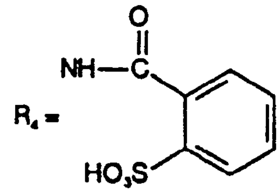

Préparé selon le mode opératoire de l'exemple 27 en utilisant l'anhydride cyclique de l'acide orthosulfobenzoïque.

Cristaux de point de fusion 216-217°C.

Exemple 58 : **(Trifluoro-2,2,2 éthyl)-1 n-Propyl-3 (N,N-diméthyl carbamoyl) oxy-5 (nitro-4 benzyl)-4 pyrazole**

Formule (XI) : $R_1$ = n-Propyl, $R_2$ = $CH_2CF_3$, $R_3$ = 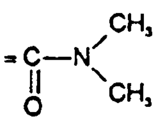

V = $NO_2$

Préparé selon le mode opératoire de l'exemple 52.

Cristaux de point de fusion 70°C.

Exemple 59 : **(Trifluoro-2,2,2 éthyl)-1 n-Propyl-3 (N,N-diméthyl carbamoyl) oxy-5 (amino-4 benzyl)-4 pyrazole**

**Formule (I)** : $R_1$ = n-Propyl, $R_2$ = $CH_2CF_3$, A = $OR_3$,

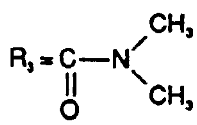 , $R_4$ = $NH_2$

Préparé selon le mode opératoire de l'exemple 21.

Cristaux de point de fusion 82°C.

Exemple 60 : **Acide [[(trifluoro-2,2,2 éthyl)-1 n-Propyl-3 (N,N-diméthyl carbamoyl) oxy-5 pyrazol-yl-4] méthyl phényl-yl-4] amino carbonyl-2 benzène sulfonique**

**Formule (I)** : $R_1$ = n-Propyl, $R_2$ = $CH_2CF_3$, A = $OR_3$,

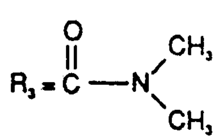 , 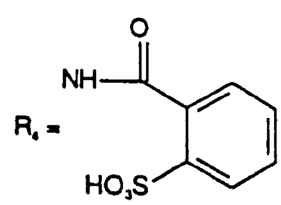

Préparé selon le mode opératoire de l'exemple 27 en utilisant l'anhydride cyclique de l'acide orthosulfobenzoïque.

Cristaux de point de fusion 159-161°C.

Exemple 61 : **(Trifluoro-2,2,2 éthyl)-1 n-Propyl-3 (nitro-4 benzyl)-4 (hydroxy-2 éthoxy)-5 pyrazole**

**Formule (XI)** : $R_1$ = n-Propyl, $R_2$ = $CH_2CF_3$, $R_3$ = $CH_2CH_2OH$, V = $NO_2$

Préparé selon le mode opératoire de l'exemple 49.

Cristaux de point de fusion 71°C.

Exemple 62 : **(Trifluoro-2,2,2 éthyl)-1 n-Propyl-3 (amino-4 benzyl)-4 (hydroxy-2 éthoxy)-5 pyrazole**

**Formule (I)** : $R_1$ = n-Propyl, $R_2 = CH_2CF_3$, A = $OR_3$, $R_3 = CH_2CH_2OH$, $R_4 = NH_2$
Préparé selon le mode opératoire de l'exemple 21.
Huile utilisée telle quelle pour la suite.

Exemple 63 : **Acide [[(trifluoro-2,2,2 éthyl)-1 n-Propyl-3 (hydroxy-2 éthoxy)-5 pyrazol-yl-4] méthyl-4 phényl-yl-4] amino carbonyl-2 benzène sulfonique**

**Formule (I)** : $R_1$ = n-Propyl, $R_2 = CH_2CF_3$, A = $OR_3$, $R_3 = CH_2CH_2OH$,

$R_4$ = 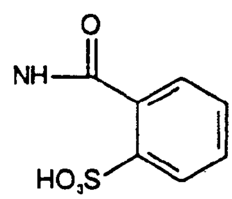

Préparé selon le mode opératoire de l'exemple 27 en utilisant l'anhydride cyclique de l'acide orthosulfobenzoïque.
Cristaux de point de fusion 211-3°C

Exemple 64 : **(Méthoxy-2 phényl)-1 [méthyl-1 n-Propyl-3 (nitro-4 benzyl)-4 pyrazol-yl-5] oxy acétyl-4 pipérazine**

**Formule (XI)** : $R_1$ = n-Propyl, $R_2$ = Méthyl,

$R_3$ = 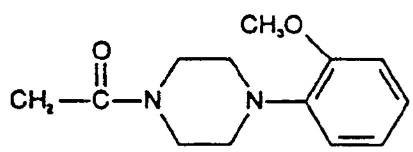 , V = $NO_2$

Préparé selon le mode opératoire de l'exemple 14 à partir de la (chloro acétyl)-1 (méthoxy-2 phényl)-4 pipérazine.
Huile utilisée telle quelle pour la suite.
La préparation de la (chloro acétyl)-1 (méthoxy-2 phényl)-4 pipérazine est réalisée selon le mode opératoire utilisé pour la synthèse de la N-chloro acétyl morpholine décrit dans l'exemple 46.

Exemple 65 : **(Méthoxy-2 phényl)-1 [méthyl-1 n-Propyl-3 (amino-4 benzyl)-4 pyrazol-yl-5] oxy acétyl-4 pipérazine**

**Formule (I)** : $R_1$ = n-Propyl, $R_2$ = Méthyl, A = $OR_3$,

$R_3$ = 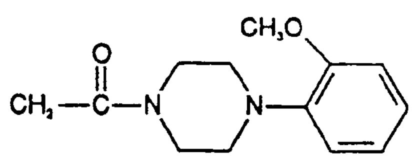 , $R_4 = NH_2$

Préparé selon le mode opératoire de l'exemple 21.
Cristaux de point de fusion 145°C.

Exemple 66 : **(Méthoxy-2 phényl)-1 [[méthyl-1 n-propyl-3 [(sulfo-2 benzoyl amino)-4 benzyl]-4 pyrazol-yl-5] oxyacétyl]-4 pipérazine**

**Formule (I)** : $R_1$ = n-Propyl, $R_2$ = Méthyl, A = $OR_3$,

$R_3$ = 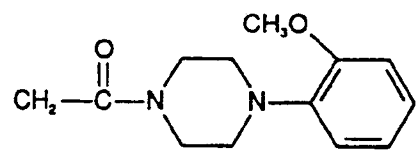 , $R_4$ = 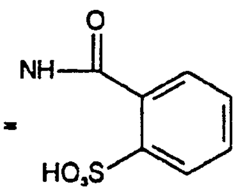

Préparé selon le mode opératoire de l'exemple 27 en utilisant l'anhydride cyclique de l'acide orthosulfobenzoïque.

Cristaux de point de fusion 226-227°C.

Exemple 67 : **(Cyano-2' biphényl-yl-4) méthyl-2 oxo-3 hexanoate d'éthyle**

**Formule (V)** : $R_1$ = n-Propyl,

V = 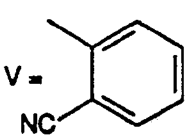 , $R_{10}$ = Ethyl

Préparé selon le mode opératoire de l'exemple 6.

Huille utilisée telle quelle pour la suite.

Exemple 68 : **Méthyl-1 n-Propyl-3 (cyano-2' biphényl-yl-4) méthyl-4 hydroxy-5 pyrazole**

**Formule (IX)** : $R_1$ = n-Propyl,

V = 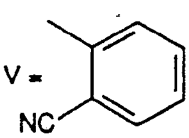 , $R_2$ = Méthyl

Préparé selon le mode opératoire de l'exemple 7.

Cristaux de point de fusion 164°C.

Exemple 69 : **[Méthyl-1 n-Propyl-3 (cyano-2' biphényl-yl-4) méthyl-4 pyrazol-yl-5] oxy acétate d'éthyle**

**Formule (XI)** : $R_1$ = n-Propyl, $R_2$= Méthyl, $R_3$ = $CH_2CO_2Et$,

$R_4$ = 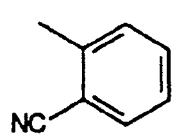

Préparé selon le mode opératoire de l'exemple 14.

Huile utilisée telle quelle pour la suite.

Exemple 70 : **[Méthyl-1 n-Propyl-3 [(tétrazolyl-5)-2′ biphényl-yl-4] méthyl-4 pyrazol-yl-5] oxy acétate d'éthyle**

**Formule (I)** : $R_1$ = n-Propyl, $R_2$ = Méthyl, A = $OR_3$, $R_3$ = $CH_2CO_2$ Et,

$R_4$ =

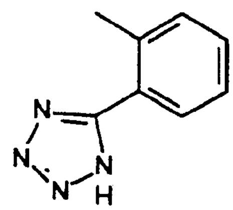

11 g de [Méthyl-1 n-Propyl-3 (cyano-2′ biphényl-yl-4)méthyl-4 pyrazol-yl-5] oxy acétate d'éthyle préparés à l'exemple 69 sont dissous dans 75 ml de toluène. 6,5 g d'azoture de triméthyl étain sont ajoutés et le mélange est chauffé au reflux pendant 24 heures. Le solvant est évaporé sous vide et le résidu est chromatographié sur gel de silice, dans un éluant Chloroforme/Méthanol 9/1, pour donner 6,7 g de [Méthyl-1 n-Propyl-3 [(tétrazolyl-5)-2′ biphényl-yl-4] méthyl-4 pyrazol-yl-5] oxy acétate d'éthyle sous forme de cristaux de point de fusion 187-189°C.

Exemple 71 : **Méthyl-1 n-Propyl-3 hydroxy-5 [(tétrazolyl-5)-2′ biphényl-yl-4] méthyl-4 pyrazole**

**Formule (I)** : $R_1$ = n-Propyl, $R_2$ = Méthyl, A = OH,

$R_4$ =

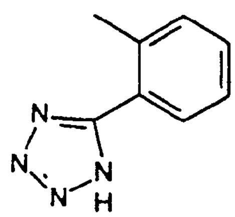

5 g de Méthyl-1 n-Propyl-3 (cyano-2′ biphényl-yl-4) méthyl-4 hydroxy-5 pyrazole, préparé à l'exemple 68, sont dissous dans 50 ml de toluène. 4 g d'azoture de triméthyl étain sont ajoutés et le mélange est chauffé au reflux pendant 14 heures. Après refroidissement 10 ml de méthanol et 10 ml de chloroforme sont ajoutés, les cristaux formés sont essorés, puis repris dans 40 ml de toluène et 10 ml de tétrahydrofurane. De l'acide chlorhydrique gazeux est mis à barboter dans le mélange réactionnel pendant 10 minutes, puis le mélange est agité 1 heure à température ambiante. Les solvants sont évaporés sous vide et le résidu est repris avec une solution de soude diluée, puis lavé à l'acétate d'éthyle. La phase aqueuse est neutralisée par barbotage de dioxyde de soufre, le résidu cristallise lentement dans l'eau pour donner après lavage à l'acétone 3,1 g de Méthyl-1 n-Propyl-3 hydroxy-5 [(tétrazolyl-5)-2′ biphényl-yl-4] méthyl-4 pyrazole sous forme de cristaux de point de fusion 150-153°C.

Exemple 72 : **Méthyl-1 n-Butyl-3 hydroxy-5 [(tétrazolyl-5)-2′ biphényl-yl-4] méthyl-4 pyrazole**

**Formule (I)** : $R_1$ = n-Butyl, $R_2$ = Méthyl, A = OH,

$R_4$ =

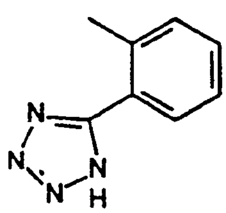

Préparé selon le mode opératoire de l'exemple 71.

Cristaux de point de fusion 176-7°C.

Exemple 73 : **(Trifluoro-2,2,2 éthyl)-1 n-Propyl-3 hydroxy-5 (cyano-2' biphényl-yl-4) méthyl-4 pyrazole**

**Formule (IX)** : $R_1$ = n-Propyl, $R_2$ = $CH_2CF_3$,

V =
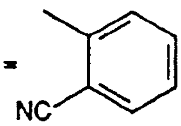

Préparé selon le mode opératoire de l'exemple 7 à partir de la (trifluoro-2,2,2-éthyl) hydrazine.
Cristaux de point de fusion 154°C.

Exemple 74 : **(Trifluoro-2,2,2 éthyl)-1 n-Propyl-3 hydroxy-5 [(tétrazolyl-5)-2' biphényl-yl-4] méthyl-4 pyrazole**

**Formule (I)** : $R_1$ = n-Propyl, $R_2$ = $CH_2CF_3$, A = OH,

$R_4$ =
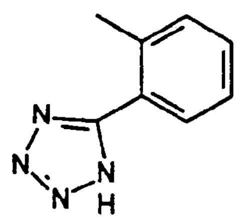

Préparé selon le mode opératoire de l'exemple 71.
Cristaux de point de fusion 218-220°C.

Exemple 75 : **[Méthyl-1 n-Butyl-3 (cyano-2' biphényl-yl-4) méthyl-4 pyrazol-yl-5] oxy-2 éthanol**

**Formule (XI)** : $R_1$ = n-Butyl, $R_2$ = Méthyl, $R_3$ = $CH_2CH_2OH$,

V =
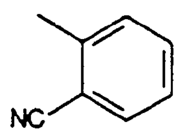

Préparé selon le mode opératoire de l'exemple 49.
Huile utilisée telle quelle pour la suite.

Exemple 76 : **[Méthyl-1 n-Butyl-3 [(tétrazolyl-5)-2' biphényl-yl-4] méthyl-4 pyrazole-yl-5] oxy-2 éthanol**

**Formule (I)** : $R_1$ = n-Butyl, $R_2$ = Méthyl, A = $OR_3$, $R_3$ = $CH_2CH_2OH$,

$R_4$ =
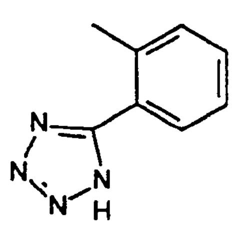

Préparé selon le mode opératoire de l'exemple 70.
Cristaux de point de fusion 105-8°C.

Exemple 77 : **[(Trifluoro-2,2,2 éthyl)-1 n-Butyl-3 (cyano-2' biphényl-yl-4) méthyl-4 pyrazol-yl-5] oxy acétate d'éthyle**

**Formule (XI)** : $R_1$ = n-Butyl, $R_2$ = $CH_2CF_3$, $R_3$ = $CH_2CO_2Et$,

V = 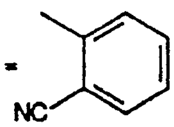

Préparé selon le mode opératoire 14.
Huile utilisée telle quelle pour la suite.

Exemple 78 : **[(trifluoro-2,2,2éthyl)-1 n-Butyl-3 [(tétrazolyl-5)-2' biphényl-yl-4] méthyl-4 pyrazol-yl-5] oxy acétate d'éthyle**

**Formule (I)** : $R_1$ = n-Butyl, $R_2$ = $CH_2CF_3$, A = $OR_3$, $R_3$ = $CH_2CO_2Et$,

$R_4$ = 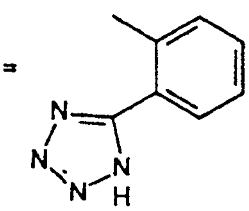

Préparé selon le mode opératoire de l'exemple 70.
Cristaux de point de fusion 140-1°C.

Exemple 79 : **(Trifluoro-2,2,2 éthyl)-1 n-Butyl-3 (cyano-2' biphényl-yl-4) méthyl-4 (N,N-diméthyl carbamoyl) oxy-5 pyrazole**

**Formule (XI)** : $R_1$ = n-Butyl, $R_2$ = $CH_2CF_3$,

$R_3$ = 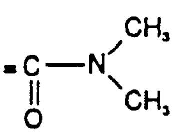 , V = 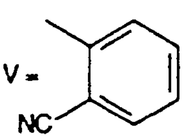

Préparé selon le mode opératoire de l'exemple 52.
Huile utilisée telle quelle pour la suite.

Exemple 80 : **(Trifluoro-2,2,2 éthyl)-1 n-Butyl-3 [(tétrazolyl-5)-2'biphényl -yl-4] méthyl-4 (N,N-diméthyl carbamoyl) oxy-5 pyrazole**

**Formule (I)** : $R_1$ = n-Butyl, $R_2$ = $CH_2CF_3$, A = $OR_3$,

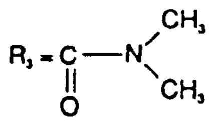 , $R_4$ = 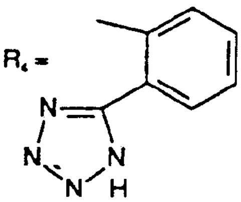

Préparé selon le mode opératoire de l'exemple 70.
Cristaux de point de fusion 151-2°C.

Exemple 81 : **Méthyl-1 n-Butyl-3 (cyano-2′ biphényl-yl-4) méthyl-4 (N,N-diméthyl carbamoyl) oxy-5 pyrazole**

**Formule (XI)** : $R_1$ = n-Butyl, $R_2$ = Méthyl,

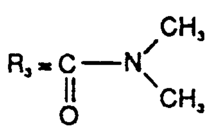 , V = 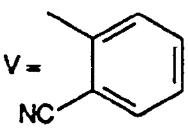

Préparé selon le mode opératoire de l'exemple 52.
Huile utilisée telle quelle pour la suite.

Exemple 82 : **Méthyl-1 n-Butyl-3 [(tétrazolyl-5)-2′ biphényl-yl-4] méthyl-4 (N,N-diméthyl carbamoyl) oxy-5 pyrazole**

**Formule (I)** : $R_1$ = n-Butyl, $R_2$ = Méthyl, A = $OR_3$,

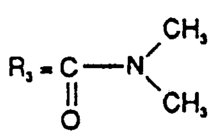 , $R_4$ = 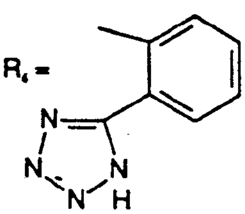

Préparé selon le mode opératoire de l'exemple 70.
Cristaux de point de fusion 160°C.

Exemple 83 : **[(Trifluoro-2,2,2 éthyl)-1 n-Propyl-3 (cyano-2′ biphényl-yl-4) méthyl-4 pyrazol-yl-5] oxy acétate d'éthyle**

**Formule (XI)**: $R_1$ = n-Propyl, $R_2$ = $CH_2CF_3$, $R_3$ = $CH_2CO_2Et$,

V = 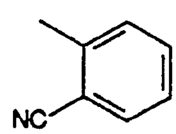

Préparé selon le mode opératoire de l'exemple 14.
Huile utilisée telle quelle pour la suite.

Exemple 84 : **[(Trifluoro-2,2,2 éthyl)-1 n-Propyl-3 [(tétrazolyl-5)-2′ biphényl-yl-4] méthyl-4 pyrazol-yl-5] oxy acétate d'éthyle**

**Formule (I)** : $R_1$ = n-Propyl, $R_2$ = $CH_2CF_3$, A = $OR_3$, $R_3$ = $CH_2CO_2Et$,

$R_4$ = 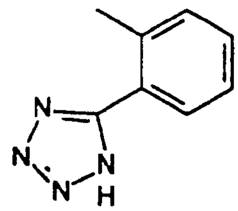

Préparé selon le mode opératoire de l'exemple 70.
Cristaux de point de fusain 146°C.

Exemple 85 : **Méthyl-1 n-Propyl-3 (cyano-2′biphényl-yl-4) méthyl-4 (N,N-diméthyl carbamoyl) oxy-5 pyrazole**

**Formule (XI)** : $R_1$ = n-Propyl, $R_2$ = Méthyl,

$R_3$ = 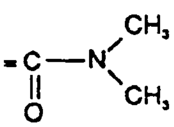 , V = 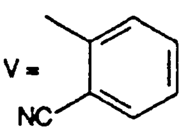

Préparé selon le mode opératoire de l'exemple 52.
Huile utilisée telle quelle pour la suite.

Exemple 86 : **Méthyl-1 n-Propyl-3 [(tétrazolyl-5)-2′ biphényl-yl-4] méthyl-4 (N,N-diméthyl carbamoyl) oxy-5 pyrazole**

**Formule (I)** : $R_1$ = n-Propyl, $R_2$ = Méthyl, A = $OR_3$,

$R_3$ = 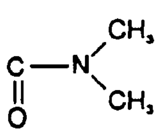 , $R_4$ = 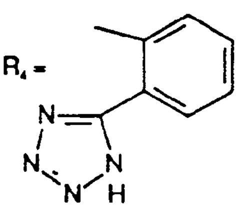

Préparé selon le mode opératoire de l'exemple 70.
Cristaux de point de fusion 103-5°C.

Exemple 87 : **[Méthyl-1 n-Propyl-3 (cyano-2′biphényl-yl-4) méthyl-4 pyrazol-yl-5] oxy-2 éthanol**

**Formule (XI)** : $R_1$ = n-Propyl, $R_2$ = Méthyl, $R_3$ = $CH_2CH_2OH$,

V = 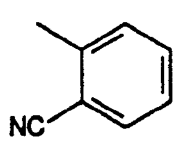

Préparé selon le mode opératoire de l'exemple 49.

Huile utilisée telle quelle pour la suite.

Exemple 88 : **[Méthyl-1 n-Propyl-3 [(tétrazolyl-5)-2'biphényl-yl-4] méthyl-4 pyrazol-yl-5] oxy-2 éthanol**

**Formule (I)** : $R_1$ = n-Propyl, $R_2$ = Méthyl, A = $OR_3$, $R_3$ = $CH_2CH_2OH$,

$R_4$ = 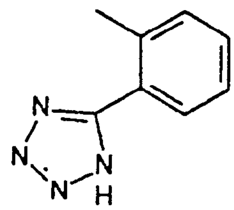

Préparé selon le mode opératoire de l'exemple 70.
Cristaux de point de fusion 120-122°C.

Exemple 89 : **[Méthyl-1 n-Butyl-3 (cyano-2'biphényl-yl-4) méthyl-4 pyrazol-yl-5] oxy acétate d'éthyle**

**Formule (XI)** : $R_1$ = n-Butyl, $R_2$ = Méthyl, $R_3$ = $CH_2CO_2Et$,

V = 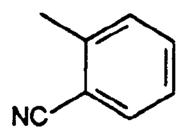

Préparé selon le mode opératoire de l'exemple 14.
Huile utilisée telle quelle pour la suite.

Exemple 90 : **[Méthyl-1 n-Butyl-3 [(tétrazolyl-5)-2'biphényl-yl-4] méthyl-4 pyrazol-yl-5] oxy acétate d'éthyle**

**Formule (I)** : $R_1$ = n-Butyl, $R_2$ = Méthyl, A = $OR_3$, $R_3$ = $CH_2CO_2Et$,

$R_4$ = 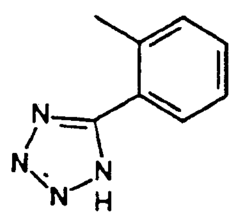

Préparé selon le mode opératoire de l'exemple 70.
Cristaux de point de fusion 142-3°C.

Exemple 91 : **[(trifluoro-2,2,2éthyl)-1 n-Propyl-3 (cyano-2'biphényl-yl-4) méthyl-4 pyrazol-yl-5] oxy-2 éthanol**

**Formule (XI)** : $R_1$ = n-Propyl, $R_2$ = $CH_2CF_3$, $R_3$ = $CH_2CH_2OH$,

V = 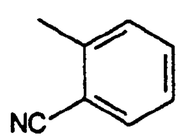

Préparé selon le mode opératoire de l'exemple 49.
Huile utilisée telle quelle pour la suite.

Exemple 92 : **[(trifluoro-2,2,2 éthyl)-1 n-Propyl-3 [(tétrazolyl-5)-2' biphényl-yl-4] méthyl-4 pyrazol-yl-5] oxy-2 éthanol**

**Formule (I)** : $R_1$ = n-Propyl, $R_2 = CH_2CF_3$, A = $OR_3$, $R_3 = CH_2CH_2OH$,

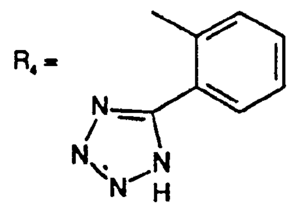

Préparé selon le mode opératoire de l'exemple 70.
Cristaux de point de fusion 167°C.

Exemple 93 : **Méthoxy-1 octane dione-2,4**

**Formule (III)** : $R_1$ = n-Butyl, R'= $CH_3$, q = 1

200 ml d'hexanone-2 et 102 ml de méthoxy acétate de méthyle sont ajoutés goutte à goutte dans 400 ml de toluène contenant 26,9 g de sodium divisé à chaud. La température lors de l'addition est maintenue entre 60°C et 70°C. A la fin de l'addition le mélange est agité 2 heures à 55°C. Après refroidissement, 20 ml de méthanol sont ajoutés. Le mélange est agité puis repris avec une solution de soude diluée, la phase aqueuse est lavée à l'éther puis acidifiée à l'acide chlorhydrique dilué et extraite à l'éther. La phase organique est évaporée sous vide à 30°C après séchage sur sulfate de magnésium, le résidu est distillé sous pression réduite pour donner 98,6 g de méthoxy-1 octane dione-2,4 sous forme d'un liquide de point d'ébullition $E_{b15}$ = 115-120°C.

Exemple 94 : **Méthoxy-1 heptane dione-2,4**

**Formule (III)** : $R_1$ = n-Propyl, R' = $CH_3$, q = 1
Préparé selon le mode opératoire de l'exemple 93 à partir de la pentanone-2.
Liquide de point d'ébullition Eb $_{20}$ = 100-110°C.

Exemple 95: **Méthoxy-1 (nitro-4 benzyl)-3 heptane dione-2,4**

**Formule (VI)** : $R_1$ = n-Propyl, R'= $CH_3$, V = $NO_2$, q = 1

135 g de méthoxy-1 heptane dione-2,4 préparé à l'exemple 94, sont dissous dans 1,5 litres de tétrahydrofurane. 123,3 g de bromure de nitro-4 benzyle, 197 ml de N,N-diisopropyl éthylamine et 49,5 g de bromure de lithium sont ajoutés et le mélange est chauffé 15 heures au reflux. Le solvant est évaporé sous vide et le résidu est repris à l'acide chlorhydrique dilué et extrait à l'acétate d'éthyle. La phase organique est évaporée à sec et les cristaux obtenus sont lavés à l'éther plusieurs fois, puis au méthanol à chaud. Les filtrats sont réunis et évaporés à sec puis le résidu est repris à l'éther isopropylique. Les cristaux obtenus sont lavés à l'éther isopropylique et séchés à 30°C sous vide pour donner 85,7 g de (nitro-4 benzyl)-3 méthoxy-1 heptane dione-2,4 sous forme de cristaux de point de fusion 55°C.

Selon le même mode opératoire les deux exemples suivants ont été préparés:

Exemple 96 : **Méthoxy-1 (cyano-2'biphényl-yl-4) méthyl-3 heptane dione-2,4**

**Formule (VI)** : $R_1$ = n-Propyl, R'= $CH_3$,

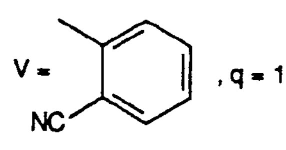

Huile utilisée telle quelle pour la suite.

Exemple 97 : **Méthoxy-1 (Cyano-2′ biphényl-yl-4) méthyl-3 octane dione-2,4**

**Formule (VI)** : $R_1$ = n-Butyl , R′= $CH_3$,

V = 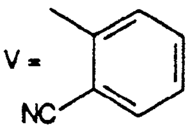 , q = 1

Huile utilisée telle quelle pour la suite.

Exemple 98 : **n-Propyl-3 (nitro-4 benzyl)-4 méthoxy méthyl-5 pyrazole**

**Formule (XIII)** : $R_1$ = n-Propyl, R′= $CH_3$, q = 1, V = $NO_2$

20 g de (nitro-4 benzyl)-3 méthoxy-1 heptane dione-2,4, préparée à l'exemple 95, sont dissous dans 400 ml d'éthanol et 4 ml d'hydrate d'hydrazine sont ajoutés, le mélange est chauffé au reflux pendant 2 heures. Le solvant est évaporé à sec sous vide et le résidu est repris à l'éther isopropylique, les cristaux obtenus sont essorés pour donner 19,5 g de (nitro-4 benzyl)-4 n-Propyl-3 méthoxy méthyl-5 pyrazole sous forme de cristaux de point de fusion 124°C.

Selon le même mode opératoire, les deux exemples suivants ont été préparés:

Exemple 99 : **n-Propyl-3 (cyano-2′biphényl-yl-4) méthyl-4 méthoxy méthyl-5 pyrazole**

**Formule (XIII)** : $R_1$ = n-Propyl, R′ = $CH_3$,

V = 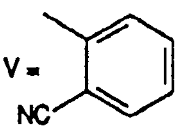 , q = 1

Huile utilisée telle quelle pour la suite.

Exemple 100 : **n-Butyl-3 (cyano-2′ biphényl-yl-4) méthyl-4 méthoxy méthyl-5 pyrazole**

**Formule (XIII)** : $R_1$ = n-Butyl , R′ = $CH_3$,

V = 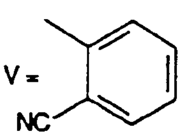 , q = 1

Huile utilisée telle quelle pour la suite.

Exemple 101 : **(Trifluoro-2,2,2-éthyl)-1 n-butyl-3 (cyano-2′ biphényl-yl-4) méthyl-4 méthoxy méthyl-5 pyrazole**

Formule (XII) : $R_1$ = n-Butyl, $R_2$ = $CH_2CF_3$, $R_3$ = $CH_3$, q = 1

V = 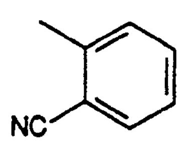

Préparé selon le mode opératoire de l'exemple 98 à partir de la (trifluoro-2,2,2-éthyl) hydrazine.

Après une chromatographie sur silicagel on obtient le produit recherché à l'éluant acétate d'éthyle / cyclohexane 2 / 8 sous forme d'une huile utilisée telle quelle pour la suite.

Exemple 102 : **Méthyl-1 n-Propyl-3 (nitro-4 benzyl)-4 méthoxy méthyl-5 pyrazole**

**Formule (XII)** : $R_1$ = n-Propyl, $R_2$ = Méthyl, R'= Méthyl, V = $NO_2$, q = 1

5 g de n-Propyl-3 (nitro-4 benzyl)-4 méthoxy méthyl-5 pyrazole, préparé à l'exemple 98, sont dissous dans 50 ml d'acétone en présence de 3 ml d'iodure de méthyle et 3 ml de DBU (1,8-diazabicyclo-[5.4.0] undec-7 ene). Le mélange est chauffé pendant 8 heures à 45°C, puis le solvant est évaporé. Le résidu est repris à l'acide chlorhydrique dilué, puis extrait à l'éther. La phase organique est séchée sur sulfate de magnésium puis évaporée sous vide pour donner un résidu huileux qui est chromatographié sur gel de silice avec un éluant éther/cyclopentane (7/3) pour donner 2,5 g de méthyl-1 n-propyl-3 (nitro-4 benzyl)-4 méthoxy méthyl-5 pyrazole sous forme de cristaux de point de fusion 73°C. Les deux exemples suivants ont été préparés selon le mode opératoire de l'exemple 102:

Exemple 103 : **Méthyl-1 n-propyl-3 (cyano-2' biphényl-yl-4) méthyl-4 méthoxy méthyl-5 pyrazole**

**Formule (XII)** : $R_1$ = n-Propyl, $R_2$ = Méthyl, R'= Méthyl,

V = 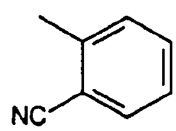 , q = 1

Huile utilisée telle quelle pour la suite.

Exemple 104 : **Méthyl-1 n-butyl-3 (cyano-2'biphényl-yl-4) méthyl-4 méthoxy méthyl-5 pyrazole**

**Formule (XII)** : $R_1$ = n-Butyl, $R_2$ = Méthyl, R' = Méthyl,

V = 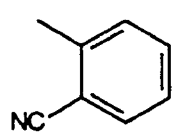 , q = 1

Huile utilisée telle quelle pour la suite.

Exemple 105 : **Méthyl-1 n-propyl-3 (amino-4 benzyl)-4 méthoxy méthyl-5 pyrazole**

**Formule (I)** : $R_1$ = n-Propyl, $R_2$ = Méthyl, A = $CH_2OCH_3$, $R_4$ = $NH_2$

Préparé selon le mode opératoire de l'exemple 21.

Huile utilisée telle quelle pour la suite.

Exemple 106 : **n-Propyl-3 (amino-4 benzyl)-4 méthoxy méthyl-5 pyrazole**

**Formule (I)** : $R_1$ = n-Propyl, $R_2$ = H, A = $CH_2O\ CH_3$, $R_4$ = $NH_2$

Préparé selon le mode opératoire de l'exemple 21.

Cristaux de point de fusion 126°C.

Exemple 107 : **Acide [(Méthyl-1 n-propyl-3 méthoxy méthyl-5 pyrazol-yl-4) méthyl phényl-yl-4] amino carbonyl-2 benzène sulfonique**

**Formule (I)** : $R_1$ = n-Propyl, $R_2$ = Méthyl, A = $CH_2OCH_3$,

$R_4$ = 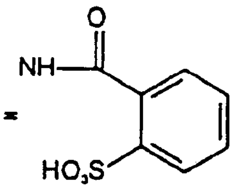

Préparé selon le mode opératoire de l'exemple 27 en utilisant l'anhydride cyclique de l'acide orthosulfobenzoïque.

Cristaux de point de fusion 126°C.

<u>Exemple 108</u> : **Acide [(n-propyl-3 méthoxy méthyl-5 pyrazol-yl-4) méthyl phényl-yl-4] amino carbonyl-2 benzène sulfonique**

**Formule (I)** : $R_1$ = n-Propyl, $R_2$ = H, A = $CH_2OCH_3$,

$R_4$ = 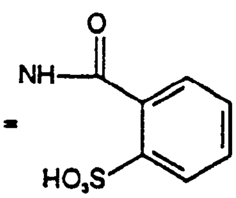

Préparé selon le mode opératoire de l'exemple 27 en utilisant l'anhydride cyclique de l'acide orthosulfobenzoïque.

Cristaux de point de fusion 253-256°C.

<u>Exemple 109</u> : **Méthyl-1 n-Propyl-3 (nitro-4 benzyl)-4 bromo méthyl-5 pyrazole**

**Formule (I)** : $R_1$ = n-Propyl, $R_2$ = Méthyl, A = $CH_2Br$, $R_4$ = $NO_2$

4,5 g de Méthyl-1 n-Propyl-3 (nitro-4 benzyl)-4 méthoxy méthyl-5 pyrazole, préparé à l'exemple 102, sont dissous dans 140 ml de chloroforme. 2,8 ml de tribromure de bore sont ajoutés goutte à goutte à une température comprise entre 0°C et 5°C, le mélange est ensuite agité une heure à cette température, puis 2 heures à température ambiante. Une solution de soude diluée est ajoutée au mélange et la phase organique est décantée, séchée et évaporée sous vide pour donner 5 g de méthyl-1 n-Propyl-3 (nitro-4 benzyl)-4 bromométhyl-5 pyrazole sous forme d'une huile utilisée telle quelle pour la suite.

<u>Exemple 110</u> : **n-Propyl-3 (nitro-4 benzyl)-4 bromométhyl-5 pyrazole**

**Formule (I)** : $R_1$ = n-Propyl, $R_2$ = H, A = $CH_2Br$, $R_4$ = $NO_2$

Préparé selon le mode opératoire de l'exemple 109.

Cristaux de point de fusion 120°C.

<u>Exemple 111</u> : **Méthyl-1 n-Propyl-3 (nitro-4 benzyl)-4 hydroxy méthyl-5 pyrazole**

**Formule (XII)** : $R_1$ = n-Propyl, $R_2$ = Méthyl, R'= H, V = $NO_2$, q = 1

6,5 g de méthyl-1 n-Propyl-3 (nitro-4 benzyl)-4 bromométhyl-5 pyrazole, préparé à l'exemple 109, sont dissous dans un mélange composé de 50 ml de dioxane et 50 ml d'eau. 5 g de carbonate de sodium sont ajoutés et le mélange est chauffé 3 heures au refux. Les solvants sont évaporés sous vide et le résidu est repris à l'eau et extrait à l'éther. La phase organique est séchée sur sulfate de magnésium puis évaporée sous vide pour donner un résidu qui cristallise dans l'éther isopropylique pour donner 3,8 g de méthyl-1 n-propyl-3 (nitro-4 benzyl)-4 hydroxy méthyl-5 pyrazole sous forme de cristaux de point de fusion 134°C.

<u>Exemple 112</u> : **n-Propyl-3 (nitro-4 benzyl)-4 hydroxy méthyl-5 pyrazole**

**Formule (XIII)** : $R_1$ = n-Propyl, R' = H, V = $NO_2$, q = 1

Préparé selon le mode opératoire de l'exemple 111.
Cristaux de point de fusion 125°C.

Exemple 113 : **Méthyl-1 n-Propyl-3 (amino-4 benzyl)-4 hydroxy méthyl-5 pyrazole**

**Formule (I)** : $R_1$ = n-Propyl, $R_2$ = Méthyl, A = $CH_2OH$, $R_4$ = $NH_2$
Préparé selon le mode opératoire de l'exemple 21.
Cristaux de point de fusion 133°C.

Exemple 114 : **n-Propyl-3 (amino-4 benzyl)-4 hydroxy méthyl-5 pyrazole**

**Formule (I)** : $R_1$ = n-Propyl, $R_2$ = H, A = $CH_2OH$, $R_4$ = $NH_2$
Préparé selon le mode opératoire de l'exemple 21.
Huile utilisée telle quelle pour la suite.

Exemple 115 : **Acide [( méthyl-1 n-propyl-3 hydroxy méthyl-5 pyrazol-yl-4) méthyl phényl-yl-4] amino carbonyl-2 benzène sulfonique**

**Formule (I)** : $R_1$ = n-Propyl, $R_2$ = Méthyl, A = $CH_2OH$,

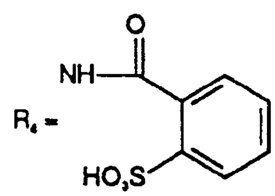

Préparé selon le mode opératoire de l'exemple 27 en utilisant l'anhydride cyclique de l'acide orthosulfobenzoïque.
Cristaux de point de fusion 255-257°C.

Exemple 116 : **Acide [(n-Propyl-3 hydroxy méthyl-5 pyrazol-yl-4) méthyl phényl-yl-4] amino carbonyl-2 benzène sulfonique**

**Formule (I)** : $R_1$ = n-Propyl, $R_2$ = H, A = $CH_2OH$

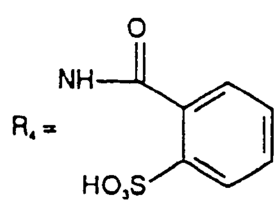

Préparé selon le mode opératoire de l'exemple 27 en utilisant l'anhydride cyclique de l'acide orthosulfobenzoïque.
Cristaux de point de fusion 168-170°C.

Exemple 117 : **Méthyl-1 n-propyl-3 [(tétrazolyl-5)-2'biphényl-yl-4] méthyl-4 méthoxy méthyl-5 pyrazole**

**Formule (I)** : $R_1$ = n-Propyl, $R_2$ = Méthyl, A = $CH_2OCH_3$

$R_4$ =

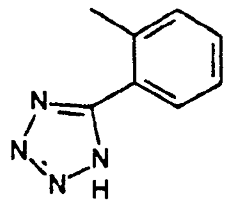

Préparé selon le mode opératoire de l'exemple 71. Cristaux de point de fusion 128-130°C.

<u>Exemple 118</u> : **Méthyl-1 n-Butyl-3 [(tétrazolyl-5)-2' biphényl-yl-4] méthyl-4 méthoxy méthyl-5 pyrazole**

**Formule (I)** : $R_1$ = n-Butyl, $R_2$ = Méthyl, A = $CH_2OCH_3$

$R_4$ =

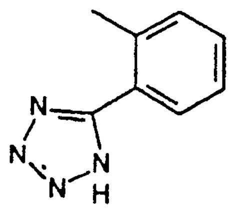

Préparé selon le mode opératoire de l'exemple 71. Cristaux de point de fusion 134- 135°C.

<u>Exemple 119</u> : **(Trifluoro-2,2,2 éthyl)-1 n-butyl-3 [(tétrazolyl-5)-2' biphényl-yl-4] méthyl-4 méthoxy méthyl-5 pyrazole**

**Formule (I)** : $R_1$ = n-Butyl, $R_2$ = $CH_2CF_3$, A = $CH_2OCH_3$

$R_4$ =

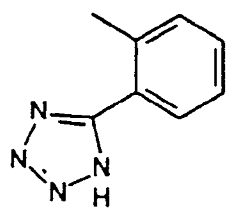

Préparé selon le mode opératoire de l'exemple 71. Cristaux de point de fusion 172-173°C.

<u>Exemple 120</u> : **Oxo-3 méthyl-5 hexanoate d'éthyle**

**Formule (II)** : $R_1$ = méthyl-2 propyl, $R_{10}$ = Ethyl
Préparé selon le même opératoire de l'exemple 1.
Liquide de point d'ébullition $Eb_{10}$ = 100-110°C.

<u>Exemple 121</u> : **(Cyano-2' biphényl-yl-4) méthyl-2 oxo-3 méthyl-5 hexanoate d'éthyle**

**Formule (V)** : $R_1$ = Méthyl-2 Propyl, $R_{10}$ = Ethyl,

V =

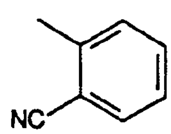

Préparé selon le mode opératoire de l'exemple 6.
Huile utilisée telle quelle pour la suite.

<u>Exemple 122</u> : **Méthyl-1 (méthyl-2 propyl)-3 (cyano-2' biphényl-yl-4) méthyl-4 hydroxy-5 pyrazole**

**Formule (IX)** : $R_1$ = Méthyl-2 Propyl, $R_2$ = Méthyl,

V =

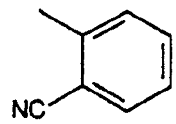

Préparé selon le mode opératoire de l'exemple 7.
Cristaux de point de fusion 163°C.

<u>Exemple 123</u> : **(Trifluoro-2,2,2 éthyl)-1 (méthyl-2 propyl)-3 (cyano-2' biphényl-yl-4) méthyl-4 hydroxy-5 pyrazole**

**Formule (IX)** : $R_1$ = Méthyl-2 Propyl, $R_2$ = $CH_2CF_3$,

V =

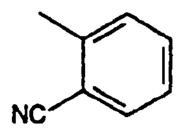

Préparé selon le mode opératoire de l'exemple 7.
Huile utilisée telle quelle pour la suite.

<u>Exemple 124</u> : **[Méthyl-1 (méthyl-2 propyl)-3 (cyano-2'biphényl-yl-4) méthyl-4 pyrazol-yl-5] oxy acétate d'éthyle**

**Formule (XI)** : $R_1$ = Méthyl-2 Propyl, $R_2$ = Méthyl, $R_3$ = $CH_2CO_2$ Et,

V =

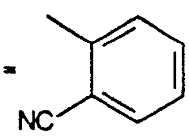

Préparé selon le mode opératoire de l'exemple 14.
Huile utilisée telle quelle pour la suite.

<u>Exemple 125</u> : **[Méthyl-1 (méthyl-2 propyl)-3 [(tétrazolyl-5)-2'biphényl-yl-4] méthyl-4 pyrazol-yl-5] oxy acétate d'éthyle**

**Formule (I)** : $R_1$ = Méthyl-2 Propyl, $R_2$ = Méthyl, A = $OR_3$, $R_3$ = $CH_2CO_2Et$,

$R_4$ =

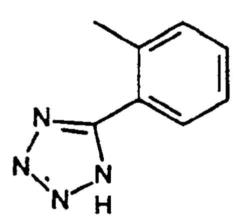

Préparé selon le mode opératoire de l'exemple 70.

Cristaux de point de fusion 172-173°C.

Exemple 126 : **[(Trifluoro-2,2,2 éthyl)-1 (méthyl-2 propyl)-3 (cyano-2' biphényl-yl-4) méthyl-4 pyrazol-yl-5] oxy acétate d'éthyle**

**Formule (XI)** : $R_1$ = Méthyl-2 Propyl, $R_2 = CH_2CF_3$, $R_3 = CH_2CO_2Et$,

V = 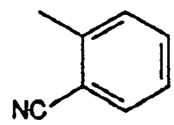

Préparé selon le mode opératoire de l'exemple 14.
Huile utilisée telle quelle pour la suite.

Exemple 127 : **[(Trifluoro-2,2,2éthyl)-1 (méthyl-2 propyl)-3 [(tétrazolyl-5)-2'biphényl-yl-4] méthyl-4 pyrazol-yl-5] oxy acétate d'éthyle**

**Formule (I)** : $R_1$ = Méthyl-2 Propyl, $R_2 = CH_2CF_3$, $A = OR_3$

$R_3 = CH_2CO_2Et$, $R_4$ = 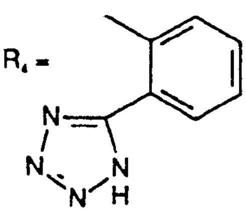

Préparé selon le mode opératoire de l'exemple 70.
Cristaux de point de fusion 114-115°C.

Exemple 128 : **[(Trifluoro-2,2,2 éthyl)-1 (méthyl-2 propyl)-3 (cyano-2' biphényl-yl-4) méthyl-4 pyrazol-yl-5] oxy-2 éthanol**

**Formule (XI)** : $R_1$ = Méthyl-2 Propyl, $R_2 = CH_2CF_3$, $R_3 = CH_2CH_2OH$,

V = 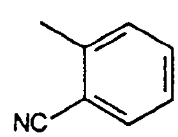

Préparé selon le mode opératoire de l'exemple 49.
Cristaux de point de fusion 116°C.

Exemple 129 : **[(Trifluoro-2,2,2 éthyl)-1 (méthyl-2 propyl)-3 [(tétrazolyl-5)-2' biphényl-yl-4] méthyl-4 pyrazol-yl-5] oxy-2 éthanol**

**Formule (I)** : $R_1$ = Méthyl-2 Propyl, $R_2 = CH_2CF_3$, $A = OR_3$,

$R_3 = CH_2CH_2OH$, $R_4$ = 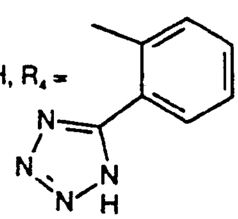

Préparé selon le mode opératoire de l'exemple 70.
Cristaux de point de fusion 145-146°C.

Exemple 130 : **N-[[Méthyl-1 n-Butyl-3 [(trifluoro méthyl sulfonyl amino-2 benzoyl) amino-4 benzyl]-4 pyrazol-yl-5] oxy acétyl] morpholine**

**Formule (I)** : $R_1$ = n-Butyl, $R_2$ = Méthyl, A = $OR_3$,

$R_3$ = 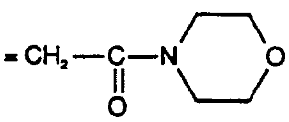 , $R_4$ = 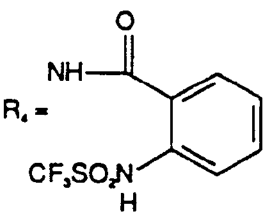

4,3 g de N-[[Méthyl-1 n-Butyl-3 (amino-4 benzyl)-4 pyrazol-yl-5] oxy acétyl] morpholine, préparés à l'exemple 47, sont dissous dans 20 ml de chloroforme en présence de 1,54 ml de triéthylamine. 3,52 g du chlorure de l'acide N-(trifluorométhyl sulfonyl) anthranilique (préparé selon les références CA: 96 ( 13) : 103651 z et CA 97(7) : 55500 w) dissous dans 10 ml de chloroforme, sont ajoutés goutte à goutte à température ambiante, puis le mélange est chauffé 2 heures à 50°C et abandonné une nuit à température ambiante. La solution est lavée à l'acide chlorhydrique dilué, séchée et évaporée sous vide. Le résidu est repris au dichlorométhane et extrait avec une solution de soude diluée. La phase aqueuse est acidifiée à pH 2 et extraite à l'acétate d'éthyle, la phase organique est séchée sur sulfate de magnésium puis évaporée à sec, l'huile obtenue cristallise dans l'éther pour donner 4,6 g de N-[[méthyl-1 n-butyl-3 [(trifluorométhyl sulfonyl amino-2 benzoyl) amino-4 benzyl]-4 pyrazol-yl-5] oxy acétyl] morpholine sous forme de cristaux de point de fusion 190°C.

Exemple 131 : **(Nitro-2' biphényl-yl-4) méthyl-2 oxo-3 hexanoate d'éthyle**

**Formule (V)** : $R_1$ = n-Propyl, $R_{10}$ = Ethyl ,

V = 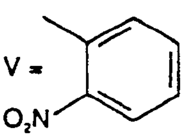

Préparé selon le mode opératoire de l'exemple 6, à partir du nitro-2'chloro méthyl-4 biphényle.
Huile utilisée telle quelle pour la suite.
Le nitro-2' chloro méthyl-4 biphényle a été préparé par chlorométhylation du nitro-2 biphényle selon les références :

- CA 70 (25): 114837 d
- CA 69 (2) : 3704 t

Exemple 132 : **Méthyl-1 n-Propyl-3 (Nitro-2' biphényl-yl-4) méthyl-4 hydroxy-5 pyrazole**

**Formule (IX)** : $R_1$ = n-Propyl, $R_2$ = Méthyl,

V = 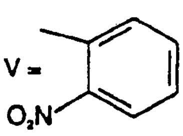

Préparé selon le mode opératoire de l'exemple 7.
Cristaux de point de fusion 179-182°C.

Exemple 133 : **[Méthyl-1 n-Propyl-3 (nitro-2′ biphényl-yl-4) méthyl-4 pyrazol-yl-5] oxy acétate de méthyle**

**Formule (XI)** : $R_1$ = n-Propyl, $R_2$ = Méthyl, $R_3$ = $CH_2CO_2Me$,

$R_4$ =

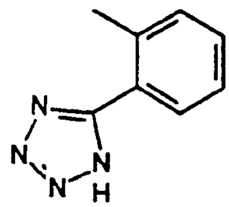

Préparé selon le mode opératoire de l'exemple 14.
Huile utilisée telle quelle pour la suite.

Exemple 134 : **[Méthyl-1 n-propyl-3 (amino-2′biphényl-yl-4) méthyl-4 pyrazol-yl-5] oxy acétate de méthyle**

**Formule (I)** : $R_1$ = n-Propyl, $R_2$ = Méthyl, A = $OR_3$ $R_3$ = $CH_2CO_2Me$,

$R_4$ =

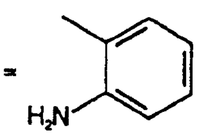

Préparé selon le mode opératoire de l'exemple 21.
Huile utilisée telle quelle pour la suite.

Exemple 135 : **[Méthyl-1 n-Propyl-3 (trifluoro méthyl sulfonyl amimo-2′ biphényl-yl-4) méthyl-4 pyrazol-yl-5] oxy acétate de méthyle**

**Formule (I)** : $R_1$ = n-Propyl, $R_2$ = Méthyl, A = $OR_3$, $R_3$ = $CH_2CO_2Me$,

$R_4$ =

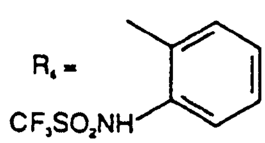

1 g de [Méthyl-1 n-Propyl-3 (amino-2′biphényl-yl-4) méthyl-4 pyrazol-yl-5] oxy acétate d'éthyle, préparé à l'exemple 134, est dissous dans 30 ml de chloroforme en présence de 0,4 ml de triéthylamine ; le mélange est refroidi à 0°C et 0,43 ml d'anhydride trifluoro méthane sulfonique sont ajoutés goutte à goutte. Le mélange est ensuite agité une heure à température ambiante, la phase organique est lavée à l'eau, séchée sur sulfate de magnésium et évaporée sous vide. Le résidu obtenu cristallise dans l'éther isopropylique pour donner 0,7 g de [Méthyl-1 n-Propyl-3 (trifluoro méthyl sulfonyl amino-2′ biphényl-yl-4) méthyl-4 pyrazol-yl-5] oxy acétate d'éthyle sous forme de cristaux de point de fusion 114-116°C.

Exemple 136 : **[Méthyl-1 n-Propyl-3 (carbéthoxy-2′ biphényl-yl-4) méthyl-4 pyrazol-yl-5] oxy-2 éthanol**

**Formule (XI)** : $R_1$ = n-Propyl, $R_2$ = Méthyl, $R_3$ = $CH_2CH_2OH$,

V =

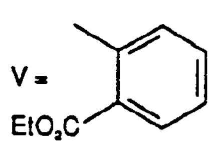

Préparé selon le mode opératoire de l'exemple 49.
Huile utilisée telle quelle pour la suite.

Exemple 137 : **[Méthyl-1 n-Propyl-3 (carboxy-2'biphényl-yl-4) méthyl-4 pyrazol-yl-5] oxy-2 éthanol**

**Formule (I)** : $R_1$ = n-Propyl, $R_2$ = Méthyl, A = $OR_3$, $R_3$ = $CH_2CH_2OH$,

$R_4$ =
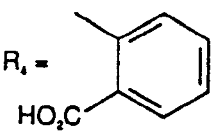

Préparé selon le mode opératoire de l'exemple 37.
Cristaux de point de fusion 168-170°C.

Exemple 138 : **[(Cyano-2 thiényl-3)-4 benzyl]-2 oxo-3 hexanoate d'éthyle**

**Formule (V)** : $R_1$ = n-Propyl, $R_{10}$ = Ethyl,

V =
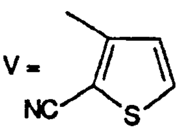

Préparé selon le mode opératoire de l'exemple 6 à partir du bromure de (cyano-2 thiényl-3)-4 benzyle.

Préparation du bromure de (cyano-2 thiényl-3)-4 benzyle:

A) Acide (méthyl-4 phényl)-3 thiophène-2 carboxylique:

25 g de (méthyl-4 phényl)-3 thiophène-2 carboxylate d'éthyle préparé selon la référence, FISSELMANN. H ; HABITCH. H ; Ger. Offen. : 1,092,929 (1960), CA: 57, 5894 g, sont dissous dans 580 ml d'éthanol et 50 ml d'eau. 6,2 g de soude en pastille sont ajoutés et le mélange est chauffé 2 heures au reflux, versé dans l'eau et acidifié par l'acide chlorhydrique concentré. Les cristaux obtenus sont essorés et séchés pour donner 20,7 g d'acide (méthyl-4 phényl)-3 thiophène-2 carboxylique sous forme de cristaux de point de fusion 172°C.

B) (Méthyl-4 phényl)-3 thiophène-2 carboxamide:

20,7 g d'acide (Méthyl-4 phényl)-3 thiophène-2 carboxylique, préparé en A), sont dissous dans 200 ml de toluène anhydre avec 10,4 ml de chlorure de thionyle. Le mélange est porté au reflux pendant trois heures puis est évaporé à sec. Le résidu huileux est additionné goutte à goutte dans une solution d'ammoniaque à 28 % à 25°C. Après une heure d'agitation, le mélange est extrait au chloroforme, la phase organique est lavée à l'eau, séchée sur sulfate de magnésium et évaporée sous vide pour donner 18 g de (méthyl-4 phényl)-3 thiophène-2 carboxamide sous forme de cristaux de point de fusion 128-130°C.

C) (Méthyl-4 phényl)-3 thiophène-2 carbonitrile:

18 g de (méthyl-4 phényl)-3 thiophène-2 carboxamide, préparé en B), sont dissous dans 36 ml d'oxychlorure de phosphore et le mélange est chauffé au reflux pendant une heure. L'oxychlorure de phosphore est concentré sous vide et le résidu est repris dans l'eau et l'acétate d'éthyle. La phase organique est extraite, séchée et filtrée sur noir animal puis évaporée sous vide pour donner 12 g de (méthyl-4 phényl)-3 thiophène-2 carbonitrile sous forme d'une huile utilisée telle quelle pour la suite.

D) Bromure de (cyano-2 thiényl-3)-4 benzyle:

12 g de (méthyl-4 phényl)-3 thiophène-2 carbonitrile, préparé en C), sont dissous dans 100 ml de tétrachlorure de carbone. 11.3 g de N-bromo succinimide sont ajoutés ainsi que 10 mg de péroxyde de benzoyle. Le mélange est chauffé au reflux jusqu'à ce que le succinimide soit totalement passé au dessus de la solution. Les cristaux sont filtrés et le solvant concentré sous vide pour donner 16 g de bromure de (cyano-2 thiényl-3)-4 benzyle sous forme d'une huile utilisée telle quelle pour la suite.

Exemple 139 : **Méthyl-1 n-propyl-3 [(Cyano-2 thiényl-3)-4 benzyl]-4 hydroxy-5 pyrazole**

**Formule (IX)** : $R_1$ = n-Propyl, $R_2$ = Méthyl,

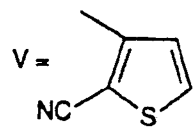

Préparé selon le mode opératoire de l'exemple 7.
Cristaux de point de fusion 110-115°C.

Exemple 140 : **[Méthyl-1 n-propyl-3 [(Cyano-2 thiényl-3)-4 benzyl]-4 pyrazol-yl-5] oxy acétate d'éthyle**

**Formule (XI)** : $R_1$ = n-Propyl, $R_2$ = Méthyl, $R_3$ = $CH_2CO_2Et$,

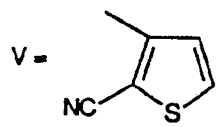

Préparé selon le mode opératoire de l'exemple 14.
Huile utilisée telle quelle pour la suite.

Exemple 141 : **[Méthyl-1 n-propyl-3 [[(tétrazolyl-5)-2 thiényl-3]-4 benzyl]-4 pyrazol-yl-5] oxy acétate d'éthyle**

**Formule (I)** : $R_1$ = n-Propyl, $R_2$ = Méthyl, A = $OR_3$, $R_3$ = $CH_2CO_2Et$,

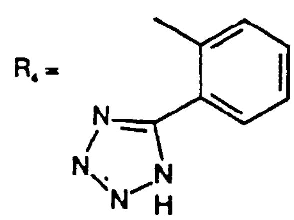

Préparé selon le mode opératoire de l'exemple 71.
Cristaux de point de fusion 201 °C.

Exemple 142 : **[(carbéthoxy-2 thiényl-3)-4 benzyl]-2 oxo-3 hexanoate d'éthyle**

**Formule (V)** : $R_1$ = n-Propyl, $R_{10}$ = Ethyl,

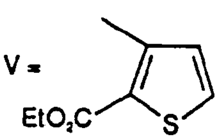

Préparé selon le mode opératoire de l'exemple 6 à partir du bromure de (carbéthoxy-2 thiényl-3)-4 benzyle.

Huile utilisée telle quelle pour la suite.

Préparation du bromure de (carbéthoxy-2 thiényl-3)-4 benzyle:

43,4 g de (méthyl-4 phényl)-3 thiophène-2 carboxylate d'éthyle, préparé selon la référence, FISSELMANN. H; HABITCH. ; Ger. Offen.: 1,092,929 (1960); CA: 57, 5894 g, sont dissous dans 250 ml de tétrachlorure de carbone. 33 g de N-bromo succinimide et 10 mg de péroxyde de benzoyle sont ajoutés et le mélange est chauffé au reflux pendant une heure, les cristaux de succinimide sont essorés et le solvant est évaporé sous vide pour donner 56,6 g de bromure de (carbéthoxy-2 thiényl-3)-4 benzyle sous forme de cristaux de point de fusion 55°C.

Exemple 143 : **Méthyl-1 n-Propyl-3 [(carbéthoxy-2 thiényl-3)-4 benzyl]-4 hydroxy-5 pyrazole**

**Formule (IX)** : $R_1$ = n-Propyl, $R_2$ = Méthyl,

V =
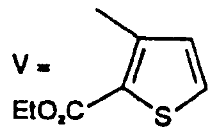

Préparé selon le mode opératoire de l'exemple 7.

Cristaux de point de fusion 130-132°C.

Exemple 144 : **[Méthyl-1 n-Propyl-3 [(carbéthoxy-2 thiényl-3)-4 benzyl]-4 pyrazol-yl-5] oxy-2 éthanol**

**Formule (XI)** : $R_1$ = n-Propyl, $R_2$ = Méthyl, $R_3$ = $CH_2CH_2OH$,

V =
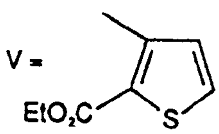

Préparé selon le mode opératoire de l'exemple 49.

Huile utilisée telle quelle pour la suite.

Exemple 145 : **[Méthyl-1 n-Propyl-3 [(carboxy-2 thiényl-3)-4 benzyl]-4 pyrazol-yl-5] oxy-2 éthanol**

**Formule (I)** : $R_1$ = n-Propyl, $R_2$ = Méthyl, A = $OR_3$, $R_3$ = $CH_2CH_2OH$,

V =
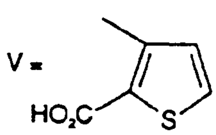

Préparé selon le mode opératoire de l'exemple 37.

Cristaux de point de fusion 118-120°C.

Exemple 146 : **(Méthoxy-2 phényl)-1 [(trifluoro-2,2,2 éthyl)-1 n-Propyl-3 (cyano-2' biphényl-yl-4) méthyl-4 pyrazol-yl-5] oxy acétyl-4 pipérazine**

**Formule (XI)** : $R_1$ = n-Propyl, $R_2$ = $CH_2$ $CF_3$,

$R_3$ =
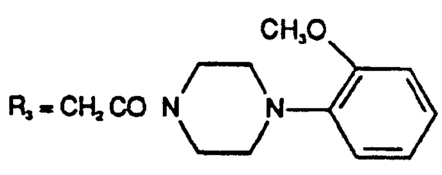
, V =
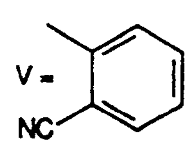

Préparé selon le mode opératoire de l'exemple 64.
Huile utilisée telle quelle pour la suite.

Exemple 147 : **(Méthoxy-2 phényl)-1 [(trifluoro-2,2,2 éthyl)-1 n-Propyl-3 [(tétrazolyl-5)-2′ biphényl-yl-4) méthyl-4 pyrazol-yl-5] oxy acétyl-4 pipérazine**

**Formule (I)** : $R_1$ = n-Propyl, $R_2$ = $CH_2CF_3$, A = $OR_3$,

$R_3$ = 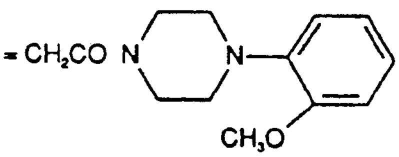 , $R_4$ = 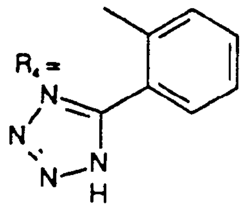

Préparé selon le mode opératoire de l'exemple 70.
Cristaux de point de fusion 158-160°C.

Exemple 148 : **N-[[(trifluoro-2,2,2 éthyl)-1 n-Propyl-3 (cyano-2′biphényl-yl-4) méthyl-4 pyrazol-yl-5] oxy acétyl] morpholine**

**Formule (XI)** : $R_1$ = n-Propyl, $R_2$ = $CH_2CF_3$,

$R_3$ = 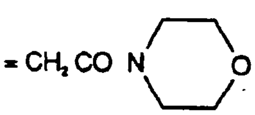 , V = 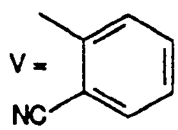

Préparé selon le mode opératoire de l'exemple 46.
Huile utilisée telle quelle pour la suite.

Exemple 149 : **N-[[(trifluoro-2,2,2 éthyl)-1 n-Propyl-3 [(tétrazolyl-5)-2′biphényl-yl-4] méthyl-4 pyrazol-yl-5] oxy acétyl] morpholine**

**Formule (I)** : $R_1$ = n-Propyl, $R_2$ = $CH_2CF_3$, A = $OR_3$,

$R_3$ = 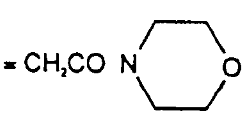 , $R_4$ = 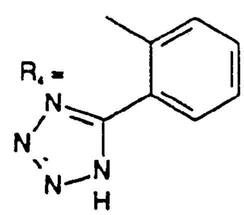

Préparé selon le mode opératoire de l'exemple 70.
Cristaux de point de fusion 129-31°C.

Exemple 150 : **(Trifluoro-2,2,2 éthyl)-1 n-Butyl-3 hydroxy-5 [(tétrazolyl-5)-2′biphényl-yl-4] méthyl-4 pyrazole**

**Formule (I)** : $R_1$ = n-Butyl, $R_2$ = $CH_2$ $CF_3$, A = OH,

$R_4$ = 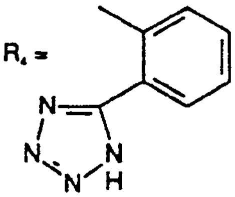

Préparé selon le mode opératoire de l'exemple 71.
Cristaux de point de fusion 210-11°C.

Exemple 151 : **Méthyl-1 n-Butyl-3 (cyano-2'biphényl-yl-4) méthyl-4 bromométhyl-5 pyrazole**

**Formule (I)** : $R_1$ = n-Butyl, $R_2$ = Méthyl, A = $CH_2Br$,

$R_4$ = 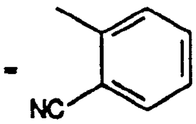

Préparé selon le mode opératoire de l'exemple 109 à partir du méthyl-1 n-Butyl-3 (cyano-2' biphényl-yl-4) méthyl-4 méthoxy méthyl-5 pyrazole préparé à l'exemple 104.
Huile utilisée telle quelle pour la suite.

Exemple 152 : **Méthyl-1 n-Butyl-3 (cyano-2'biphényl-yl-4) méthyl-4 hydroxy méthyl-5 pyrazole**

**Formule (XII)** : $R_1$ = n-Butyl, $R_2$ = Méthyl, R'= H,

V = 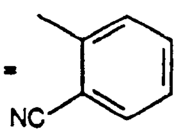 , q = 1

Préparé selon le mode opératoire de l'exemple 111.
Huile utilisée telle quelle pour la suite.

Exemple 153 : **Méthyl-1 n-Butyl-3 [(tétrazolyl-5)-2'biphényl-yl-4] méthyl-4 hydroxy méthyl-5 pyrazole**

**Formule (I)** : $R_1$ = n-Butyl, $R_2$ = Méthyl, A = $CH_2OH$,

$R_4$ = 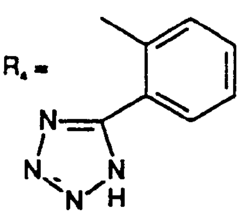

Préparé selon le mode opératoire de l'exemple 71.
Cristaux de point de fusion 110-12°C.

Exemple 154 : **Méthyl-1 n-Butyl-3 (cyano-2'biphényl-yl-4) méthyl-4 formyl-5 pyrazole**

**Formule (I)** : $R_1$ = n-Butyl, $R_2$ = Méthyl, A = CHO,

$R_4$ = 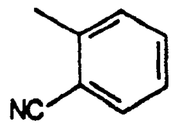

2,3 g de nitro-2 propane sont dissous dans une solution de méthylate de sodium, préparée à partir de 0,7 g de sodium et de 40 ml de méthanol. 10 g de méthyl-1 n-Butyl-3 (cyano-2′ biphényl-yl-4) méthyl-4 bromométhyl-5 pyrazole, préparés à l'exemple 151, sont dissous dans 30 ml de méthanol et cette solution est ajoutée goutte à goutte au mélange réactionnel. Le mélange est agité deux heures à 50°C puis additionné d'eau et extrait à l'acétate d'éthyle. La phase organique est évaporée à sec sous vide et le résidu obtenu est chromatographié sur gel de silice pour donner 4,3 g de Méthyl-1 n-Butyl-3 (cyano-2′ biphényl-yl-4) méthyl-4 formyl-5 pyrazole sous forme d'huile utilisée telle quelle pour la suite.

Exemple 155 : **Méthyl-1 n-Butyl-3 (cyano-2′biphényl-yl-4) méthyl-4 (dioxolane-yl-2)-5 pyrazole**

**Formule (I)** : $R_1$ = n-Butyl, $R_2$ = Méthyl,

A = 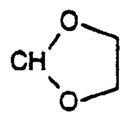 , $R_4$ = 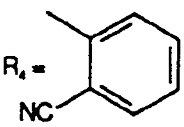

4,3 g de Méthyl-1 n-Butyl-3 (cyano-2′biphényl-yl-4) méthyl-4 formyl-5 pyrazole, préparés à l'exemple 154, sont dissous dans 50 ml de toluène. 1 g d'éthylène glycol est rajouté ainsi que 10 mg d'acide para toluène sulfonique. Le mélange est porté au reflux et l'eau est éliminée pendant 3 heures au moyen d'un Dean-Stark. Le mélange est ensuite repris à l'eau et extrait à l'éther, puis séché sur sulfate de magnésium et évaporé sous vide pour donner 4 g de méthyl-1 n-butyl-3 (cyano-2′biphényl-yl-4) méthyl-4 (dioxolane-yl-2)-5 pyrazole sous forme d'une huile utilisée telle quelle pour la suite.

Exemple 156 : **Méthyl-1 n-Butyl-3 [(tétrazolyl-5)-2′biphényl-yl-4] méthyl-4 (dioxolane-yl-2)-5 pyrazole**

**Formule (I)** : $R_1$ = n-Butyl, $R_2$ = Méthyl,

A = 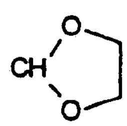 , $R_4$ = 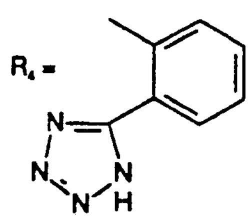

Préparé selon le mode opératoire de l'exemple 70.
Cristaux de point de fusion 144-6°C.

Exemple 157 : **[(Trifluoro-2,2,2 éthyl)-1 n-Butyl-3 (cyano-2′biphényl-yl-4) méthyl-4 pyrazol-yl-5] oxy-2 éthanol**

**Formule (XI)** : $R_1$ = n-Butyl, $R_2$ = $CH_2CF_3$, $R_3$ = $CH_2CH_2OH$,

V = 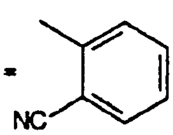

Préparé selon le mode opératoire de l'exemple 49.
Huile utilisée telle quelle pour la suite.

Exemple 158 : **[(Trifluoro-2,2,2 éthyl)-1 n-Butyl-3 [(tétrazolyl-5)-2'biphényl-yl-4] méthyl-4 pyrazol-yl-5] oxy-2 éthanol**

**Formule (I)** : $R_1$ = n-Butyl, $R_2$ = $CH_2CF_3$, A = $OR_3$, $R_3$ = $CH_2CH_2OH$,

$R_4$ =

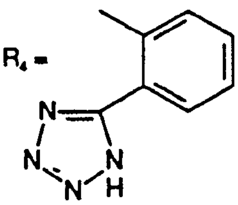

Préparé selon le mode opératoire de l'exemple 71.
Cristaux de point de fusion 107-10°C.

Exemple 159 : **Oxo-3 [(cyano-3 thiényl-yl-2)-4 benzyl]-2 heptanoate d'éthyle**

**Formule (V)** : $R_1$ = n-Butyl, $R_{10}$ = Ethyl,

V =

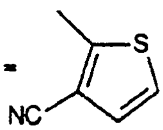

1,1 g d'oxo-3 heptanoate d'éthyle, préparé à l'exemple 1, 1,2 g de bromure de (cyano-3 thiényl-yl-2)-4 benzyle, 0,6 g de bromure de lithium et 2,2 ml de di-isopropyl éthyl amine sont ajoutés à 15 ml de tétrahydrofurane. Le mélange est agité au reflux durant 20 heures, repris à l'eau et extrait à l'éther. La phase organique est séchée sur sulfate de magnésium puis évaporée. L'excès d'oxo-3 heptanoate d'éthyle est évaporé à la pompe à palette et 1,5 g d'oxo-3 (cyano-3 thiényl-yl-2)-4 benzyl-2 heptanoate d'éthyle sont obtenus sous forme d'une huile utilisée telle quelle pour la suite.

Préparation du bromure de (cyano-3 thiényl-2)-4 benzyle:

A) Méthyl-4' chloro-4 butyrophénone:

53 ml de toluène et 70,5 g de chlorure de l'acide chloro-4 butyrique sont dissous dans 100 ml de dichlorométhane et la solution est ajoutée à 10°C à une suspension de 74 g de chlorure d'aluminium dans 200 ml de dichlorométhane. On laisse ensuite la température s'élever pendant un quart d'heure, le mélange est traité par de l'eau glacée. La phase organique est séchée sur sulfate de magnésium et évaporée sous vide pour donner 96,9 g de méthyl-4' chloro-4 butyrophénone sous forme d'une huile utilisée telle quelle pour la suite.

B) α-chloro β-(chloro-2éthyl) cinnamaldéhyde:

130 ml d'oxychlorure de phosphore sont ajoutés lentement, à 0°C, sur 130 ml de diméthyl formamide, puis 117,5 g de méthyl-4' chloro-4 butyrophénone, préparée en A), en solution dans 50 ml de diméthyl formamide sont ajoutés goutte à goutte. Le mélange est ensuite agité à température ambiante pendant une heure, puis à 50°C pendant 2 heures et à 70°C pendant 1 heure. Le mélange est alors versé sur de la glace et repris à l'éther, la phase éthérée est lavée avec une solution saturée de bicarbonate de sodium, séchée sur sulfate de sodium et évaporée sous vide pour donner 133,8 g d'α-chloro β-(chloro-2 éthyl) cinnamaldéhyde sous forme d'une huile utilisée telle quelle pour la suite.

C) (méthyl-4 phényl)-2 dihydro-4,5-thiophène-3 carboxaldéhyde:

15,9 g d'α-chloro β-(chloro-2 éthyl) cinnamaldéhyde préparés en B) et 22 g de sulfure de sodium (9 $H_2O$) sont ajoutés à 200 ml de THF. Une quantité d'eau suffisante est ajoutée afin que le sulfure de sodium passe entièrement en solution et le mélange est ensuite chauffé pendant 3 heures au reflux, refroidi puis repris à l'éther. La phase organique est décantée, lavée à l'eau puis séchée sur sulfate de magnésium et évaporée sous vide pour donner 13,5 g de (méthyl-4 phényl)-2 dihydro-4,5-thiophène-3 carboxaldéhyde sous forme d'une huile utilisée telle quelle pour la suite.

D) (Méthyl-4 phényl)-2 cyano-3 dihydro-4,5-thiophène:

15 g de (méthyl-4 phényl)-2 dihydro-4,5-thiophène-3 carboxaldéhyde, préparés en C) et 6,5 g de chlorhydrate d'hydroxylamine sont mélangés dans 40 ml d'éthanol et 10 ml d'eau. Une solution de 4,7 g de carbonate de sodium dans 10 ml d'eau est ajoutée. Le mélange est agité à température ambiante pendant une demi-heure, puis extrait à l'éther. La phase éthérée est lavée à l'eau, puis séchée sur sulfate de sodium et évaporée sous vide pour donner 15,2 g d'un résidu jaune gommeux. Ce résidu est ajouté à 13 ml d'anhydride acétique et le mélange chauffe légèrement, brunit et devient liquide. Le mélange est ensuite chauffé 1 heure au reflux, puis versé sur de la glace et extrait au dichlorométhane et lavé par une solution saturée de bicarbonate de soude, puis la phase organique est séchée sur sulfate de magnésium et évaporée sous vide, le résidu obtenu est chromatographié sur gel de silice dans le dichlorométhane pour donner 10 g de (méthyl-4 phényl)-2 cyano-3 dihydro-4,5-thiophène sous forme d'une huile utilisée telle quelle pour la suite.

E) (Méthyl-4 phényl)-2 cyano-3 thiophène:

49,9 g de (Méthyl-4 phényl)-2 cyano-3 dihydro-4,5-thiophène, préparés en D) sont dissous dans 200 ml de tétrachlorure de carbone, le mélange est chauffé au reflux et, au bout de deux heures, 11 g de brome en solution dans 200 ml de tétrachlorure de carbone sont ajoutés goutte à goutte. Le reflux est poursuivi jusqu'à cessation du dégagement d'acide bromhydrique puis le solvant est évaporé sous vide. Le résidu est repris dans 200 ml de tétrahydrofurane anhydre et 28 g de tertiobutylate de potassium sont ajoutés. Le mélange est chauffé une heure au reflux puis refroidi et ambitionné d'eau et de chlorure de sodium et extrait à l'éther. La phase organique est évaporée sous vide pour donner 31,8 g de (méthyl-4 phényl)-2 cyano-3 thiophène sous forme d'huile utilisée telle quelle pour la suite.

F) Bromure de (cyano-3 thiényl-yl-2)-4 benzyle:

24,5 g de (méthyl-4 phényl)-2 cyano-3 thiophène, préparés en E), sont dissous dans 200 ml de tétrachlorure de carbone. 21,9 g de N-bromo succinimide sont ajoutés ainsi que 0,1 g de péroxyde de benzoyle. Le mélange est chauffé 24 heures au reflux. Les cristaux de succinimide sont filtrés et le solvant évaporé sous vide. Le résidu est repris dans un mélange d'hexane et d'acétate d'éthyle et la solution est gardée 24 heures au congélateur. Les cristaux formés sont essorés pour donner 14 g de bromure de (cyano-3 thiényl-yl-2)-4 benzyle sous forme de cristaux de point de fusion 80°C.

Exemple 160 : **Méthyl-1 n-Butyl-3 [(cyano-3 thiényl-yl-2)-4 benzyl]-4 hydroxy-5 pyrazole**

**Formule (IX)** : $R_1$ = n-Butyl, $R_2$ = Méthyl,

V =
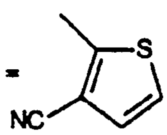

1,4 g de [(cyano-3 thiényl-yl-2)-4 benzyl]-2 oxo-3 heptanoate d'éthyle préparés à l'exemple 159, sont dissous dans 10 ml d'éthanol. 2,2 ml de méthyl hydrazine sont ajoutés et le mélange est porté au reflux pendant 13 heures, puis additionné d'eau et extrait à l'éther puis à l'acétate d'éthyle. Les phases organiques sont réunies et évaporées sous vide pour donner un résidu huileux qui donne, après chromatographie sur gel de silice dans un éluant dichlorométhane/méthanol 95/5, 0,8 g de Méthyl-1 n-Butyl-3 [(cyano-3 thiényl-yl-2)-4 benzyl]-4 hydroxy-5 pyrazole sous forme de cristaux de point de fusion 120°C.

Exemple 161 : **Méthyl-1 n-propyl-3 (cyano-2'biphényl-yl-4) méthyl-4 (N,N-diéthyl carbamoyl) oxy-5 pyrazole**

**Formule (IX)** : $R_1$ = n-Propyl, $R_2$ = Méthyl,

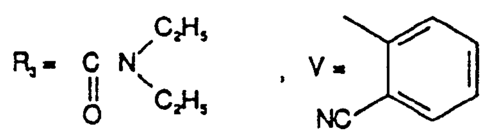

Préparé selon le mode opératoire de l'exemple 52.
Huile utilisée telle quelle pour la suite.

Exemple 162 : **Méthyl-1 n-propyl-3 [(tétrazolyl-5)-2'biphényl-yl-4] méthyl-4 (N,N-diéthyl carbamoyl) oxy-5 pyrazole**

**Formule (I)** : $R_1$ = n-Propyl, $R_2$ = Méthyl, A = $OR_3$,

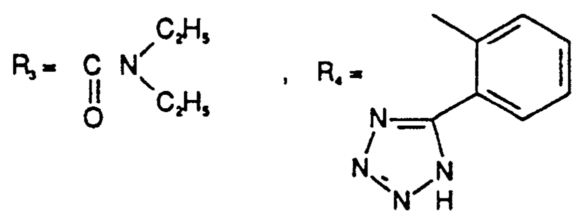

Préparé selon le mode opératoire de l'exemple 70.
Cristaux de point de fusion 134-135°C.

Exemple 163 : **N-[[(trifluoro-2,2,2 éthyl)-1 n-propyl-3 (cyano-2'biphényl-yl-4) méthyl-4 pyrazol-yl-5] oxy acétyl] thiomorpholine**

**Formule (XI)** : $R_1$ = n-Propyl, $R_2$ = $CH_2CF_3$

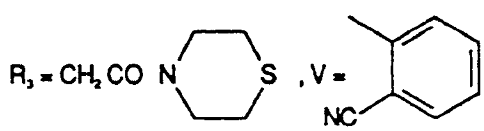

Préparé selon le mode opératoire de l'exemple 46 à partir de la N-(chloro acétyl) thiomorpholine.
Huile utilisée telle quelle pour la suite.

Exemple 164 : **N-[[(trifluoro-2,2,2 éthyl)-1 n-propyl-3 [(tétrazolyl-5)-2'biphényl-yl-4] méthyl-4 pyrazol-yl-5] oxy acétyl] thiomorpholine**

**Formule (I)** : $R_1$ = n-Propyl, $R_2$ = $CH_2CF_3$, A = $OR_3$,

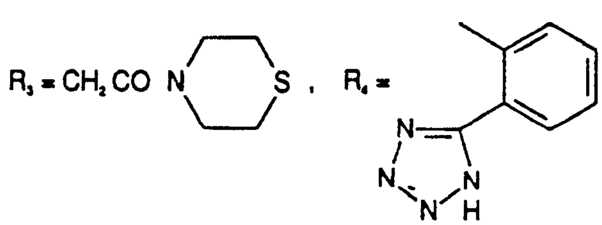

Préparé selon le mode opératoire de l'exemple 70.

Cristaux de point de fusion 127-128°C.

<u>Exemple 165</u> : **(Méthoxy-2 phényl)-1 [méthyl-1 n-propyl-3 (cyano-2'biphényl-yl-4) méthyl-4 pyrazol-yl-5] oxy acétyl-4 pipérazine**

**Formule (XI)** : $R_1$ = n-Propyl, $R_2$ = Méthyl,

$R_3$ = 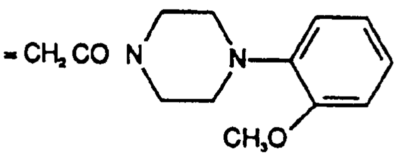 , V = 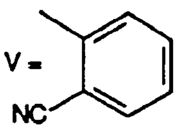

Préparé selon le mode opératoire de l'exemple 64.
Huile utilisée telle quelle pour la suite.

<u>Exemple 166</u> : **(Méthoxy-2 phényl)-1 [méthyl-1 n-propyl-3 [(tétrazolyl-5)-2'biphényl-yl-4] méthyl-4 pyrazol-yl-5] oxy acétyl-4 pipérazine**

**Formule (I)** : $R_1$ = n-Propyl, $R_2$ = Méthyl, A = $OR_3$,

$R_3$ = 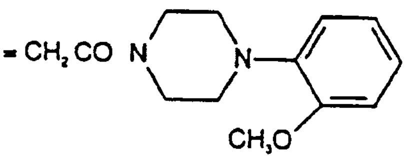 , V = 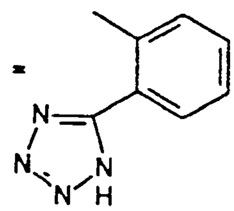

Préparé selon le mode opératoire de l'exemple 70.
Cristaux de point de fusion 139-141°C.

<u>Exemple 167</u> : **n-Propyl-3 méthyl-1 (cyano-2'biphényl-yl-4) méthyl-4 (diméthylamino-2 éthoxy)-5 pyrazole**

**Formule (XI)** : $R_1$ = n-Propyl, $R_2$ = Méthyl,

$R_3$ = 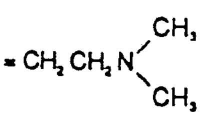 , V = 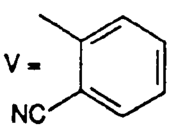

10 g de n-Propyl-3 méthyl-1 (cyano-2' biphényl-yl-4) méthyl-4 hydroxy-5 pyrazole, préparés à l'exemple 68, sont dissous dans 100 ml de butanone-2 en présence de 10 g de carbonate de sodium et de 8,6 g de chlorhydrate de N,N diméthyl chloro-2 éthyl amine. Le mélange est porté 20 heures au reflux puis concentré sous vide, repris à l'eau et extrait à l'éther. La phase éthérée est lavée à l'eau, séchée puis évaporée sous vide. Le résidu est chromatographié sur gel de silice dans un éluant chloroforme/méthanol 95/5 pour donner 4,6 g de n-propyl-3 méthyl-1 (cyano-2' biphényl-yl-4) méthyl-4 (diméthylamino-2 éthoxy)-5 pyrazole sous forme d'une huile utilisée telle quelle pour la suite.

<u>Exemple 168</u> : **n-Propyl-3 méthyl-1 [(tétrazolyl-5)-2'biphényl-yl-4] méthyl-4 (diméthylamino-2 éthoxy)-5 pyrazole**

**Formule (I)** : $R_1$ = n-Propyl, $R_2$= Méthyl, A = $OR_3$,

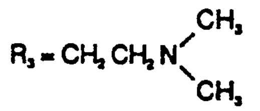 , $R_4$ = 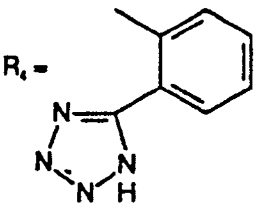

Préparé selon le mode opératoire de l'exemple 70.
Cristaux de point de fusion 88-90°C.

Exemple 169 : **N-[[Méthyl-1 n-propyl-3 (cyano-2'biphényl-yl-4) méthyl-4 pyrazol-yl-5] oxy-2 éthyl] morpholine**

**Formule (XI)** : $R_1$ = n-Propyl, $R_2$ = Méthyl,

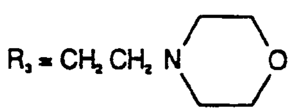 , V = 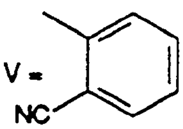

Préparé selon le mode opératoire de l'exemple 167 à partir de la N-(chloro-2 éthyl) morpholine.
Huile utilisée telle quelle pour la suite.

Exemple 170 : **N-[[Méthyl-1 n-propyl-3 [(tétrazolyl-5)-2'biphényl-yl-4] méthyl-4 pyrazol-yl-5] oxy-2 éthyl] morpholine**

**Formule (I)** : $R_1$ = n-Propyl, $R_2$ = Méthyl, A = $OR_3$,

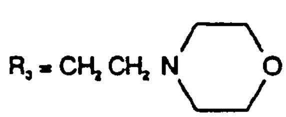 , $R_4$ = 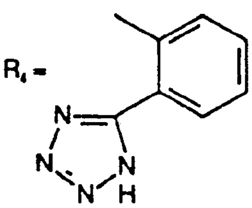

Préparé selon le mode opératoire de l'exemple 70.
Cristaux de point de fusion 132-134°C.

Exemple 171 : **(Trifluoro-2,2,2 éthyl)-1 n-butyl-3 (cyano-2'biphényl-yl-4) méthyl-4 hydroxy méthyl-5 pyrazole**

**Formule (XII)** : $R_1$ = n-Butyl, $R_2$ = $CH_2CF_3$, R' = H,

V = 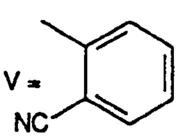 , q = 1

Préparé selon le mode opératoire de l'exemple 111 à partir du (trifluoro-2,2,2 éthyl)-1 n-butyl-3 (cyano-2' biphényl-yl-4) méthyl-4 bromométhyl-5 pyrazole qui est préparé selon le mode opératoire de l'exemple 109.
Huile utilisée telle quelle pour la suite.

Exemple 172 : **(Trifluoro-2,2,2 éthyl)-1 n-butyl-3 [(tétrazolyl-5)-2′ biphényl-yl-4] méthyl-4 hydroxy méthyl-5 pyrazole**

**Formule (I)** : $R_1$ = n-Butyl, $R_2$ = $CH_2CF_3$, A = $CH_2OH$,

$R_4$ = 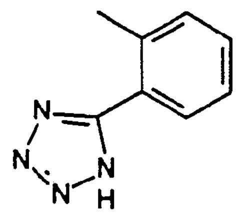

Préparé selon le mode opératoire de l'exemple 71.
Cristaux de point de fusion 114-117°C.

Exemple 173 : **(Trifluoro-2,2,2 éthyl)-1 n-propyl-3 (cyano-2′biphényl-yl-4) méthyl-4 méthoxy méthyl-5 pyrazole**

**Formule (XII)** : $R_1$ = n-Propyl, $R_2$ = $CH_2CF_3$, R′ = $CH_3$, q = 1,

V = 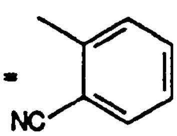

Préparé selon le mode opératoire de l'exemple 101.
Huile utilisée telle quelle pour la suite.

Exemple 174 : **(Trifluoro-2,2,2 éthyl)-1 n-propyl-3 [(tétrazolyl-5)-2′biphényl-yl-4] méthyl-4 méthoxy méthyl-5 pyrazole**

**Formule (I)** : $R_1$ = n-Propyl, $R_2$ = $CH_2CF_3$ A = $CH_2OCH_3$,

$R_4$ = 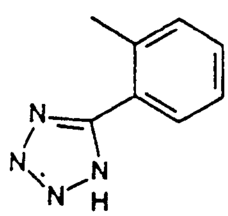

Préparé selon le mode opératoire de l'exemple 71.
Cristaux de point de fusion 177-178°C.

Exemple 175 : **N-[[Méthyl-1 n-butyl-3 (cyano-2′biphényl-yl-4) méthyl-4 pyrazol-yl-5] oxy acétyl] morpholine**

**Formule (XI)** : $R_1$ = n-Butyl, $R_2$ = Méthyl,

$R_3$ = $CH_2$ CO 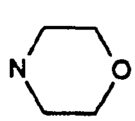 , V = 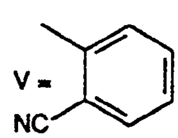

Préparé selon le mode opératoire de l'exemple 46.

Huile utilisée telle quelle pour la suite.

Exemple 176 : **N-[[Méthyl-1 n-propyl-3 (cyano-2'biphényl-yl-4) méthyl-4 pyrazol-yl-5] oxy acétyl] morpholine**

**Formule (XI)** : $R_1$ = n-Propyl, $R_2$ = Méthyl,

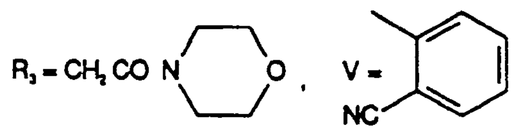

Préparé selon le mode opératoire de l'exemple 46.
Huile utilisée telle quelle pour la suite.

Exemple 177 : **N-[[Méthyl-1 n-Butyl-3 [(tétrazolyl-5)-2'biphényl-yl-4] méthyl-4 pyrazol-yl-5] oxy acétyl] morpholine**

**Formule (I)** : $R_1$ = n-Butyl, $R_2$ = Méthyl, A = $OR_3$,

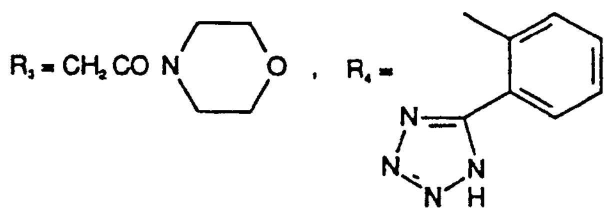

Préparé selon le mode opératoire de l'exemple 70.
Cristaux de point de fusion 140-141 °C.

Exemple 178 : **N-[[Méthyl-1 n-Propyl-3 [(tétrazolyl-5)-2'biphényl-yl-4] méthyl-4 pyrazol-yl-5] oxy acétyl] morpholine**

**Formule (I)** : $R_1$ = n-Propyl, $R_2$ = Méthyl, A = $OR_3$,

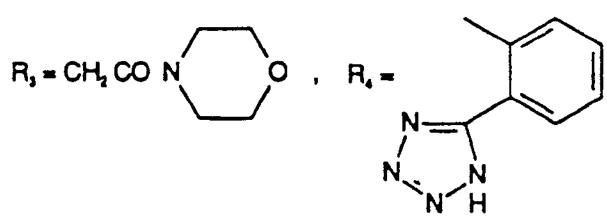

Préparé selon le mode opératoire de l'exemple 70.
Cristaux de point de fusion 106-110°C.

Exemple 179 : **(Trifluoro-2,2,2 éthyl)-1 n-Propyl-3 (cyano-2'biphényl-yl-4) méthyl-4 hydroxy méthyl-5 pyrazole**

**Formule (XII)** : $R_1$ = n-Propyl, $R_2$ = $CH_2CF_3$, R' = H

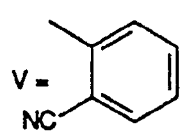 , q = 1

Préparé selon le mode opératoire de l'exemple 111 à partir du (trifluoro-2,2,2 éthyl)-1 n-propyl-3 (cyano-2'biphényl-yl-4) méthyl-4 bromométhyl-5 pyrazole qui est préparé selon le mode opératoire de l'exemple 109.
Huile utilisée telle quelle pour la suite.

Exemple 180 : **(Trifluoro-2,2,2 éthyl)-1 n-propyl-3 [(tétrazolyl-5)-2' biphényl-yl-4] méthyl-4 hydroxy méthyl-5 pyrazole**

**Formule (I)** : $R_1$ = n-Propyl, $R_2$ = $CH_2CF_3$, A = $CH_2OH$,

$R_4$ = 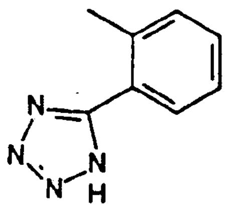

Préparé selon le mode opératoire de l'exemple 71.
Cristaux de point de fusion 110-2°C.

Exemple 181 : **Méthyl-1 n-propyl-3 (cyano-2'biphényl-yl-4) méthyl-4 hydroxy méthyl-5 pyrazole**

**Formule (XII)** : $R_1$ = n-Propyl, $R_2$ = Méthyl, R' = H,

V = 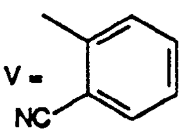 , q = 1

Préparé selon le mode opératoire de l'exemple 111.
Huile utilisée telle quelle pour la suite.

Exemple 182 : **Méthyl-1 n-propyl-3 [(tétrazolyl-5)-2'biphényl-yl-4] méthyl-4 hydroxyméthyl-5 pyrazole**

**Formule (I)** : $R_1$ = n-Propyl, $R_2$ = Méthyl, A = $CH_2OH$,

$R_4$ = 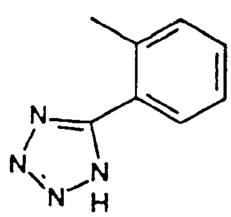

Préparé selon le mode opératoire de l'exemple 71.
Cristaux de point de fusion 136-9°C.

Exemple 183 : **(Méthoxy-2 phényl)-1 [méthyl-1 n-butyl-3 (cyano-2'biphényl-yl-4) méthyl-4 pyrazol-yl-5] oxyacétyl-4 pipérazine**

**Formule (XI)** : $R_1$ = n-Butyl, $R_2$ = Méthyl,

$R_3$ = $CH_2CO$ 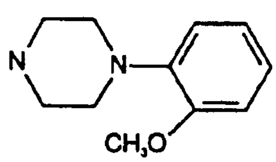 , V = 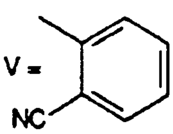

Préparé selon le mode opératoire de l'exemple 64.
Huile utilisée telle quelle pour la suite.

Exemple 184 : **(Méthoxy-2 phényl)-1 [méthyl-1 n-butyl-3 [(tétrazolyl-5)-2'biphényl-yl-4] méthyl-4 pyrazol-yl-5] oxyacétyl-4 pipérazine**

**Formule (I)** : $R_1$ = n-Butyl, $R_2$ = Méthyl, A = $OR_3$,

$R_3$ = 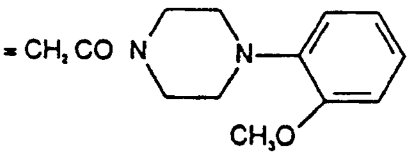 , $R_4$ = 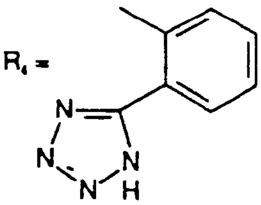

Préparé selon le mode opératoire de l'exemple 70.
Cristaux de point de fusion 153-5°C.

Exemple 185 : **N-[[méthyl-1 n-propyl-3 (cyano-2'biphényl-yl-4) méthyl-4 pyrazol-yl-5] oxyacétyl] thiomorpholine**

**Formule (XI)** : $R_1$ = n-Propyl, $R_2$ = Méthyl,

$R_3$ = 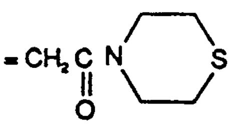 , V = 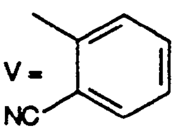

Préparé selon le mode opératoire de l'exemple 46, à partir de la N-(chloroacétyl) thiomorpholine.
Huile utilisée telle quelle pour la suite.

Exemple 186 : **N-[[méthyl-1 n-propyl-3 [(tétrazolyl-5)-2'biphényl-yl-4] méthyl-4 pyrazol-yl-5] oxyacétyl] thiomorpholine**

**Formule (I)** : $R_1$ = n-Propyl, $R_2$ = Méthyl, A = $OR_3$,

$R_3$ = 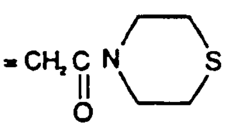 , $R_4$ = 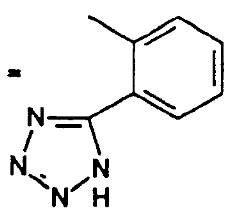

Préparé selon le mode opératoire de l'exemple 70.
Cristaux de point de fusion 140-1°C.

TABLEAU

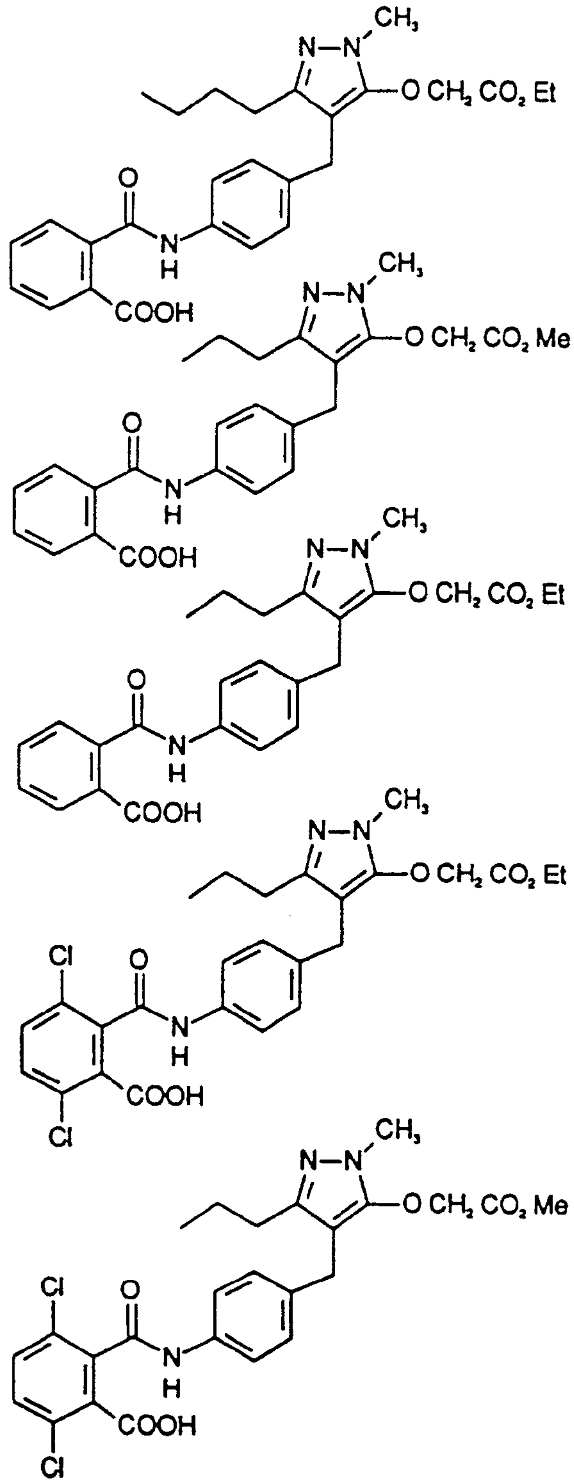

Exemple 27 UP 221-1

Exemple 28 UP 221-6

Exemple 29 UP 221-11

Exemple 30 UP 221-13

Exemple 31 UP 221-12

Exemple 32

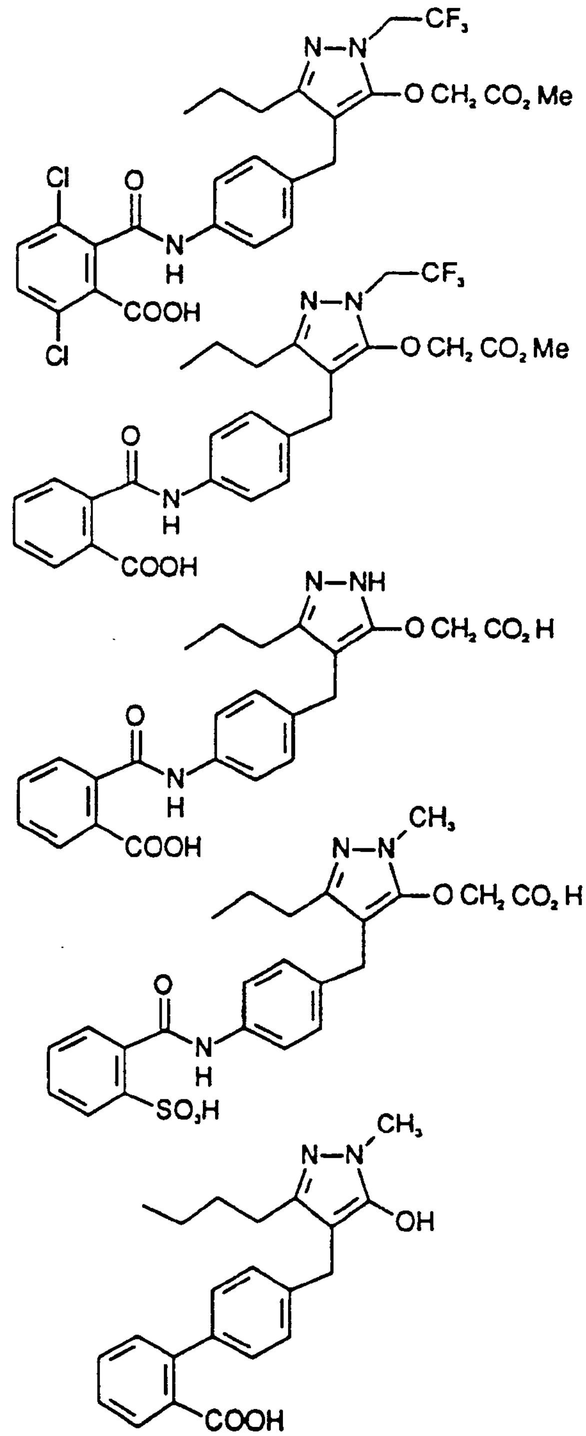

UP 221-7

Exemple 33

UP 221-4

Exemple 34

UP 221-16

Exemple 35

UP 221-14

Exemple 36

UP 221-15

Exemple 37

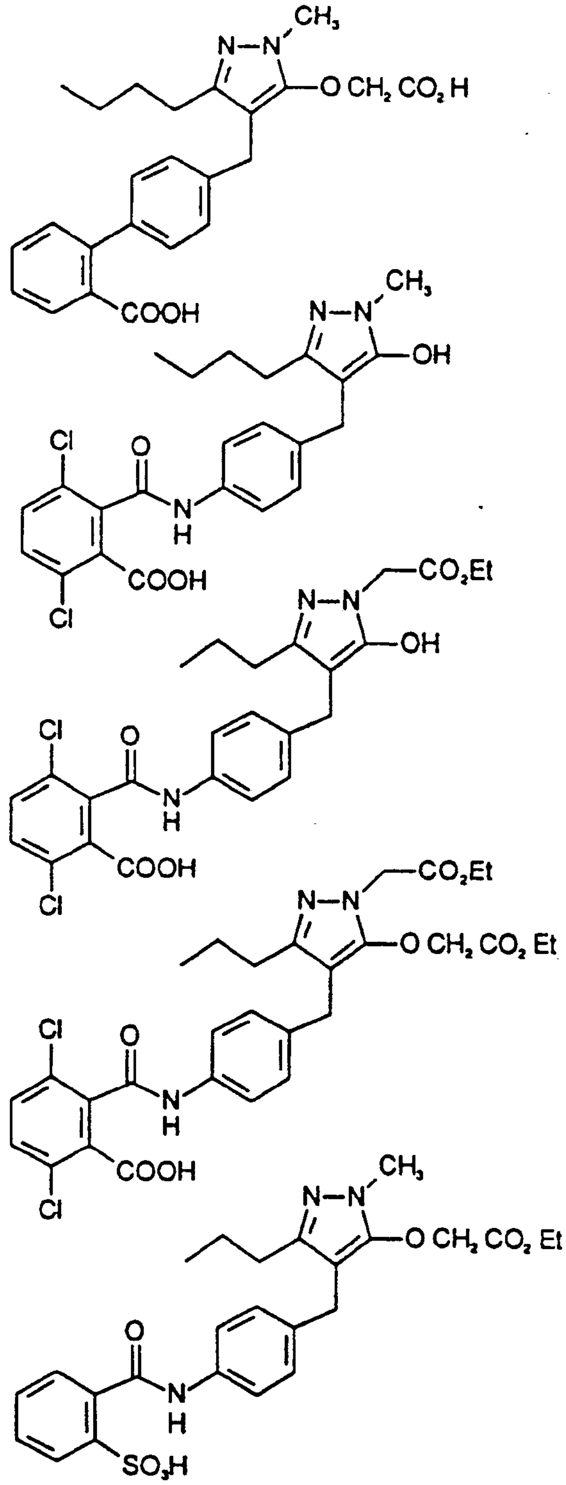

UP 221-17

Exemple 40

UP 221-18

Exemple 41

UP 221-19

Exemple 44

UP 221-20

Exemple 45

UP 221-21

Exemple 48

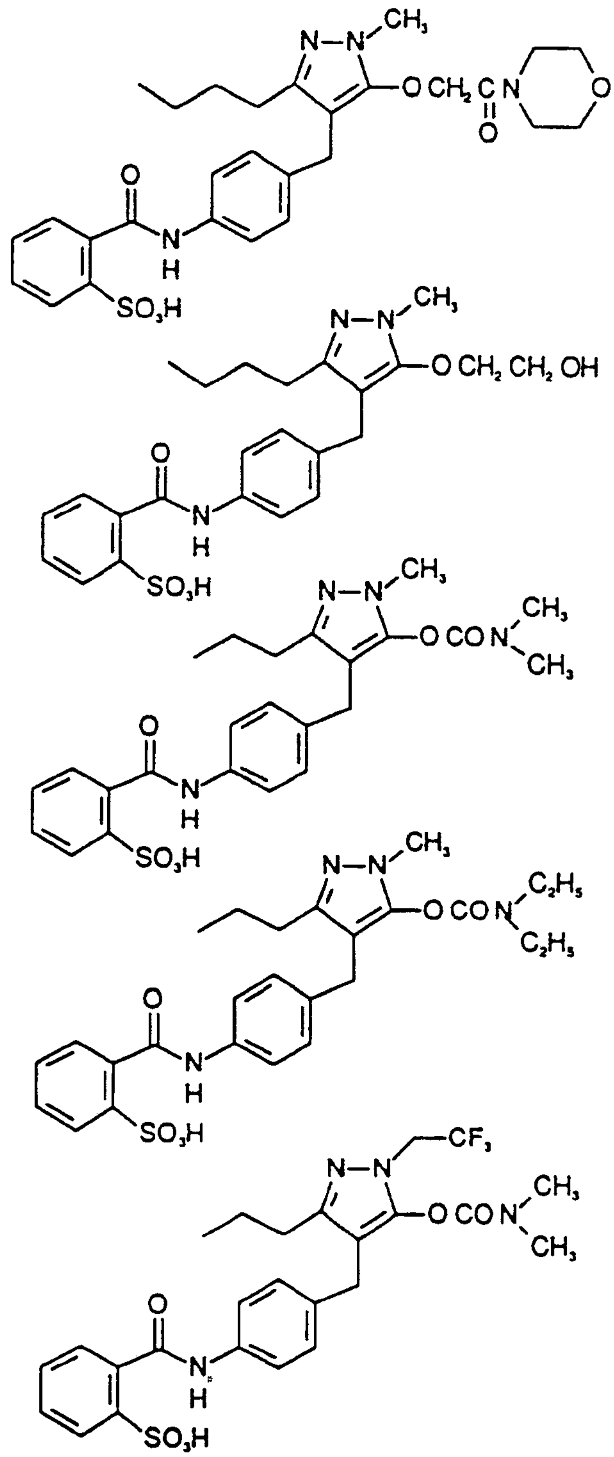

UP 221-22

Exemple 51

UP 221-23

Exemple 54

UP 221-27

Exemple 57

UP 221-28

Exemple 60

UP 221-30

Exemple 63 UP 221-32

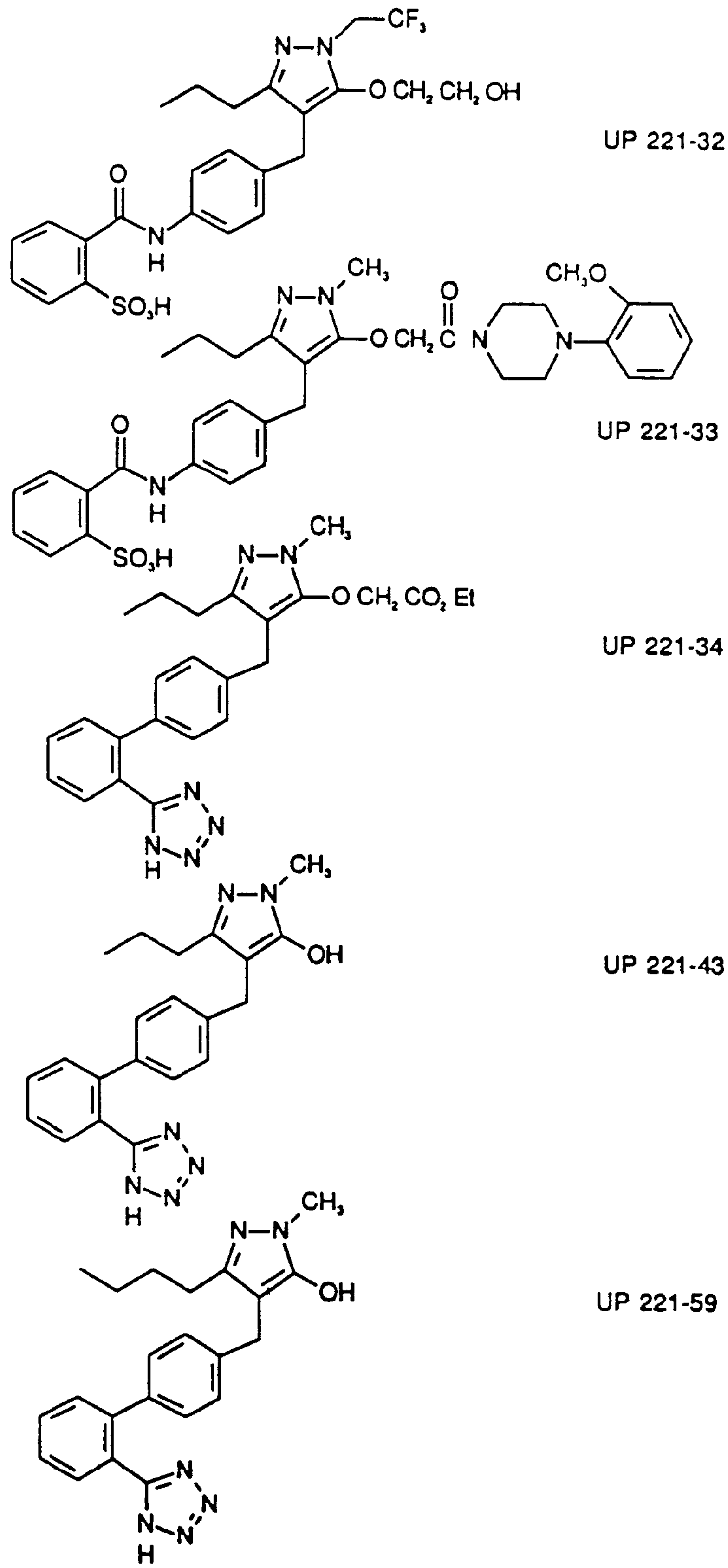

Exemple 66 UP 221-33

Exemple 70 UP 221-34

Exemple 71 UP 221-43

Exemple 72 UP 221-59

Exemple 74

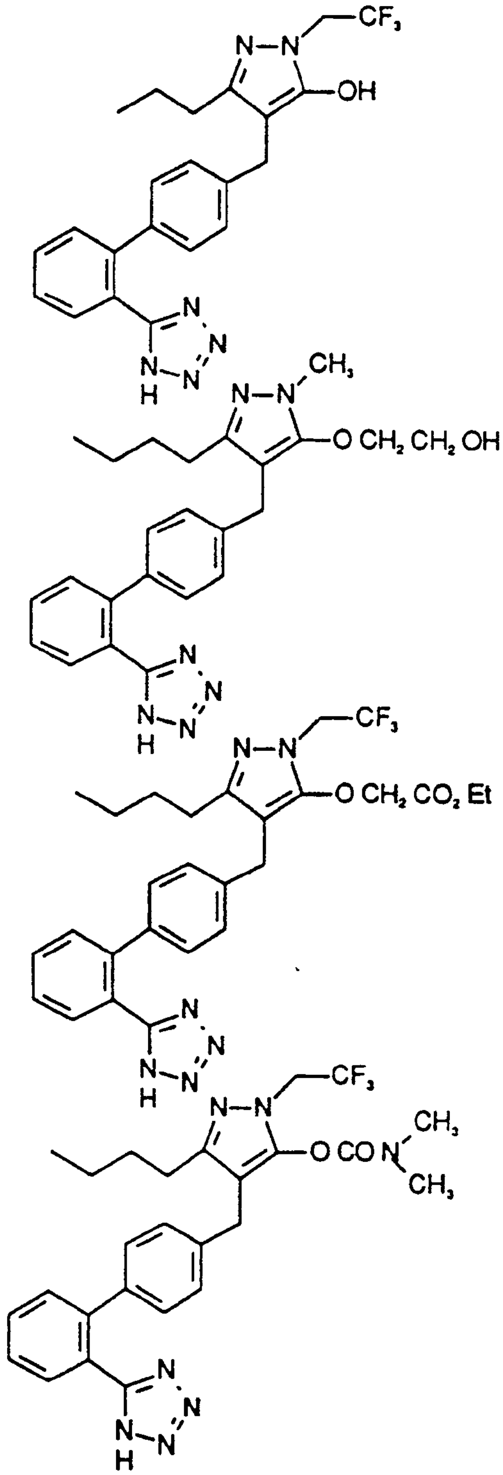

UP 221-56

Exemple 76

UP 221-57

Exemple 78

UP 221-54

Exemple 80

UP 221-55

Exemple 82

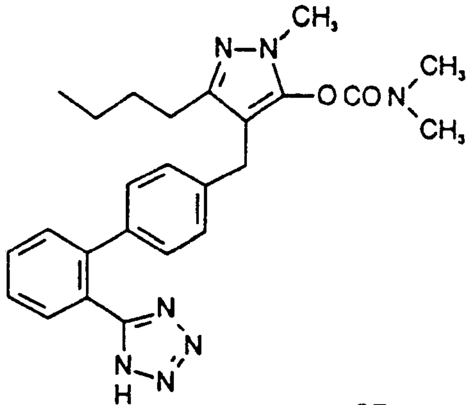

UP 221-51

Exemple 84

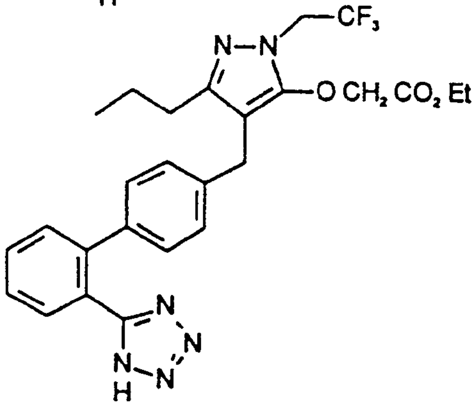

UP 221-44

Exemple 86

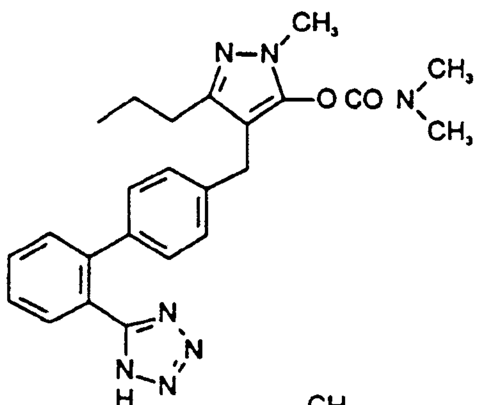

UP 221-45

Exemple 88

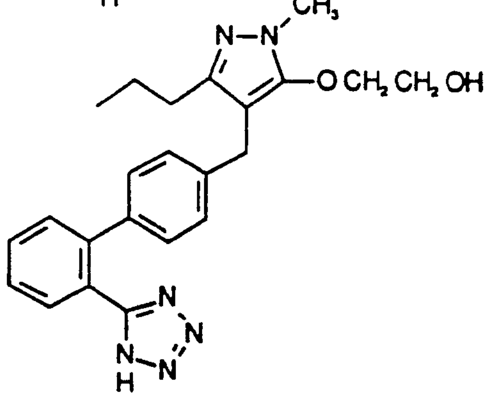

UP 221-46

Exemple 90

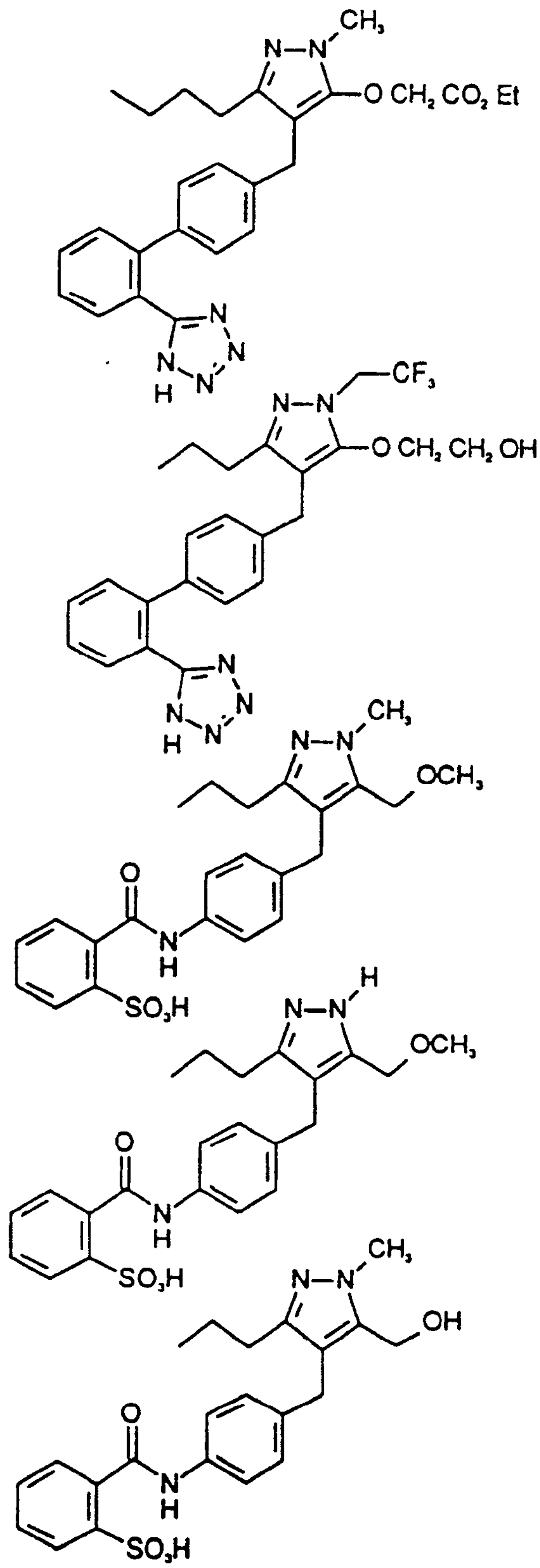

UP 221-47

Exemple 92

UP 221-50

Exemple 107

UP 221-38

Exemple 108

UP 221-36

Exemple 115

UP 221-42

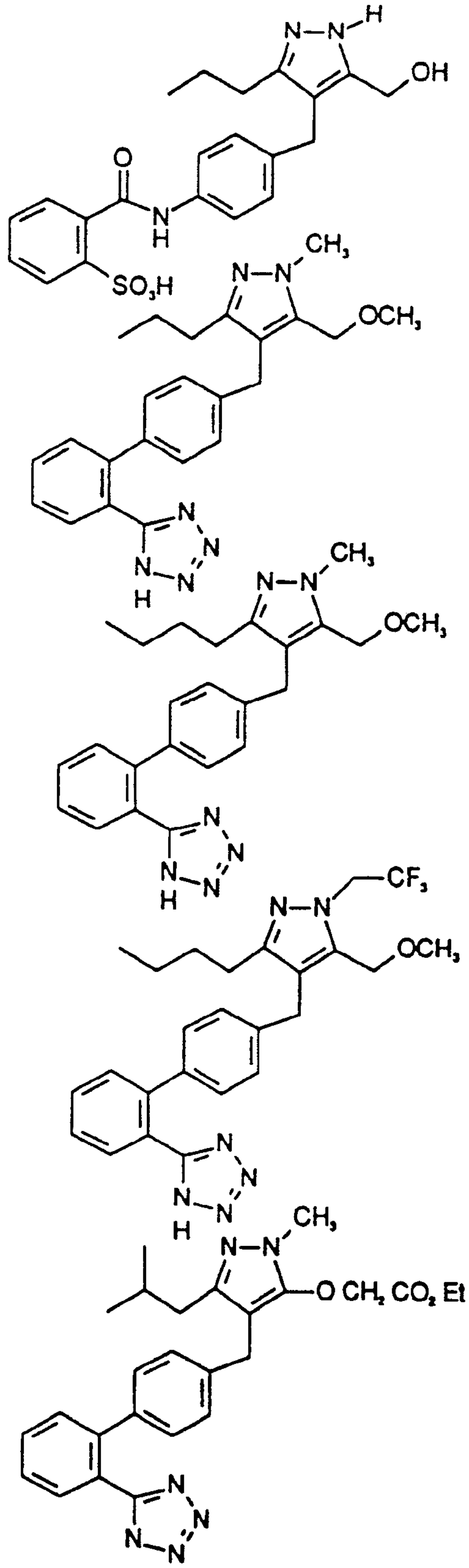

Exemple 116 UP 221-40

Exemple 117 UP 221-48

Exemple 118 UP 221-52

Exemple 119 UP 221-53

Exemple 125 UP 221-58

Exemple 127

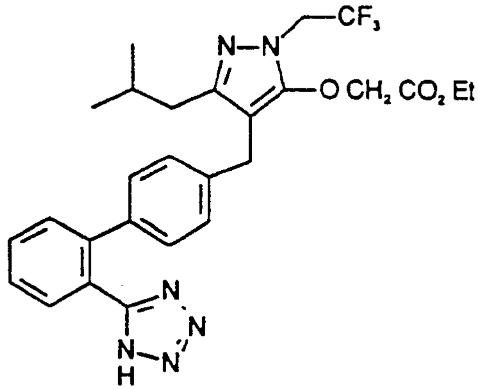

UP 221-61

Exemple 129

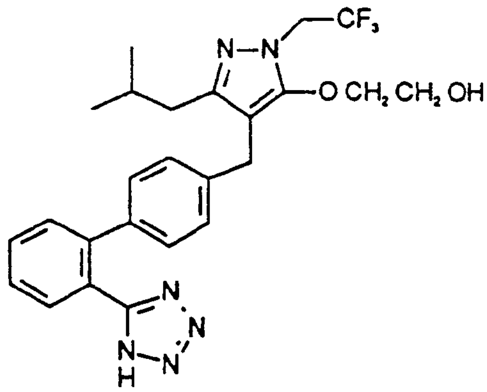

UP 221-60

Exemple 130

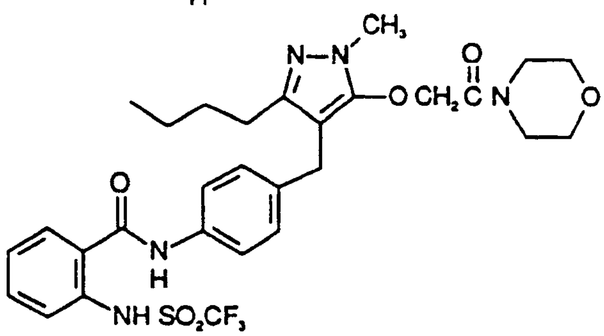

UP 221-25

Exemple 135

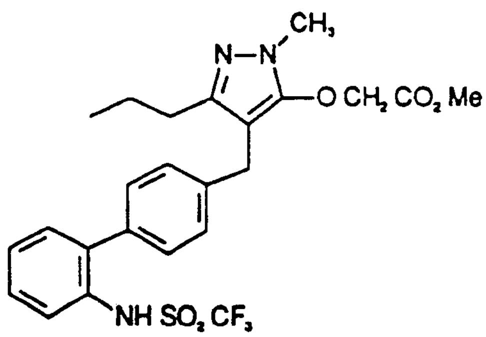

UP 221-26

Exemple 137

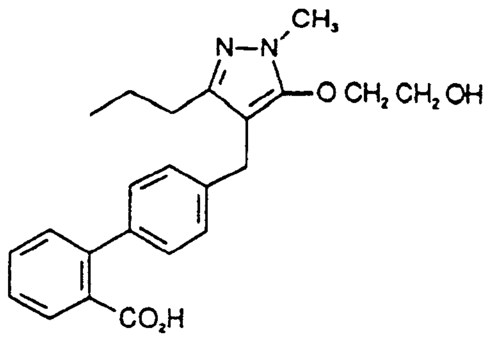

UP 221-29

Exemple 141

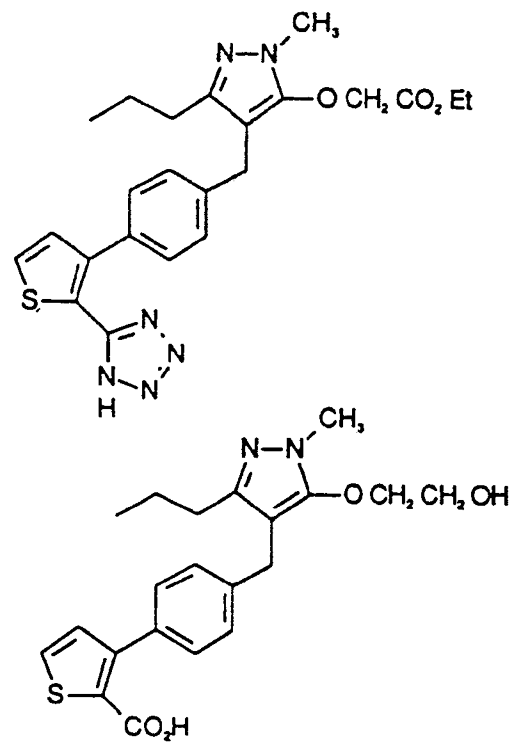

UP 221-35

Exemple 145

UP 221-39

Exemple 147

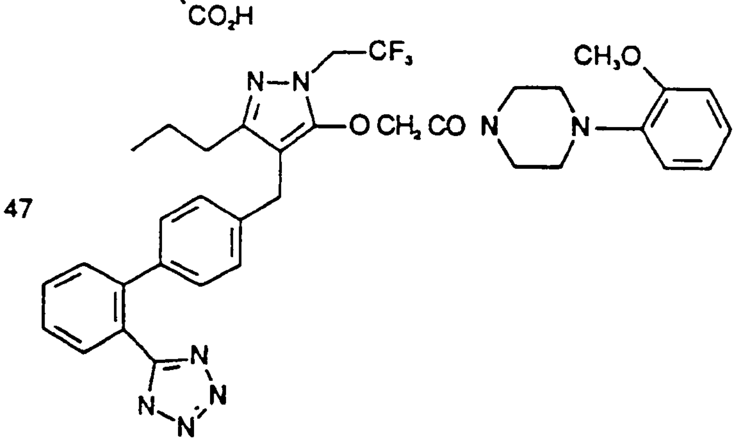

UP 221-62

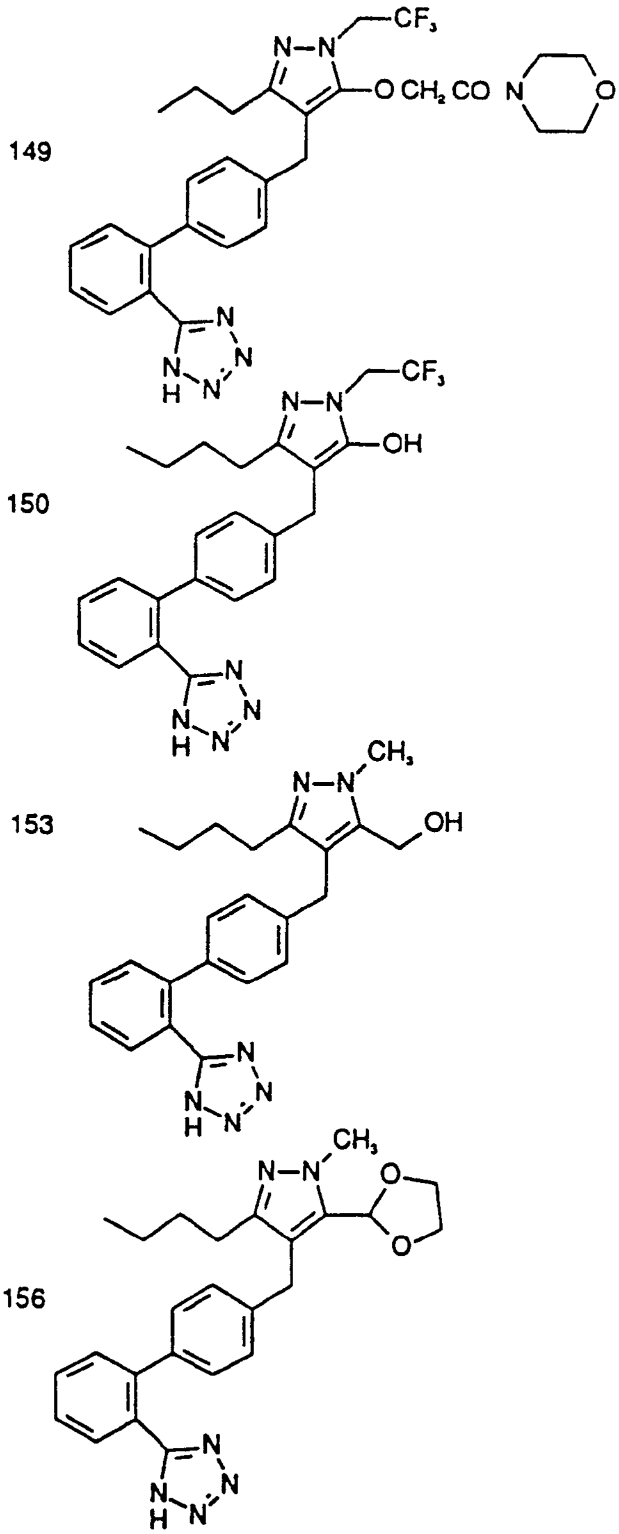

Exemple 149 UP 221-63

Exemple 150 UP 221-64

Exemple 153 UP 221-65

Exemple 156 UP 221-66

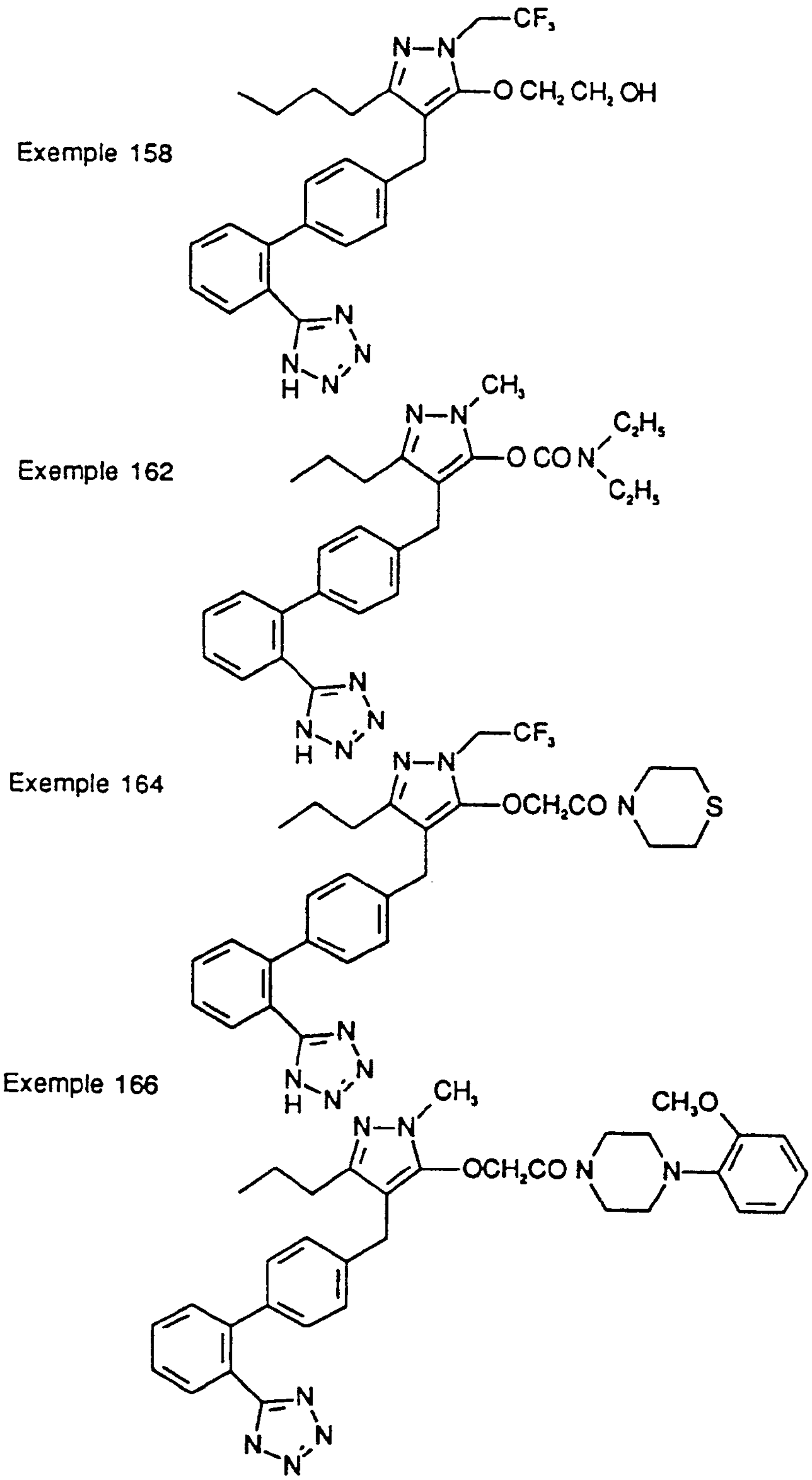

Exemple 158 UP 221-67

Exemple 162 UP 221-68

Exemple 164 UP 221-69

Exemple 166 UP 221-70

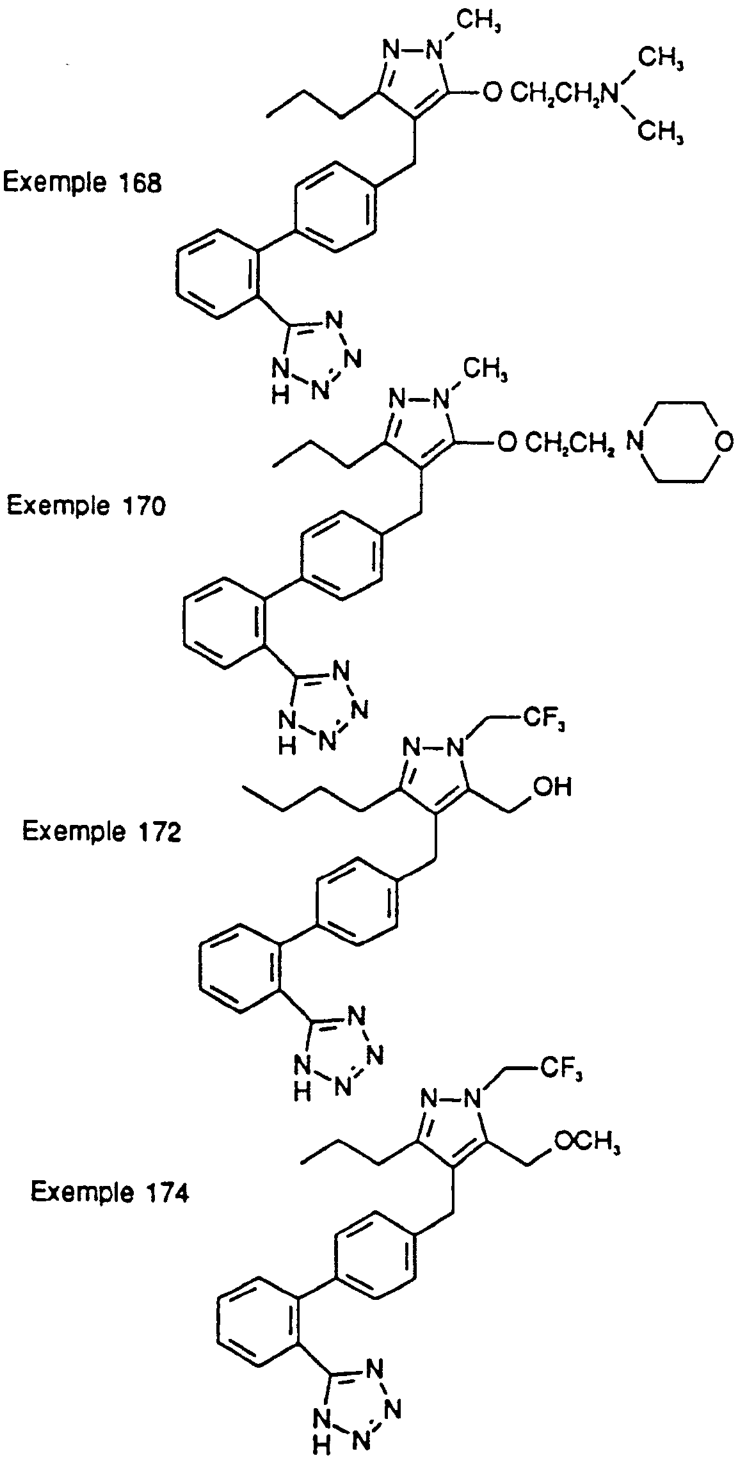

Exemple 168 UP 221-71

Exemple 170 UP 221-72

Exemple 172 UP 221-73

Exemple 174 UP 221-74

Exemple 177

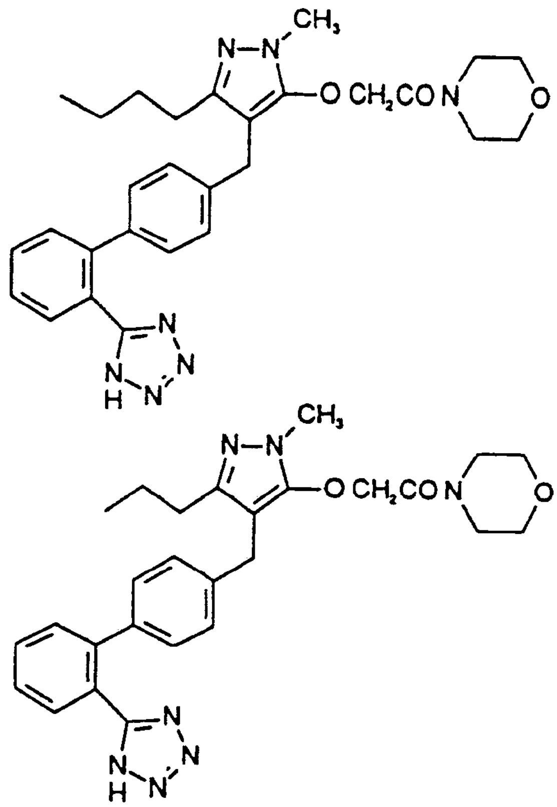

UP 221-76

Exemple 178

UP 221-77

Exemple 180

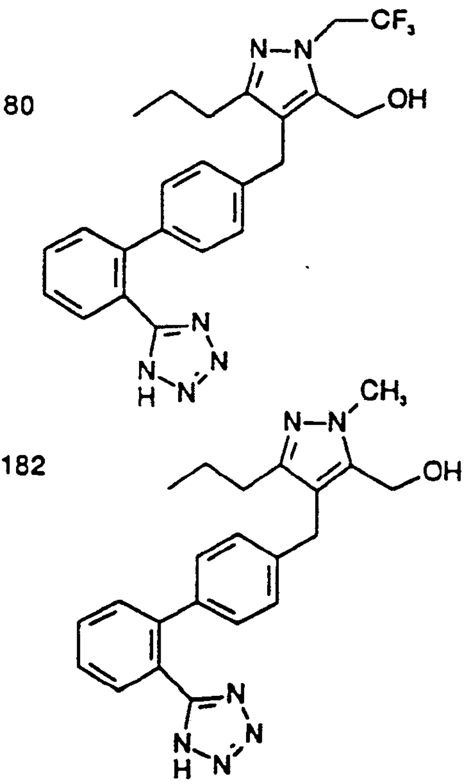

UP 221-78

Exemple 182

UP 221-79

Exemple 184 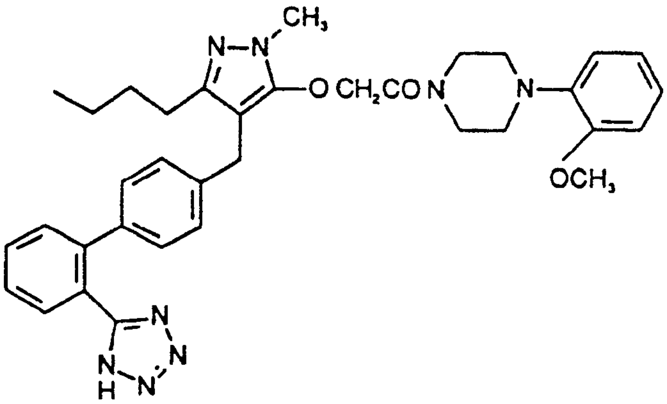 UP 221-80

Exemple 186 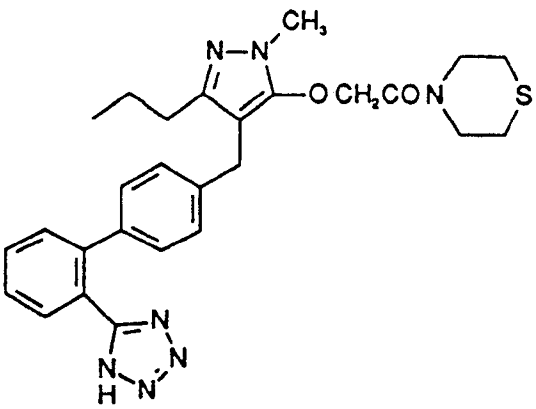 UP 221-81

PHARMACOLOGIE

I. Principe

L'affinité des produits des exemples pour les récepteurs de l'angiotensine II est évaluée par technique de déplacement d'un radioligand spécifiquement fixé sur les récepteurs surrénaliens de l'angiotensine II, chez le rat.

II. Mode opératoire

Une aliquote d'un homogénat de surrénales de rat incube en présence d'une concentration unique de $[^{125}I]$ - SIAII ($Sar^1$, $Tyr^4$, $Ile^8$ - angiotensine II), antagoniste des récepteurs de l'angiotensine II, et de deux concentrations d'agents compétiteurs ($10^{-5}$ M, $10^{-7}$ M) durant 60 min à 25°C.

La réaction est achevée par ajout de tampon, puis rapide filtration à travers des filtres de papier de verre. La liaison non spécifique est déterminée en présence d'angiotensine II.

III. Expression des résultats

Les résultats sont exprimés, pour les concentrations testées, en pourcentage de déplacement du radioligand spécifiquement fixé sur les récepteurs surrénaliens de l'angiotensine II.

4. Résultats

| Produit de l'exemple | % de déplacement du ligand marqué | |
|---|---|---|
| | 1E-5 M | 1E-7 M |
| 27 | 92 | 39 |
| 28 | 82 | 34 |
| 29 | 80 | 40 |
| 30 | 96 | 63 |
| 31 | 93 | 60 |
| 32 | 98 | 52 |
| 33 | 94 | 26 |
| 35 | 85 | 48 |
| 36 | 62 | 10 |
| 37 | 58 | 9 |
| 40 | 83 | 0 |
| 41 | 85 | 2 |
| 44 | 96 | 9 |
| 45 | 92 | 60 |
| 48 | 80 | 58 |
| 51 | 83 | 60 |
| 54 | 77 | 62 |
| 57 | 80 | 60 |
| 60 | 84 | 49 |
| 63 | 91 | 64 |
| 66 | 92 | 63 |
| 70 | 67 | 49 |
| 71 | 58 | 24 |

| Produit de l'exemple | % de déplacement du ligand marqué | |
|---|---|---|
| | 1E-5 M | 1E-7 M |
| 74 | 66 | 58 |
| 78 | 73 | 58 |
| 80 | 69 | 48 |
| 82 | 71 | 47 |
| 84 | 65 | 51 |
| 86 | 58 | 41 |
| 88 | 60 | 38 |
| 90 | 64 | 40 |
| 92 | 75 | 70 |
| 107 | 83 | 62 |
| 108 | 86 | 19 |
| 115 | 80 | 57 |
| 116 | 82 | 12 |
| 117 | 61 | 35 |
| 118 | 67 | 35 |
| 119 | 70 | 54 |
| 130 | 74 | 33 |
| 135 | 65 | 34 |
| 137 | 63 | 5 |
| 141 | 64 | 9 |
| 145 | 53 | 0 |

TOXICOLOGIE

Les produits des exemples décrits présentent, après administration par voie orale une excellente tolérance. Leur dose létale 50 chez le rat a été évaluée supérieure à 300 mg/kg.

CONCLUSION

Les produits des exemples décrits présentent une bonne affinité pour les récepteurs à l'Angiotensine II. A ce titre, ils pourront être utilisés avec bénéfice dans les diverses pathologies où l'Angiotensine II est impliquée, en particulier dans les traitements de l'hypertension artérielle pt de l'insuffisance cardiaque, à des posologies de 1 à 400 mg par voie orale et 0,01 à 50 mg par voie intraveineuse, en une ou plusieurs prises par jour.

## Revendications

1. Dérivés de pyrazole caractérisés en ce qu'ils répondent à la formule générale:

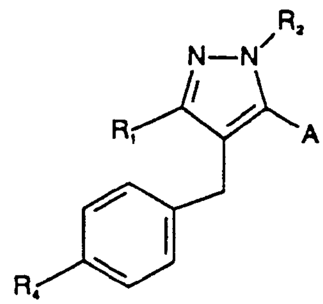

Formule (I)

dans laquelle :

$R_1$ est un radical alkyle inférieur de 1 à 6 atomes de carbone, un radical alkényle inférieur de 2 à 6 atomes de carbone, un radical cycloalkyle en $C_3$-$C_7$,

$R_2$ est l'atome d'hydrogène, un radical alkyle inférieur de 1 à 6 atomes de carbone, un radical halogérioalkyle inférieur de 1 à 6 atomes de carbone, un radical cycloalkyle en $C_3$-$C_7$, un groupement -$(CH_2)_mCOOR_5$, un groupement -$CH_2$-$(CH_2)_m$-$OR_5$ ou un groupement -$CH_2$-$(CH_2)_m$-S-$R_5$, m étant un nombre entier de 0 à 5, $R_5$ étant un atome d'hydrogène ou un radical alkyle inférieur de 1 à 6 atomes de carbone,

A peut représenter un groupement :

-$(CH_2)_q$ OR', R' étant un atome d'hydrogène ou un radical alkyle inférieur de 1 à 6 atomes de carbone ou un radical cycloalkyle en $C_3$-$C_7$, q étant un nombre entier de 1 à 5,
-$(CH_2)_q$L, L étant un atome d'halogène, préférentiellement chlore ou brome, q ayant la même signification que ci-dessus,
-CHO, un acétal, un dioxolane,
-COOR', R' étant défini comme ci-dessus,
-CONR"R"', R" et R"' représentant indépendamment un atome d'hydrogène, un radical akyle inférieur de 1 à 6 atomes de carbone, un radical cycloalkyle en $C_3$-$C_7$ ou pouvant former, avec l'atome d'azote auquel ils sont rattachés un hétérocycle comme la pyrrolidine, la pipéridine, la morpholine, la thiomorpholine ou une pipérazine,
-CN
-$(CH_2)_q$-CN, q étant défini comme ci-dessus,
-$(CH_2)_q$-COOR', R' et q étant définis comme ci-dessus,
-$(CH_2)_q$CONR"R"', R", R"' et q étant définis comme ci-dessus,
-$(CH_2)_q$NR"R"', R", R"' et q étant définis comme ci-dessus,
-$OR_3$, $R_3$ étant un atome d'hydrogène, un radical alkyle inférieur de 1 à 6 atomes de carbone, un radical cycloalkyle en $C_3$-$C_7$, un groupement -$(CH_2)_n$-$COOR_6$, un groupement -$(CH_2)(CH_2)_n$-CN, un groupement -$CH_2$-$(CH_2)_n$-O-$R_6$, un groupement -$CH_2$-$(CH_2)_n$-S-$R_6$, un groupement -CO-$R_6$, n étant un nombre entier de 0 à 5, $R_6$ étant un atome d'hydrogène ou un radical alkyle inférieur de 1 à 6 atomes de carbone, $R_3$ peut encore représenter un groupement -$(CH_2)_p$-$CONR_7R_8$ ou -$(CH_2)_p$-$CH_2$-$CH_2NR_7R_8$, p étant un nombre entier de 0 à 5, $R_7$ et $R_8$ représentant indépendamment un atome d'hydrogène, un radical alkyle inférieur de 1 à 6 atomes de carbone, un radical cycloalkyle en $C_3$-$C_7$ ou pouvant former avec l'atome d'azote auquel ils sont rattachés un hétérocycle comme la pyrrolidine, la pipéridine, la morpholine, le thiomorpholine ou une pipérazine,

$R_4$ peut représenter un groupement nitro, amino, -$COOR_9$, $R_9$ étant l'atome d'hydrogène, un radical alkyle inférieur de 1 à 6 atomes de carbone, $R_4$ peut encore représenter les radicaux suivants :

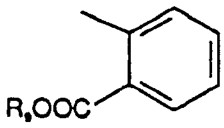 , 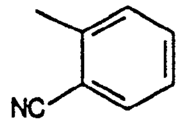 , 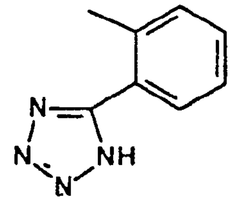 , 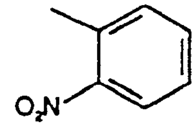

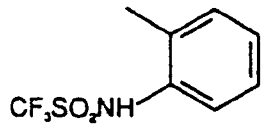 , 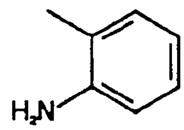 , 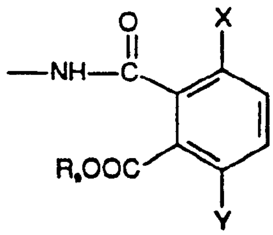

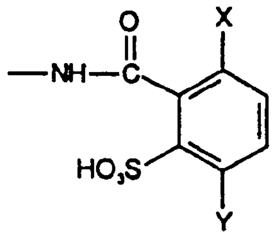 , 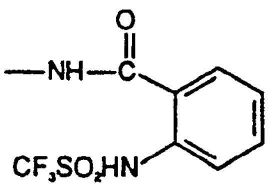 , 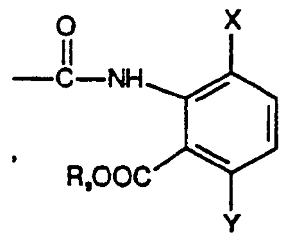

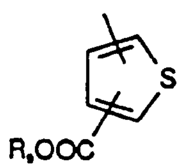 , 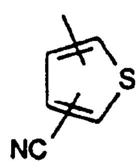 , 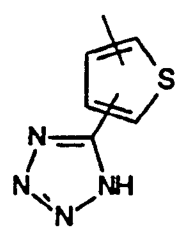

dans lesquels $R_9$ a la même signification que ci-dessus, X et Y représentant indépendamment un atome d'hydrogène, un radical alkyle inférieur, un atome d'halogène, un radical alkoxy ou un radical trifluorométhyl, ainsi que leurs sels d'addition, en particulier les sels d'addition pharmaceutiquement acceptables.

2. Dérivés selon la revendication 1, caractérisés en ce qu'ils répondent à la formule (I) dans laquelle :

$R_1$ est un radical alkyle inférieur de 1 à 6 atomes de carbone, de préférence choisi parmi n-propyle, n-butyle, méthyl-2 propyle ;

$R_2$ est l'atome d'hydrogène, un radical alkyle inférieur de 1 à 6 atomes de carbone, de préférence méthyle, un radical halogénoalkyle inférieur de 1 à 6 atomes de carbone, de préférence trifluoro-2,2,2 éthyle, ou un groupement $-(CH_2)_m-COOR_5$, m et $R_5$ étant tels que définis à la revendication 1, de préférence un groupement $CH_2-COOEt$ ;

A représente un groupement :

$-(CH_2)_qOR'$, R' étant choisi parmi un atome d'hydrogène et un groupement alkyle inférieur de 1 à 6 atomes de carbone, de préférence méthyle, et q étant un nombre entier de 1 à 5, de préférence égal à 1 ;

$-(CH_2)_qL$, L et q étant tels que définis à la revendication 1, de préférence $-CH_2Br$;

-CHO, un acétal ou un dioxolane;

$-OR_3$, $R_3$ étant choisi parmi un atome d'hydrogène; un groupement $-(CH_2)_n-COOR_6$ ou $-CH_2-(CH_2)_n-OR_6$, n étant un nombre entier de 0 à 5, de préférence égal à 0 ou 1, et $R_6$ étant un atome d'hydrogène ou un radical alkyle inférieur de 1 à 6 atomes de carbone, de préférence méthyle ou éthyle ; un groupement $-(CH_2)_p-CONR_7R_8$, p étant un nombre entier de 0 à 5, de préférence égal à 0 ou 1, $R_7$ et $R_8$ représentant indépendamment un atome d'hydrogène, un radical alkyle inférieur de 1 à 6 atomes de carbone, de préférence méthyle ou éthyle, ou bien $R_7$ et $R_8$ pris ensemble avec l'atome d'azote auquel ils sont rattachés,

formant un hétérocycle de préférence choisi parmi la morpholine, la pipérazine, la (méthoxy-2-phényl)-1 pipérazine ;

$R_4$ étant tel que défini à la revendication 1 ;

ainsi que leurs sels d'addition, en particulier les sels d'addition pharmaceutiquement acceptables.

3. Dérivés selon la revendication 1 ou 2, caractérisés en ce que $R_1$ est un groupement choisi parmi n-butyle et n-propyle.

4. Dérivés selon l'une quelconque des revendications 1 à 3, caractérisés en ce que $R_2$ est un groupement choisi parmi méthyle et trifluoro-2,2,2 éthyle.

5. Dérivés selon l'une quelconque des revendications 1 à 4, caractérisés en ce que A est un groupement choisi parmi éthoxy carbonyl méthylène oxy, diméthyl amino carbonyl oxy, méthoxy méthylène et hydroxy-2-éthyloxy.

6. Dérivés selon l'une quelconque des revendications 1 à 5, caractérisés en ce que $R_4$ est un groupement choisi parmi carboxy-2 dichloro-3,6 benzoylamino, acide sulfonique-2 benzoyl amino et (tétrazol-yl-5)-2 phényle.

7. Dérivés selon la revendication 1, caractérisés en ce qu'ils sont choisis parmi les dérivés de formule :

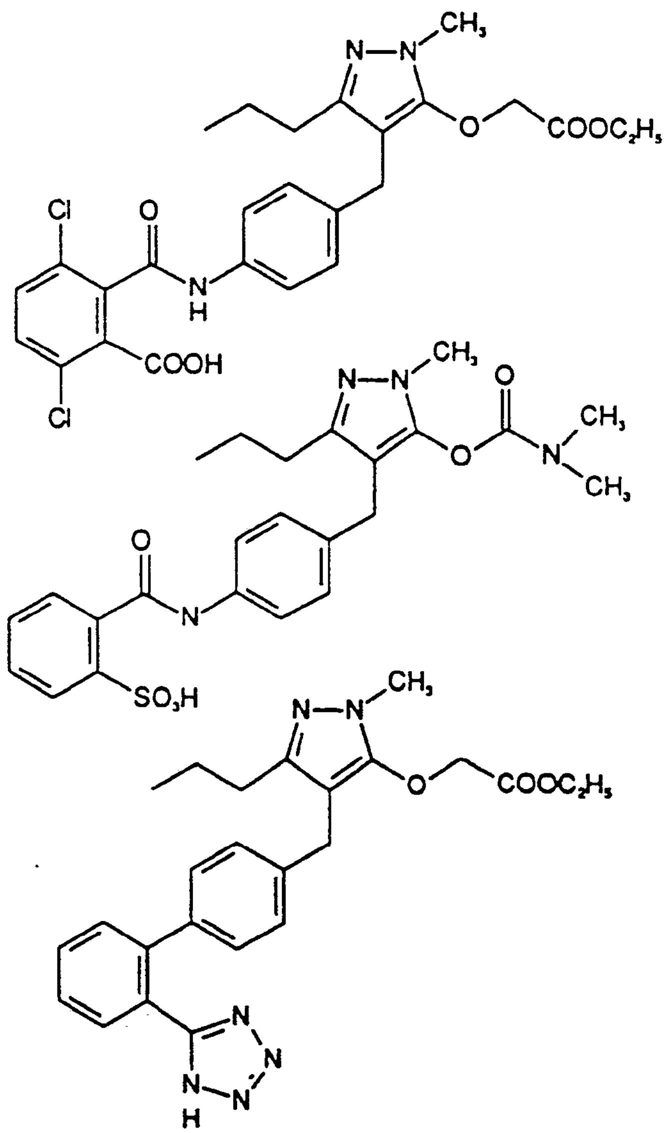

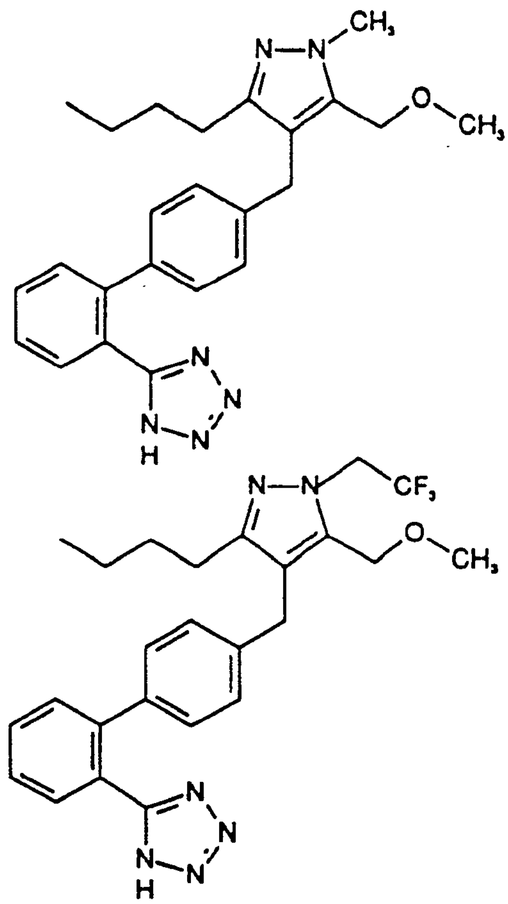

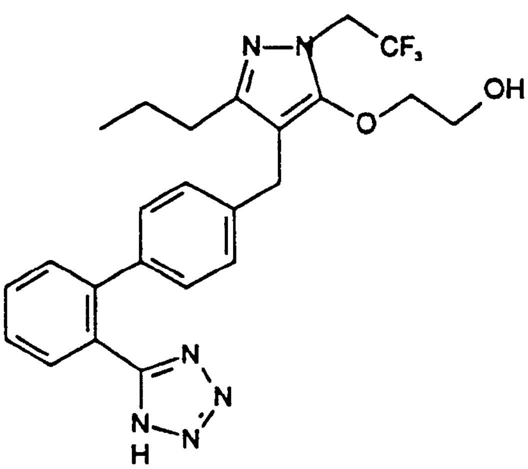

8. Procédé de préparation des dérivés de formule (I) selon l'une des revendications 1 à 7, caractérisé en ce qu'il comprend l'action d'une hydrazine de formule $H_2N\text{-}NH\text{-}R_2$, $R_2$ étant tel que défini précédemment, sur un céto ester de formule (V) ou une dicétone de formule (VI) :

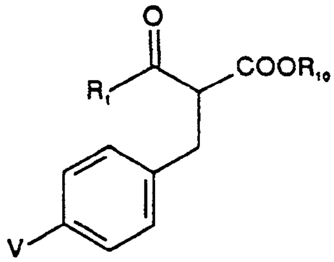

Formule (V)

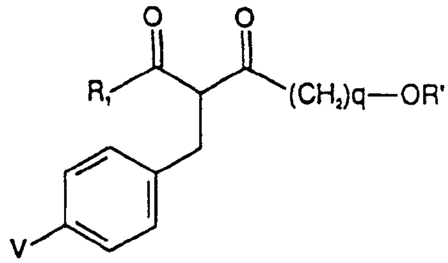

Formule (VI)

dans lesquelles $R_1$, R' et q sont tels que définis précédemment; $R_{10}$ est un alkyle inférieur, de préférence éthyle ou méthyle et V représente un groupement fonctionnel choisi parmi $NO_2$; $COOR_{11}$ ; $R_{11}$ étant un radical alkyle inférieur ou

le benzyle ; 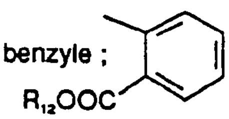 ,

$R_{12}$ étant un radical alkyle inférieur ou le benzyle ; 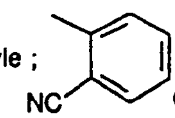 ; 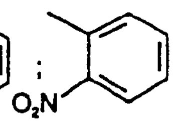 ;

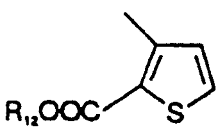 , $R_{12}$ étant tel que défini ci-dessus ; 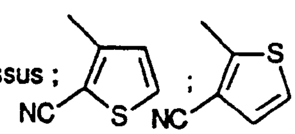 ;

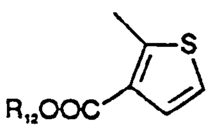 , $R_{12}$ étant tel que défini ci-dessus ;

ou bien l'action de l'hydrate d'hydrazine sur une dicétone de formule (VI) telle que mentionnée ci-dessus, suivie d'une alkylation en présence de DBU (1,8-diazabicyclo [5.4.0] uridec-7-ène) par des dérivés halogénés de formule Z-$R_2$ dans laquelle $R_2$ est tel que défini précédemment et Z représente un atome de brome, de chlore ou d'iode.

**9.** Procédé selon la revendication 8, caractérisé en ce que le céto ester de formule (V) précité est obtenu :
- par benzylation d'un oxo-3 alkanoate d'alkyl de formule (II):

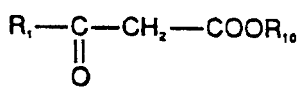

Formule (II)

dans laquelle $R_1$ et $R_{10}$ sont tels que définis précédemment à l'aide d'un composé de formule (IV):

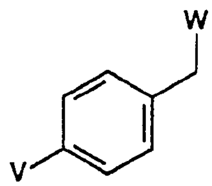

Formule (IV)

dans laquelle W représente un atome d'halogène, de préférence le chlore ou le brome et V est tel que défini précédemment;
– ou bien par condensation d'un aldéhyde de formule (VII) :

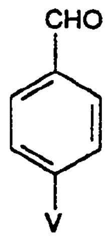

Formule (VII)

dans laquelle V est tel que défini précédemment sur ledit oxo-3 alkanoate d'alkyle de formule (II) suivie d'une hydrogénation catalytique.

**10.** Procédé selon la revendication 8, caractérisé en ce que la dicétone de formule (VI) précédée est obtenue :
– par benzylation d'une dicétone-1,3 de formule (III) :

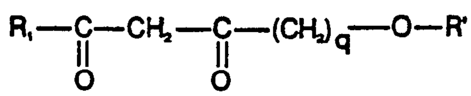

Formule (III)

dans laquelle $R_1$, R′ et q sont tels que définis précédemment à l'aide d'un composé de formule (IV) :

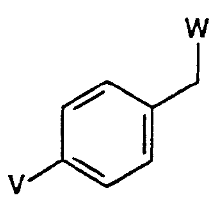

Formule (IV)

dans laquelle W représente un atome d'halogène, de préférence le chlore ou le brome et V est tel que défini précédemment ;
– ou bien par condensation d'un aldéhyde de formule (VII) :

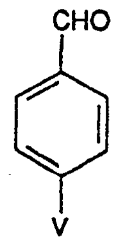

Formule (VII)

dans laquelle V est tel que défini précédemment sur ladite dicétone-1,3 de formule (III) suivie d'une hydrogénation catalytique.

**11.** Intermédiaires de synthèse pour la préparation des dérivés de formule (I) selon l'une des revendications 1 à 7, utiles notamment pour la mise en oeuvre du procédé selon la revendication 8, caractérisés en ce qu'ils répondent à l'une des formules (V) ou (VI) :

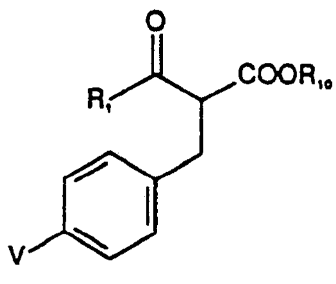

Formule (V)

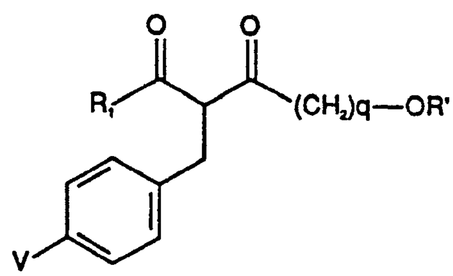

Formule (VI)

dans laquelle $R_1$, $R_{10}$, V, q et R' sont tels que définis précédemment.

**12.** Intermédiaires de synthèse pour la préparation de dérivés de formule (I) selon l'une des revendications 1 à 7, utiles notamment pour la mise en oeuvre du procédé selon la revendication 8, caractérisés en ce qu'ils répondent à l'une des formules (IX), (XI) et (XII) :

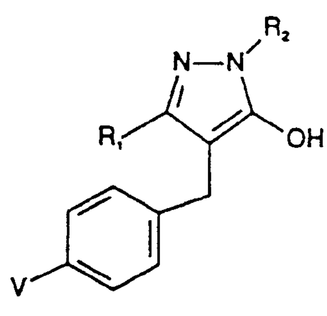

Formule (IX)

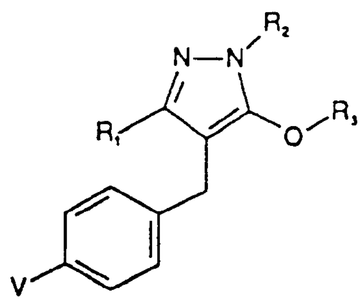

Formule (XI)

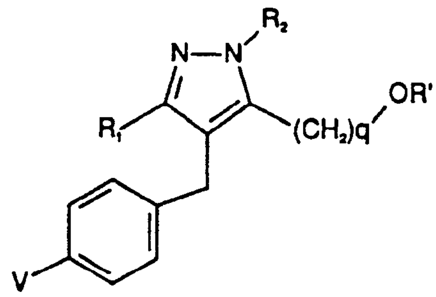

Formule (XII)

dans lesquelles $R_1$, $R_2$, $R_3$, V, q et R' sont tels que définis précédemment.

13. Composition pharmaceutique, caractérisée en ce qu'elle comprend une quantité pharmaceutiquement efficace d'au moins un composé de formule (I) selon l'une quelconque des revendications 1 à 7, ou l'un de ses sels d'addition pharmaceutiquement acceptables, en association avec un excipient, véhicule ou support pharmaceutiquement acceptable.

14. Composition pharmaceutique à activité antagoniste des récepteurs à l'angiotensine II, caractérisée en ce qu'elle comprend une quantité pharmaceutiquement efficace d'au moins un composé de formule (I) selon l'une quelconque des revendications 1 à 7, ou l'un de ses sels d'addition pharmaceutiquement acceptables, en association avec un excipient, véhicule ou support pharmaceutiquement acceptable.

15. Procédé de préparation d'une composition pharmaceutique, caractérisé en ce qu'on incorpore une quantité pharmaceutiquement efficace d'au moins un composé de formule (I) selon l'une quelconque des revendications 1 à 7, ou un de ses sels d'addition pharmaceutiquement acceptable, dans un excipient, véhicule ou support pharmaceutiquement acceptable.

16. Procédé selon la revendication 15, caractérisé en ce que la composition pharmaceutique est formulée sous forme de gélules, de comprimés dosés de 1 à 400 mg ou sous forme de préparations injectables dosées de 0,01 à 50 mg.